# EUROPÄISCHE PATENTSCHRIFT

(11) **EP 1 080 072 B1**
(45) Veröffentlichungstag und Bekanntmachung des Hinweises auf die Patenterteilung: **04.08.2004**
(21) Anmeldenummer: 99924878.4
(22) Anmeldetag: 05.05.1999
(51) Int. Cl.: C07D 207/20, C07C 271/18, C07C 205/45, A01N 43/36

(54) **2-(2-CHLORPHENYL)-3,4-DIHYDRO-2H-PYRROL-DERIVATE**
2-(2-CHLOROPHENYL)-3,4-DIHYDRO-2H-PYRROL DERIVATIVES
DERIVES DE 2-(2-CHLOROPHENYL)-3,4-DIHYDRO-2H-PYRROL

(30) Priorität: 18.05.1998 DE 19822247
(43) Veröffentlichungstag der Anmeldung: 07.03.2001
(73) Patentinhaber: Bayer CropScience AG, 40789 Monheim (DE)
(72) Erfinder: PLANT, Andrew, D-51373 Leverkusen (DE); GRAFF, Alan, D-51061 Köln (DE); KRAATZ, Udo, D-51375 Leverkusen (DE); ERDELEN, Christoph, D-42799 Leichlingen (DE); TURBERG, Andreas, D-42781 Haan (DE); MENCKE, Norbert, D-51381 Leverkusen (DE)
(86) Internationale Anmeldenummer: PCT/EP1999/003063
(87) Internationale Veröffentlichungsnummer: WO 1999/059968

(56) Entgegenhaltungen:
- EP-A- 0 167 419
- WO-A-95/04719
- WO-A-98/22438
- FR-A- 2 607 361
- PATENT ABSTRACTS OF JAPAN vol. 199, no. 607, 31. Juli 1996 (1996-07-31) -& JP 08 073446 A (NIPPON SODA CO LTD), 19. März 1996 (1996-03-19)
- W. KOLLER ET AL.: "Ein neues Darstellungsverfahren für 2-Aryl-delta1-pyrroline und 2-Aryl-delta1-piperidine" CHEMISCHE BERICHTE, Bd. 96, 1963, Seiten 93-113, XP002117316 WEINHEIM DE in der Anmeldung erwähnt

## Beschreibung

Die Erfindung betrifft neue 2-(2-Chlorphenyl)-3,4-dihydro-2H-pyrrol-Derivate, mehrere Verfahren zu ihrer Herstellung und ihre Verwendung als Schädlingsbekämpfungsmittel.

Bisher sind nur wenige substituierte cyclische α,α'-Diphenylimine bekannt geworden: Drei im 2-Phenylring alkoxysubstituierte 2,5-Diphenyl-1-pyrroline [5-(2,5-Dimethoxyphenyl)-2-phenyl-3,4-dihydro-2H-pyrrol und 5-(4-Methoxyphenyl)-2-phenyl-3,4-dihydro-2H-pyrrol aus Chem. Ber. **96,** 93 (1963) und die entsprechende 4-Propoxy-Verbindung aus J. Prakt. Chem., Serie 4, **1**, 57 (1955)] sowie das nicht weiter substituierte 2,6-Diphenyl-3,4,5,6-tetrahydropyridin [vgl. z.B. Bull. Soc. Chim. Fr. **1974**, 258 u. Chem. Ber. **116**, 3931 (1983)].

Über deren Eignung zur Verwendung als Schädlingsbekämpfungsmittel ist nichts bekannt.

Weiterhin sind aus JP-A 8-73446 ähnliche Verbindungen, nämlich 2,4-Bisaryl-5,6-dihydro-1,3-oxazin- bzw. 2,4-Bisaryl-5,6-dihydro-1,3-thiazin-Derivate, bekannt. EP-A 0 167 419 und FR-A 2 607 361 offenbaren Dihydropyrrol-Derivate, die in 2-und 5-Position jedoch nicht durch Phenyl substituiert sein können.

Es wurden neue 2-(2-Chlorphenyl)-3,4-dihydro-2H-pyrrol-Derivate der Formel (I) 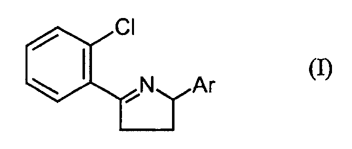 gefunden, in welcher
- Ar: für den Rest 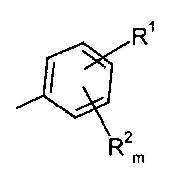 steht, wobei
- m: für 0, 1, 2, 3 steht,
- R¹: für einen Substituenten in meta- oder para-Position aus der Reihe Wasserstoff, Halogen, Cyano, Tri-(C₁-C₆-alkyl)-silyl, -CO-NR⁴R⁵, Tetrahydropyranyl oder eine der folgenden Gruppierungen

(l) -X - A

(m) -B - Z - D

(n) -Y - E

steht,
- R²: für Wasserstoff, Halogen, Cyano, Nitro, C₁-C₁₆-Alkyl, C₁-C₁₆-Alkoxy, C₁-C₆-Halogenalkyl, C₁-C₆-Halogenalkoxy, C₁-C₈-Alkoxy-C₁-C₈-alkoxy oder -S(O)ₒR³ steht,
- o: für 0, 1 oder 2 steht,
- R³: für gegebenenfalls durch Fluor oder Chlor substituiertes C ₁-C₆-Alkyl steht,
- R⁴ und R⁵: unabhängig voneinander für Wasserstoff, C₁-C₆-Alkyl, C₁-C₆-Halogenalkyl oder für jeweils gegebenenfalls einfach bis dreifach durch Reste aus der Liste W¹ substituiertes Phenyl oder Phenyl-C₁-C₄-alkyl stehen,
- X: für eine direkte Bindung, Sauerstoff, Schwefel, Carbonyl, Carbonyloxy, Oxycarbonyl, C₁-C₄-Alkylen, C₂-C₄-Alkenylen, C₂-C₄-Alkinylen, C₁-C₄-Alkylenoxy, C₁-C₄-Oxyalkylen, C₁-C₄-Thioalkylen, C₁-C₄-Alkylendioxy oder Di-C₁-C₄-alkylsilylen steht,
- A: für jeweils gegebenenfalls einfach bis vierfach durch Reste aus der Liste W¹ substituiertes Phenyl, Naphthyl oder Tetrahydronaphthyl oder für jeweils gegebenenfalls einfach bis vierfach durch Reste aus der Liste W² substituiertes 5- bis 10-gliedriges, 1 oder 2 aromatische Ringe enthaltendes Heterocyclyl mit 1 bis 4 Heteroatomen, welches 0 bis 4 Stickstoffatome, 0 bis 2 Sauerstoffatome und 0 bis 2 Schwefelatome enthält, (insbesondere Furyl, Benzofuryl, Thienyl, Benzothienyl, Oxazolyl, Benzoxazolyl, Thiazolyl, Benzthiazolyl, Pyrrolyl, Pyridyl, Pyrimidyl, 1,3,5-Triazinyl, Chinolinyl, Isochinolinyl, Indolyl, Purinyl, Benzodioxolyl, Indanyl, Benzodioxanyl oder Chromanyl) steht,
- B: für gegebenenfalls einfach oder zweifach durch Reste aus der Liste W¹ substituiertes p-Phenylen steht,
- Z: für Sauerstoff oder Schwefel steht,
- D: für Wasserstoff, C₁-C₁₆-Alkyl, C₂-C₁₆-Alkenyl, C₂-C₆-Alkinyl, C₁-C₁₆-Halogenalkyl, C₂-C₁₆-Halogenalkenyl, jeweils gegebenenfalls durch Halogen, C₁-C₄-Alkyl, C₂-C₄-Alkenyl, C₂-C₄-Halogenalkenyl, Phenyl, Styryl, Halogenphenyl oder Halogenstyryl substituiertes C₃-C₈-Cycloalkyl oder C₃-C₈-Cycloalkyl-C₁-C₆-alkyl, für jeweils gegebenenfalls durch Halogen oder C₁-C₄-Alkyl substituiertes C₅-C₈-Cycloalkenyl oder C₅-C₈-Cycloalkenyl-C₁-C₄-alkyl, für jeweils gegebenenfalls durch Nitro, Halogen, C₁-C₆-Alkyl, C₁-C₆-Alkoxy, C₁-C₆-Halogenalkyl oder C₁-C₆-Halogenalkoxy substituiertes Phenyl-C₁-C₆-alkyl, Naphthyl-C₁-C₆-alkyl, Tetrahydronaphthyl-C₁-C₆-alkyl oder 5- oder 6-ringgliedriges Hetaryl-C₁-C₆-alkyl mit 1 oder 2 Heteroatomen aus der Reihe Stickstoff, Sauerstoff und Schwefel (insbesondere Furylmethyl, Thienylmethyl, Pyrrolylmethyl, Oxazolylmethyl, Isoxazolylmethyl, Thiazolylmethyl oder Pyridylmethyl), für -CO-R⁶, -CO-NR⁷R⁸ oder für die Gruppierung

-(CH₂)ₚ - (CR⁹R¹⁰)_{q} - (CH₂)ᵣ - G

steht,
- Z und D: können auch gemeinsam für jeweils gegebenenfalls durch Nitro, Halogen, C₁-C₆-Alkyl, C₁-C₆-Alkoxy, C₁-H₆-Halogenalkyl oder C₁-C₆-Halogenalkoxy substituiertes Phenoxy-C₁-C₄-alkyl, stehen,
- Y: für eine direkte Bindung, Sauerstoff, Schwefel, Carbonyl, Carbonyloxy, Oxycarbonyl, C₁-C₄-Alkylen, C₂-C₄-Alkenylen, C₂-C₄-Alkinylen, C₁-C₄-Alkylenoxy, C₁-C₄-Oxyalkylen, C₁-C₄-Thioalkylen, C₁-C₄-Alkylendioxy oder für gegebenenfalls einfach oder zweifach durch Reste aus der Liste W¹ substituiertes p-Phenylen steht,
- E: für Wasserstoff, C₁-C₁₆-Alkyl, C₂-C₁₆-Alkenyl, C₂-C₆-Alkinyl, C₁-C₁₆-Halogenalkyl, C₂-C₁₆-Halogenalkenyl, gegebenenfalls durch Halogen, C₁-C₄-Alkyl, C₂-C₄-Alkenyl, C₂-C₄-Halogenalkenyl, Phenyl, Styryl, Halogenphenyl oder Halogenstyryl substituiertes C₃-C₈-Cycloalkyl, für gegebenenfalls durch Halogen oder C₁-C₄-Alkyl substituiertes C₅-C₈-Cycloalkenyl, für gegebenenfalls einfach bis vierfach durch Reste aus der Liste W¹ substituiertes Phenyl oder für jeweils gegebenenfalls einfach bis vierfach durch Reste aus der Liste W² substituiertes 5- oder 6-gliedriges Hetaryl mit 1 oder 2 Heteroatomen aus der Reihe Stickstoff, Sauerstoff und Schwefel (insbesondere Furyl, Thienyl, Pyrrolyl, Oxazolyl, Isoxazolyl, Thiazolyl oder Pyridyl) oder für die Gruppierung

-(CH₂)ₚ - (CR⁹R¹⁰)_{q} - (CH₂)ᵣ - G

steht,
- R⁶: für C₁-C₁₂-Alkyl, C₁-C₁₂-Alkoxy, C₂-C₁₂-Alkenyl, C₂-C₁₂-Alkenyloxy, jeweils gegebenenfalls durch Halogen, C₁-C₄-Alkyl, C₂-C₄-Alkenyl, C₁-C₄-Halogenalkyl oder C₂-C₄-Halogenalkenyl substituiertes C₃-C₈-Cycloalkyl, C₃-C₈-Cycloalkyloxy oder C₃-C₈-Cycloalkyl-C₁-C₆-alkyloxy oder für jeweils gegebenenfalls einfach bis vierfach durch Nitro, Halogen, C₁-C₁₂-Alkyl, C₁-C₁₂-Alkoxy, C₁-C₁₂-Halogenalkyl oder C₁-C₁₂-Halogenalkoxy substituiertes Phenyl oder Naphthyl steht,
- R⁷: für Wasserstoff oder C₁-C₁₂-Alkyl steht,
- R⁸: für C₁-C₁₂-Alkyl, C₁-C₁₂-Halogenalkyl, jeweils gegebenenfalls durch Halogen, C₁-C₄-Alkyl, C₂-C₄-Alkenyl, C₁-C₄-Halogenalkyl oder C₂-C₄-Halogenalkenyl substituiertes C₃-C₈-Cycloalkyl oder C₃-C₈-Cycloalkyl-C₁-C₆-alkyl oder für jeweils gegebenenfalls einfach bis vierfach durch Halogen, C₁-C₁₂-Alkyl, C₁-C₁₂-Alkoxy, C₁-C₁₂-Halogenalkyl oder C₁-C₁₂-Halogenalkoxy substituiertes Phenyl oder Phenyl-C₁-C₆-alkyl steht,
- p, q und r: unabhängig voneinander für 0, 1, 2 oder 3, wobei deren Summe kleiner 6 ist stehen,
- R⁹ und R¹⁰: unabhängig voneinander für Wasserstoff oder C₁-C₄-Alkyl stehen,
- G: für Cyano, für einen gegebenenfalls einfach bis dreifach durch Halogen, C₁-C₄-Alkyl oder C₁-C₄-Halogenalkyl und gegebenenfalls an der Verknüpfungsstelle durch den Rest R¹¹ substituierten 5- oder 6-gliedrigen Heterocyclus mit 1 bis 3 gleichen oder verschiedenen Heteroatomen aus der Reihe Stickstoff, Sauerstoff und Schwefel (insbesondere 5,6-Dihydrodioxazin-2-yl, 3-Pyridyl, 3-Furyl, 3-Thienyl, 2-Thiazolyl, 5-Thiazolyl, 2-Dioxolanyl, 1,3-Dioxan-2-yl, 2-Dithiolanyl, 1,3-Dithian-2-yl oder 1,3-Thioxan-2-yl) oder eine der folgenden Gruppierungen steht:

(a) ―CO―R¹¹

(b) ―CO―OR¹²

(c) ―CO―NR¹³R¹⁴

(d) ―CS―NR¹³R¹⁴ 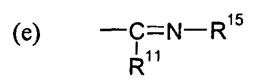 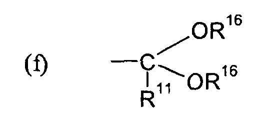 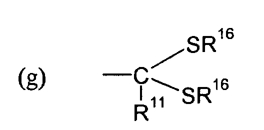 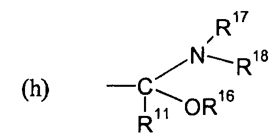 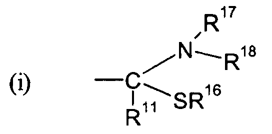 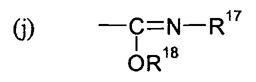 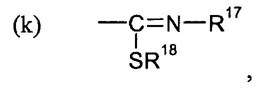
- R¹¹: für Wasserstoff, C₁-C₆-Alkyl, C₂-C₆-Alkenyl, C₁-C₄-Halogenalkyl, C₂-C₆-Halogenalkenyl, gegebenenfalls durch Halogen, C₁-C₄-Alkyl oder C₁-C₄-Halogenalkyl substituiertes C₃-C₆-Cycloalkyl oder für gegebenenfalls einfach bis fünffach durch C₁-C₄-Alkylcarbonylamino, C₁-C₄-Alkylcarbonyl-C₁-C₄-alkylamino und/oder Reste aus der Liste W³ substituiertes Phenyl steht,
- R¹²: für Wasserstoff, C₁-C₄-Alkyl, C₂-C₆-Alkenyl, C₁-C₄-Halogenalkyl, C₂-C₆-Halogenalkenyl, jeweils gegebenenfalls durch Halogen, C₁-C₄-Alkyl oder C₁-C₄-Halogenalkyl substituiertes C₃-C₆-Cycloalkyl oder C₃-C₆-Cycloalkyl-C₁-C₄-alkyl oder für gegebenenfalls einfach bis vierfach durch Reste aus der Liste W³ substituiertes C₆-C₁₀-Aryl-C₁-C₄-alkyl (insbesondere Phenyl-C₁-C₄-alkyl oder Naphthyl-C₁-C₄-alkyl) steht,
- R¹³ und R¹⁴: unabhängig voneinander für Wasserstoff, C₁-C₄-Alkyl, C₃-C₆-Alkenyl, C₁-C₄-Halogenalkyl, C₃-C₆-Halogenalkenyl, C₁-C₄-Alkoxy, jeweils gegebenenfalls durch Halogen, C₁-C₄-Alkyl oder C₁-C₄-Halogenalkyl substituiertes C₃-C₆-Cycloalkyl oder C₃-C₆-Cycloalkyl-C₁-C₄-alkyl, für jeweils gegebenenfalls einfach bis fünffach durch Reste aus der Liste W³ substituiertes Phenyl oder Phenyl-C₁-C₄-alkyl, für -OR¹² oder -NR¹¹R¹² oder gemeinsam für eine Alkylenkette mit 4 bis 6 Gliedern, in welcher gegebenenfalls eine Methylengruppe durch Sauerstoff ersetzt ist stehen,
- R¹⁵: für -OR¹², -NR¹¹R¹² oder -N(R¹¹)-COOR¹² steht,
- R¹⁶, R¹⁷ und R¹⁸: unabhängig voneinander für C₁-C₆-Alkyl stehen,
- W¹: für Wasserstoff, Halogen, Cyano, Formyl, Nitro, C₁-C₆-Alkyl, Tri-C₁-C₄-alkylsilyl, C₁-C₁₆-Alkoxy, C₁-C₆-Halogenalkyl, C₁-C₆-Halogenalkoxy, C₂-C₆-Halogenalkenyloxy, C₁-C₆-Alkylcarbonyl, C₁-C₁₆-Alkoxycarbonyl, Pentafluorthio oder -S(O)ₒR³ steht,
- W²: für Halogen, Cyano, Formyl, Nitro, C₁-C₆-Alkyl, Tri-C₁-C₄-alkylsilyl, C₁-C₁₆-Alkoxy, C₁-C₆-Halogenalkyl, C₁-C₆-Halogenalkoxy, C₁-C₆-Alkylcarbonyl, C₁-C₁₆-Alkoxycarbonyl, Pentafluorthio, -S(O)ₒR³ oder -C(R¹¹)=N-R¹⁵ steht,
- W³: für Halogen, Cyano, Nitro, C₁-C₄-Alkyl, C₁-C₄-Alkoxy, C₁-C₄-Halogenalkyl, C₁-C₄-Halogenalkoxy, Di-C₁-C₄-alkylamino, -S(O)ₒR³, -COOR¹⁹ oder -CONR²⁰R²¹ steht,
- R¹⁹: für Wasserstoff, C₁-C₄-Alkyl, C₁-C₄-Halogenalkyl, gegebenenfalls durch Halogen, C₁-C₄-Alkyl oder C₁-C₄-Halogenalkyl substituiertes C₃-C₇-Cycloalkyl oder für gegebenenfalls einfach bis fünffach durch Reste aus der Liste W⁴ substituiertes Phenyl steht,
- R²⁰ und R²¹: unabhängig voneinander für Wasserstoff, C₁-C₄-Alkyl, C₃-C₆-Alkenyl, C₁-C₄-Halogenalkyl, C₃-C₆-Halogenalkenyl, C₁-C₄-Alkoxy, jeweils gegebenenfalls durch Halogen, C₁-C₄-Alkyl oder C₁-C₄-Halogenalkyl substituiertes C₃-C₆-Cycloalkyl oder C₃-C₆-Cycloalkyl-C₁-C₄-alkyl oder für jeweils gegebenenfalls einfach bis fünffach durch Reste aus der Liste W⁴ substituiertes Phenyl oder Phenyl-C₁-C₄-alkyl, für -OR¹⁶ oder -NR¹⁷R¹⁸ oder gemeinsam für eine Alkylenkette mit 4 bis 6 Gliedern, in welcher gegebenenfalls eine Methylengruppe durch Sauerstoff ersetzt ist stehen, und
- W⁴: für Halogen, Cyano, Nitro, C₁-C₆-Alkyl, C₁-C₆-Alkoxy, C₁-C₆-Halogenalkyl, C₁-C₆-Halogenalkoxy, Di-C₁-C₄-alkylamino, C₁-C₆-Alkoxycarbonyl, Di-C₁-C₆-alkylaminocarbonyl oder -S(O)ₒR³ steht,

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Weiterhin wurde gefunden, daß man die neuen Verbindungen der Formel (I) nach einem der im folgenden beschriebenen Verfahren erhält.
A) Cyclische Imine der Formel (I) 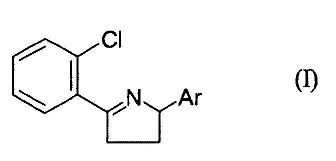 in welcher
   Ar die oben angegebenen Bedeutungen hat,
   lassen sich herstellen, indem man
   a) Aminoketonderivate der Formel (VIII) 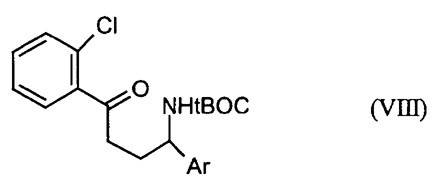 in welcher
      - Ar: die oben angegebenen Bedeutungen hat,
      mit einer Säure umsetzt und anschließend gegebenenfalls in Gegenwart eines Säurebindemittels cyclokondensiert, oder
   b) die Nitrogruppe von Nitroketonen der Formel (XVIII), 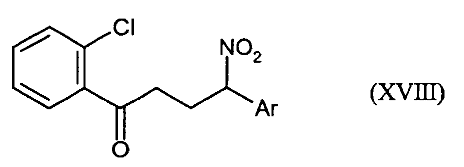 in welcher
      Ar die oben genannten Bedeutungen hat, reduziert, wobei 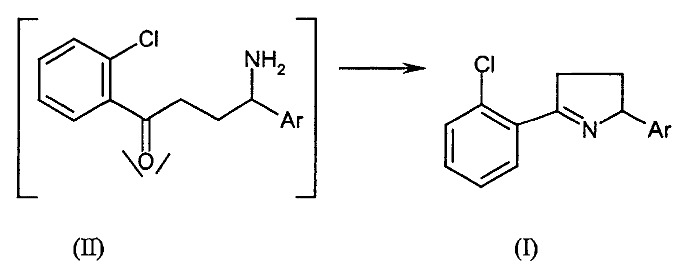 intermediär ein Aminoketon der Formel (II) auftritt, welches jedoch, insbesondere in saurem Medium, in situ zu (I) cyclokondensiert wird, oder
   c) Imine der Formel (XXVII) 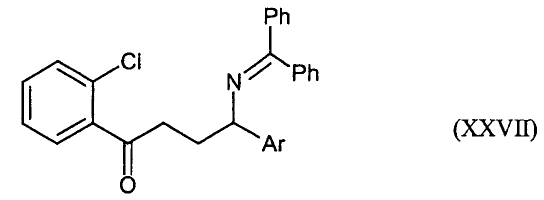 in welcher Ar die oben genannten Bedeutungen hat, mit wässrigen Säuren hydrolysiert 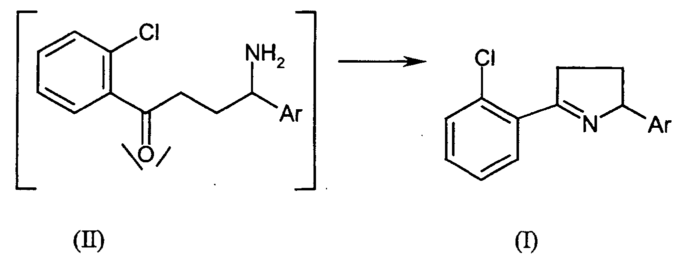 wobei
      intermediär ein Aminoketon der Formel (II) auftritt, welches jedoch in situ zu (I) cyclokondensiert wird.
B) Cyclische Imine der Formel (I) lassen sich auch herstellen, indem man cyclische *O*-Methylsulfonyloxime der Formel (III) 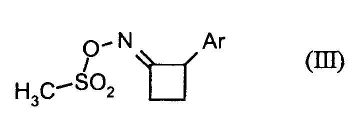 in welcher Ar die oben angegebenen Bedeutungen hat,
   mit Aryl-Grignard-Verbindungen der Formel (IV) 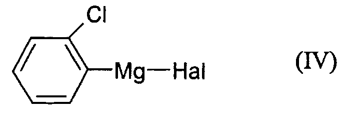 in welcher
   - Hal: für Brom oder Iod steht,
   in Gegenwart eines Verdünnungsmittels umsetzt.
C) Cyclische Imine der Formel (I-b) 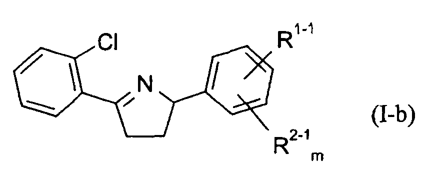 in welcher
   - m: die oben angegebenen Bedeutungen hat,
   - R¹⁻¹: für A oder eine der folgenden Gruppierungen

   (m) ―B―Z-D

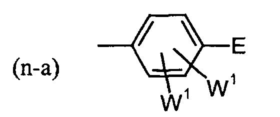

   steht, wobei
   A, B, D, E, W¹ und Z die oben angegebenen Bedeutungen haben und
   R²⁻¹ für Wasserstoff, Fluor, Cyano, Nitro, Alkyl, Alkoxy, Halogenalkyl, Halogenalkoxy, Alkoxyalkoxy oder -SR³ steht, wobei
   R³ die oben angegebenen Bedeutungen hat,
   lassen sich herstellen, indem man Verbindungen der Formel (V) 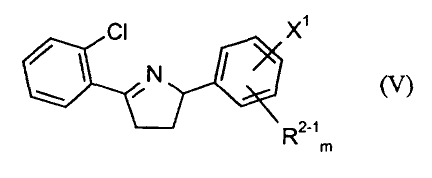 in welcher
   - R²⁻¹ und m: die oben angegebenen Bedeutungen haben und
   - X¹: für Brom, Iod oder -OSO₂CF₃ steht
   mit Boronsäuren der Formel (VI)

   R¹⁻¹ B(OH)₂ (VI),

   in welcher
   - R¹⁻¹: die oben angegebenen Bedeutungen hat,
   in Gegenwart eines Katalysators und in Gegenwart eines Säurebindemittels und in Gegenwart eines Lösungsmittels kuppelt.
D) Cyclische Imine der Formel (I-c) 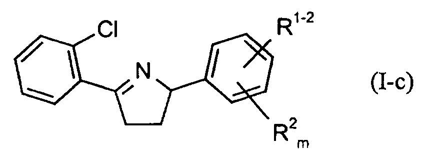 in welcher
   - R² und m: die oben angegebenen Bedeutungen haben,
   - R¹⁻²: für eine der folgenden Gruppierungen

   (m-b) -B - Z - D¹

   (n-b) -Y¹ - E¹

   steht, in denen
   B und Z die oben angegebenen Bedeutungen haben,
   Y¹ für Sauerstoff oder Schwefel steht und
   D¹ und E¹ für die Gruppierung

   -(CH₂)ₚ-(CR⁹R¹⁰)_{q}-(CH₂)ᵣ - G

   stehen, in der
   R⁹, R¹⁰, G, p, q und r die oben angegebenen Bedeutungen haben,
   lassen sich herstellen, indem man cyclische Imine der Formel (I-d) 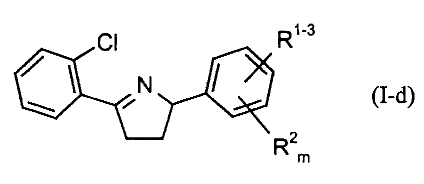 in welcher
   - R² und m: die oben angegebenen Bedeutungen haben und
   - R¹⁻³: für eine der folgenden Gruppierungen

   (m-c) -B - Z - H

   (n-c) -Y¹ - H

   steht, in denen
   B, Y¹ und Z die oben angegebenen Bedeutungen haben,
   mit Verbindungen der Formel (VII)

   Ab - (CH₂)ₚ - (CR⁹R¹⁰)_{q} - (CH₂)ᵣ - G (VII),

   in welcher
   - R⁹, R¹⁰, G, p, q und r: die oben angegebenen Bedeutungen haben und
   - Ab: für eine Abgangsgruppe steht,
   kondensiert.
E) Cyclische Imine der Formel (I-e) 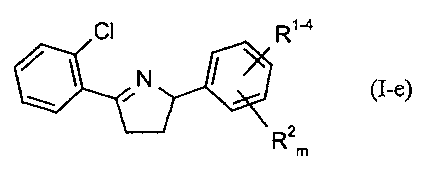 in welcher
   - R² und m: die oben angegebenen Bedeutungen haben und
   - R¹⁻⁴: für eine den Rest G enthaltenden Gruppierung aus der Beschreibung der erfindungsgemäßen Verbindungen der Formel (I) steht, wobei
   G für eine der oben genannten Gruppierungen (e) bis (k) steht,
   lassen sich herstellen durch allgemein übliche und bekannte Derivatisierungen der entsprechenden Keto-Derivate, Carbonsäure-Derivate oder Nitrile, d.h. Verbindungen der Formel (I), in denen G für Cyano oder eine der Gruppierungen (a) bis (d) steht.
F) Cyclische Imine der Formel (I-f) 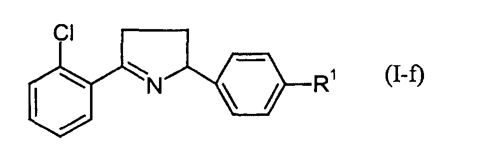 in welcher R¹ die oben angegebenen Bedeutungen hat, lassen sich auch herstellen, indem man im ersten Schritt
   α) O-Chloracetophenon der Formel (F-I) 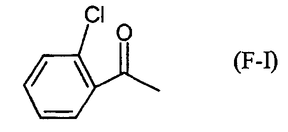 mit Dimethylmethylenammoniumchlorid der Formel (F-II) 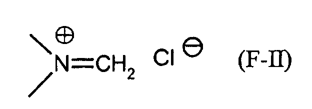 zur Verbindung der Formel (F-III) 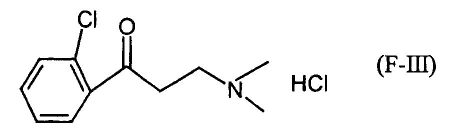 umsetzt und diese in einem zweiten Schritt
   β) mit Benzylcyaniden der Formel (F-IV) 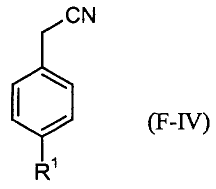 in denen R¹ die oben angegebenen Bedeutung hat, zu Verbindungen der Formel (F-V) 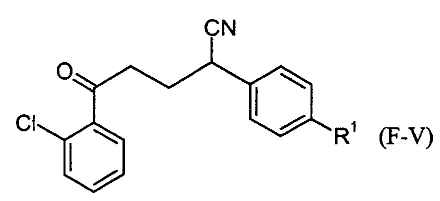 umsetzt, welche im nächsten Schritt
   δ) mit Natronlauge/H₂O₂ zu Verbindungen der Formel (F-VI) 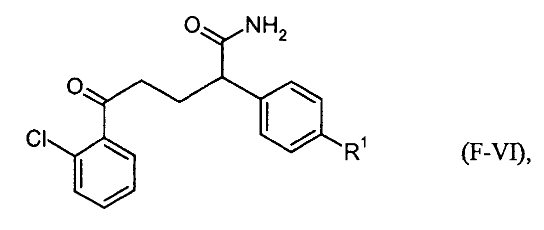 in welcher R¹ die oben angegebenen Bedeutungen besitzt, derivatisiert, und diese in einem abschließenden Schritt
   γ) durch Umsetzung mit PIFA (1,1-Bis(Trifluoroacetoxy)iodbenzol) der Formel (F-VII) 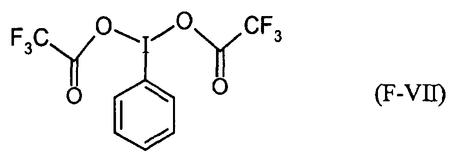 zu cyclischen Iminen der Formel (I-f) cyclisiert.
G) Cyclische Imine der Formel (I) 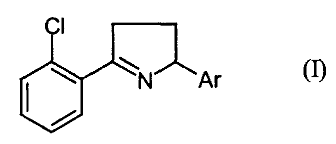 in welcher Ar die oben angegebenen Bedeutungen hat, lassen sich auch herstellen, indem man im ersten Schritt
   α) Arylbutyrolactame der Formel (XI) 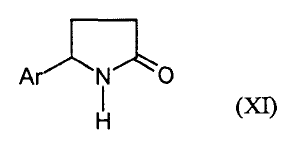 mit o-Chlorbenzoesäurechlorid 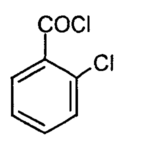 zu Verbindungen der Formel G(I) 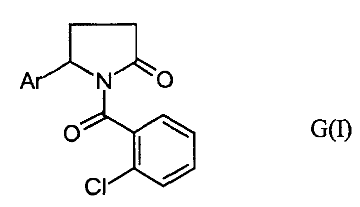 umsetzt, und diese in einem zweiten Schritt
   β) mit o-Chlorbenzoesäuremethylester 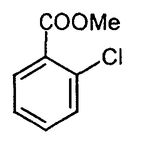 zu Verbindungen der Formel G(II) 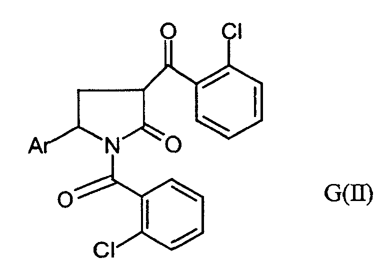 umsetzt, welche in einem abschließenden Schritt
   δ) mit HBr/Eisessig zu Verbindungen der Formel (I) 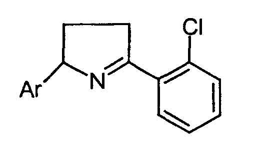 umgesetzt werden.

Weiterhin wurde gefunden, daß die neuen Verbindungen der Formel (I) bei guter Pflanzenverträglichkeit eine sehr gute Wirksamkeit als Schädlingsbekämpfungsmittel, insbesondere gegen Arthropoden in der Landwirtschaft aber auch gegen Parasiten in der Nutz- und Hobbytierhaltung aufweisen.

Die erfindungsgemäßen Verbindungen sind durch die Formel (I) allgemein definiert. Bevorzugte Substituenten bzw. Bereiche der in den oben und nachstehend erwähnten Formeln aufgeführten Reste werden im folgenden erläutert.
- Ar: steht für den Rest 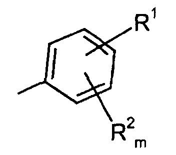
- m: steht bevorzugt für 0, 1 oder 2.
- R¹: steht bevorzugt für einen Substituenten in meta- oder para-Position aus der Reihe Wasserstoff, Fluor, Chlor, Brom, Iod, Cyano, Tri-(C₁-C₄-alkyl)-silyl, - CO-NR⁴R⁵, Tetrahydropyranyl oder eine der folgenden Gruppierungen

(l) - X - A

(m) - B - Z - D

(n) -Y-E .
- R²: steht bevorzugt für Wasserstoff, Fluor, Chlor, Brom, Iod, Cyano, Nitro, C₁-C₁₆-Alkyl, C₁-C₁₆-Alkoxy, jeweils durch Fluor oder Chlor substituiertes C₁-C₆-Alkyl oder C₁-C₆-Alkoxy, für C₁-C₈-Alkoxy-C₁-C₈-alkoxy, oder -S(O)ₒR³.
- o: steht bevorzugt für 0, 1 oder 2.
- R³: steht bevorzugt für C₁-C₄-Alkyl oder jeweils durch Fluor oder Chlor substituiertes Methyl oder Ethyl.
- R⁴ und R⁵: stehen unabhängig voneinander bevorzugt für Wasserstoff, C₁-C₆-Alkyl, durch Fluor oder Chlor substituiertes C₁-C₆-Alkyl oder für jeweils gegebenenfalls einfach oder zweifach durch Reste aus der Liste W¹ substituiertes Phenyl oder Benzyl.
- X: steht bevorzugt für eine direkte Bindung, Sauerstoff, Schwefel, Carbonyl, Carbonyloxy, Oxycarbonyl, C₁-C₄-Alkylen, C₂-C₄-Alkenylen, C₂-C₄-Alkinylen, C₁-C₄-Alkylenoxy, C₁-C₄-Oxyalkylen, C₁-C₄-Thioalkylen, C₁-C₄-Alkylendioxy oder Di-C₁-C₄-alkylsilylen.
- A: steht bevorzugt für jeweils gegebenenfalls einfach bis dreifach durch Reste aus der Liste W¹ substituiertes Phenyl, Naphthyl oder Tetrahydronaphthyl oder für jeweils gegebenenfalls einfach bis dreifach durch Reste aus der Liste W² substituiertes 5- bis 10-gliedriges, 1 oder 2 aromatische Ringe enthaltendes Heterocyclyl mit 1 bis 4 Heteroatomen, welches 0 bis 4 Stickstoffatome, 0 bis 2 Sauerstoffatome und 0 bis 2 Schwefelatome enthält, (insbesondere Furyl, Benzofuryl, Thienyl, Benzothienyl, Oxazolyl, Benzoxazolyl, Thiazolyl, Benzthiazolyl, Pyrrolyl, Pyridyl, Pyrimidyl, 1,3,5-Triazinyl, Chinolinyl, Isochinolinyl, Indolyl, Purinyl, Benzodioxolyl, Indanyl, Benzodioxanyl oder Chromanyl).
- B: steht bevorzugt für gegebenenfalls einfach oder zweifach durch Reste aus der Liste W¹ substituiertes p-Phenylen.
- Z: steht bevorzugt für Sauerstoff oder Schwefel.
- D: steht bevorzugt für Wasserstoff, C₁-C₁₆-Alkyl, C₂-C₁₆-Alkenyl, C₂-C₆-Alkinyl, jeweils durch Fluor oder Chlor substituiertes C₁-C₄-Alkyl oder C₂-C₄-Alkenyl, für jeweils gegebenenfalls durch Fluor, Chlor, Brom, C₁-C₄-Alkyl, C₂-C₄-Alkenyl, durch Fluor oder Chlor substituiertes C₂-C₄-Alkenyl, durch Phenyl, Styryl, jeweils durch Fluor, Chlor oder Brom substituiertes Phenyl oder Styryl substituiertes C₃-C₆-Cycloalkyl oder C₃-C₆-Cycloalkyl-C₁-C₄-alkyl, für jeweils gegebenenfalls durch Fluor, Chlor, Brom oder C₁-C₄-Alkyl substituiertes C₅-C₆-Cycloalkenyl oder C₅-C₆-Cycloalkenyl-C₁-C₄-alkyl, für jeweils gegebenenfalls durch Nitro, Fluor, Chlor, Brom, C₁-C₆-Alkyl, C₁-C₆-Alkoxy, jeweils durch Fluor oder Chlor substituiertes C₁-C₄-Alkyl oder C₁-C₄-Alkoxy substituiertes Phenyl-C₁-C₄-alkyl, Naphthyl-C₁-C₄-alkyl, Tetrahydronaphthyl-C₁-C₆-alkyl oder 5- oder 6-ringgliedriges Hetaryl-C₁-C₄-alkyl mit 1 oder 2 Heteroatomen aus der Reihe Stickstoff, Sauerstoff und Schwefel, insbesondere - Furylmethyl, Thienylmethyl, Pyrrolylmethyl, Oxazolylmethyl, Isoxazolylmethyl, Thioazolylmethyl oder Pyridylmethyl) für -CO-R⁶, -CO-NR⁷R⁸ oder die Gruppierung

- (CH₂)ₚ - (CR⁹R¹⁰)_{q} - (CH₂)ᵣ - G .
- Z und D: stehen auch bevorzugt gemeinsam für gegebenenfalls durch Nitro, Fluor, Chlor, Brom, C₁-C₄-Alkyl, C₁-C₄-Alkoxy, jeweils durch Fluor oder Chlor substituiertes C₁-C₄-Alkyl oder C₁-C₄-Alkoxy substituiertes Phenoxy-C₁-C₃-alkyl.
- Y: steht bevorzugt für eine direkte Bindung, Sauerstoff, Schwefel, Carbonyl, Carbonyloxy, Oxycarbonyl, C₁-C₄-Alkylen, C₂-C₄-Alkenylen, C₂-C₄-Alkinylen, C₁-C₄-Alkylenoxy, C₁-C₄-Oxyalkylen, C₁-C₄-Thioalkylen, C₁-C₄-Alkylendioxy oder für gegebenenfalls einfach oder zweifach durch Reste aus der Liste W¹ substituiertes p-Phenylen.
- E: steht bevorzugt für Wasserstoff, C₁-C₁₆-Alkyl, C₂-C₁₆-Alkenyl, C₂-C₆-Alkinyl, jeweils durch Fluor oder Chlor substituiertes C₁-C₄-Alkyl oder C₂-C₄-Alkenyl, für gegebenenfalls durch Fluor, Chlor, Brom, C₁-C₄-Alkyl, C₂-C₄-Alkenyl, durch Fluor oder Chlor substituiertes C₂-C₄-Alkenyl, durch Phenyl, Styryl oder jeweils durch Fluor, Chlor oder Brom substituiertes Phenyl oder Styryl substituiertes C₃-C₆-Cycloalkyl, für gegebenenfalls durch Fluor, Chlor, Brom oder C₁-C₄-Alkyl substituiertes C₅-C₆-Cycloalkenyl, für gegebenenfalls einfach bis dreifach durch Reste aus der Liste W¹ substituiertes Phenyl oder für jeweils gegebenenfalls einfach oder zweifach durch Reste aus der Liste W² substituiertes 5- oder 6-gliedriges Hetaryl mit 1 oder 2 Heteroatomen aus der Reihe Stickstoff, Sauerstoff und Schwefel (insbesondere Furyl, Thienyl, Pyrrolyl, Oxazolyl, Isoxazolyl, Thiazolyl oder Pyridyl) oder für die Gruppierung

- (CH₂)ₚ - (CR⁹R¹⁰)_{q} - (CH₂)ᵣ - G .
- R⁶: steht bevorzugt für C₁-C₆-Alkyl, C₁-C₆-Alkoxy, C₂-C₆-Alkenyl, C₂-C₆-Alkenyloxy, jeweils gegebenenfalls durch Fluor, Chlor, C₁-C₃-Alkyl oder jeweils durch Fluor oder Chlor substituiertes C₁-C₂-Alkyl oder C₂-C₃-Alkenyl substituiertes C₃-C₆-Cycloalkyl, C₃-C₆-Cycloalkyloxy oder C₃-C₆-Cycloalkyl-C₁-C₂-alkyloxy oder für gegebenenfalls einfach oder zweifach durch Fluor, Chlor, Brom, Iod, C₁-C₄-Alkyl, C₁-C₄-Alkoxy oder jeweils durch Fluor oder Chlor substituiertes C₁-C₃-Alkyl oder C₁-C₄-Alkoxy substituiertes Phenyl.
- R⁷: steht bevorzugt für Wasserstoff oder C₁-C₄-Alkyl.
- R⁸: steht bevorzugt für C₁-C₄-Alkyl oder für jeweils gegebenenfalls einfach oder zweifach durch Fluor, Chlor, Brom, C₁-C₄-Alkyl oder jeweils durch Fluor oder Chlor substituiertes C₁-C₄-Alkyl oder C₁-C₄-Alkoxy substituiertes Phenyl oder Benzyl.
- p, q und r: stehen bevorzugt unabhängig voneinander für 0, 1, 2 oder 3, wobei deren Summe kleiner 6 ist.
- R⁹ und R¹⁰: stehen unabhängig voneinander bevorzugt für Wasserstoff oder C₁-C₄-Alkyl.
- G: steht bevorzugt für Cyano, für einen gegebenenfalls einfach bis dreifach durch Fluor, Chlor, Brom, C₁-C₄-Alkyl oder durch Fluor oder Chlor substituiertes C₁-C₄-Alkyl und gegebenenfalls an der Verknüpfungsstelle durch den Rest R¹¹ substituierten 5- oder 6-gliedrigen Heterocyclus mit 1 bis 3 gleichen oder verschiedenen Heteroatomen aus der Reihe Stickstoff, Sauerstoff und Schwefel (insbesondere 5,6-Dihydrodioxazin-2-yl, 3-Pyridyl, 3-Furyl, 3-Thienyl, 2-Thiazolyl, 5-Thiazolyl, 2-Dioxolanyl, 1,3-Dioxan-2-yl, 2-Dithiolanyl, 1,3-Dithian-2-yl oder 1,3-Thioxan-2-yl) oder eine der folgenden Gruppierungen:

(a) ―CO―R¹¹

(b) ―CO―OR¹²

(c) ―CO―NR¹³R¹⁴

(d) ―CS―NR¹³R¹⁴ 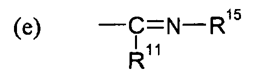 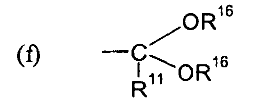 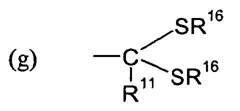 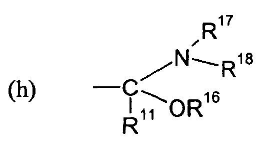 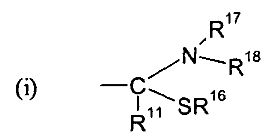 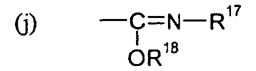 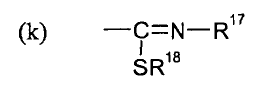
- R¹¹: steht bevorzugt für Wasserstoff, C₁-C₆-Alkyl, C₂-C₆-Alkenyl, jeweils durch Fluor oder Chlor substituiertes C₁-C₄-Alkyl oder C₂-C₆-Alkenyl, gegebenenfalls durch Fluor, Chlor, C₁-C₄-Alkyl oder durch Fluor oder Chlor substituiertes C₁-C₄-Alkyl substituiertes C₃-C₆-Cycloalkyl oder für gegebenenfalls einfach bis dreifach durch C₁-C₄-Alkylcarbonylamino, C₁-C₄-Alkylcarbonyl-C₁-C₄-alkylamino und/oder Reste aus der Liste W³ substituiertes Phenyl.
- R¹²: steht bevorzugt für Wasserstoff, C₁-C₄-Alkyl, C₃-C₆-Alkenyl, jeweils durch Fluor oder Chlor substituiertes C₁-C₄-Alkyl oder C₃-C₆-Alkenyl, jeweils gegebenenfalls durch Fluor, Chlor, C₁-C₄-Alkyl oder durch Fluor oder Chlor substituiertes C₁-C₄-Alkyl substituiertes C₃-C₆-Cycloalkyl oder C₃-C₆-Cycloalkyl-C₁-C₄-alkyl oder für jeweils gegebenenfalls einfach bis dreifach durch Reste aus der Liste W³ substituiertes Phenyl-C₁-C₄-alkyl oder Naphthyl-C₁-C₄-alkyl.
- R¹³ und R¹⁴: stehen unabhängig voneinander bevorzugt für Wasserstoff, C₁-C₄-Alkyl, C₃-C₆-Alkenyl, jeweils durch Fluor oder Chlor substituiertes C₁-C₄-Alkyl oder C₃-C₆-Alkenyl, für C₁-C₄-Alkoxy, jeweils gegebenenfalls durch Fluor, Chlor, C₁-C₄-Alkyl oder durch Fluor oder Chlor substituiertes C₁-C₄-Alkyl substituiertes C₃-C₆-Cycloalkyl oder C₃-C₆-Cycloalkyl-C₁-C₄-alkyl, für jeweils gegebenenfalls einfach bis dreifach durch Reste aus der Liste W³ substituiertes Phenyl oder Phenyl-C₁-C₄-alkyl, für -OR¹² oder -NR¹¹R¹² oder gemeinsam für -(CH₂)₅-, -(CH₂)₆- oder -(CH₂)₂-O-(CH₂)₂-
- R¹⁵: steht bevorzugt für -OR¹², -NR¹¹R¹² oder -N(R¹¹)-COOR¹².
- R¹⁶, R¹⁷ und R¹⁸: stehen unabhängig voneinander bevorzugt für C₁-C₄-Alkyl.
- W¹: steht bevorzugt für Wasserstoff, Fluor, Chlor, Brom, Iod, Cyano, Formyl, Nitro, C₁-C₄-Alkyl, C₁-C₄-Alkoxy, jeweils durch Fluor oder Chlor substituiertes C₁-C₄-Alkyl oder C₁-C₄-Alkoxy, für C₁-C₄-Alkylcarbonyl, C₁-C₄-Alkoxycarbonyl oder -S(O)ₒR³.
- W²: steht bevorzugt für Fluor, Chlor, Brom, Cyano, Formyl, Nitro, C₁-C₄-Alkyl, C₁-C₄-Alkoxy, jeweils durch Fluor oder Chlor substituiertes C₁-C₄-Alkyl oder C₁-C₄-Alkoxy, für C₁-C₄-Alkylcarbonyl, C₁-C₄-Alkoxycarbonyl, -S(O)ₒR³ oder -C(R¹¹)=N-R¹⁵.
- W³: steht bevorzugt für Fluor, Chlor, Brom, Cyano, Nitro, C₁-C₄-Alkyl, C₁-C₄-Alkoxy, jeweils durch Fluor oder Chlor substituiertes C₁-C₄-Alkyl oder C₁-C₄-Alkoxy, für Di-C₁-C₄-alkylamino, -S(O)ₒR³, -COOR¹⁹ oder -CONR²⁰R²¹.
- R¹⁹: steht bevorzugt für Wasserstoff, C₁-C₄-Alkyl, durch Fluor oder Chlor substituiertes C₁-C₄-Alkyl, gegebenenfalls durch Fluor, Chlor, C₁-C₄-Alkyl oder durch Fluor oder Chlor substituiertes C₁-C₄-Alkyl substituiertes C₃-C₆-Cycloalkyl oder für gegebenenfalls einfach bis dreifach durch Reste aus der Liste W⁴ substituiertes Phenyl.
- R²⁰ und R²¹: stehen unabhängig voneinander bevorzugt für Wasserstoff, C₁-C₄-Alkyl, C₃-C₆-Alkenyl, jeweils durch Fluor oder Chlor substituiertes C₁-C₄-Alkyl oder C₃-C₆-Alkenyl, für C₁-C₄-Alkoxy, jeweils gegebenenfalls durch Fluor, Chlor, C₁-C₄-Alkyl oder durch Fluor oder Chlor substituiertes C₁-C₄-Alkyl substituiertes C₃-C₆-Cycloalkyl oder C₃-C₆-Cycloalkyl-C₁-C₄-alkyl oder für jeweils gegebenenfalls einfach bis dreifach durch Reste aus der Liste W⁴ substituiertes Phenyl oder Phenyl-C₁-C₄-alkyl, für -OR¹⁶ oder -NR¹⁷R¹⁸ oder gemeinsam für -(CH₂)₅-, -(CH₂)₆- oder -(CH₂)₂-O-(CH₂)₂-.
- W⁴: steht bevorzugt für Fluor, Chlor, Brom, Cyano, Nitro, C₁-C₄-Alkyl, C₁-C₄-Alkoxy, jeweils durch Fluor oder Chlor substituiertes C₁-C₄-Alkyl oder C₁-C₄-Alkoxy, Di-C₁-C₄-alkylamino, C₁-C₄-Alkoxycarbonyl, Di-C₁-C₆-alkylaminocarbonyl oder -S(O)ₒR³.
- Ar: steht besonders bevorzugt für den Rest 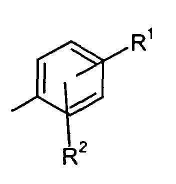
- R¹: steht besonders bevorzugt für einen Substituenten in meta- oder para-Position aus der Reihe Wasserstoff, Fluor, Chlor, Brom, Iod, Cyano, -CO-NR⁴R⁵, Tetrahydropyranyl oder eine der folgenden Gruppierungen

(l) - X - A

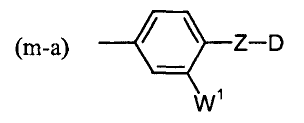

(n) - Y - E .
- R²: steht besonders bevorzugt für Wasserstoff, Fluor, Chlor, Brom, Methyl, Ethyl, Methoxy, Ethoxy, Methylthio, Ethylthio, Trifluormethyl, Difluormethoxy, Trifluormethoxy oder Trifluormethylthio.
- o: steht besonders bevorzugt für 0 oder 2.
- R³: steht besonders bevorzugt für Methyl, Ethyl, n-Propyl, Isopropyl, Difluormethyl oder Trifluormethyl.
- R⁴ und R⁵: stehen unabhängig voneinander besonders bevorzugt für Wasserstoff, Methyl, Ethyl, n-Propyl, Isopropyl, n-Butyl, Isobutyl, sec.-Butyl, tert.-Butyl oder für jeweils gegebenenfalls einfach durch einen Rest aus der Liste W¹ substituiertes Phenyl oder Benzyl.
- X: steht besonders bevorzugt für eine direkte Bindung, Sauerstoff, Schwefel, Carbonyl, -CH₂-, -(CH₂)₂-, -CH=CH- (E oder Z), -C≡C-, -CH₂O-, -(CH₂)₂O-, -CH(CH₃)O-, -OCH₂-, -O(CH₂)₂-, -SCH₂-, -S(CH₂)₂-, -SCH(CH₃)-, C₁-C₄-Alkylendioxy, insbesondere -OCH₂O-, -O(CH₂)₂O- oder -OCH(CH₃)O-.
- A: steht besonders bevorzugt für gegebenenfalls einfach oder zweifach durch Reste aus der Liste W¹ substituiertes Phenyl oder für jeweils gegebenenfalls einfach oder zweifach durch Reste aus der Liste W² substituiertes Furyl, Benzofuryl, Thienyl, Benzothienyl, Oxazolyl, Benzoxazolyl, Thiazolyl, Benzthiazolyl, Pyrrolyl, Pyridyl, Pyrimidyl, 1,3,5-Triazinyl, Chinolinyl, Isochinolinyl, Indolyl, Purinyl, Benzodioxolyl, Indanyl, Benzodioxanyl oder Chromanyl.
- Z: steht besonders bevorzugt für Sauerstoff oder Schwefel.
- D: steht besonders bevorzugt für Wasserstoff, Methyl, Ethyl, n-Propyl, Isopropyl, n-Butyl, Isobutyl, sec.-Butyl, tert.-Butyl, die isomeren Pentyle, die isomeren Hexyle, n-Heptyl, n-Octyl, n-Isooctyl, n-Nonyl, n-Decyl, n-Undecyl, n-Dodecyl, n-Tridecyl, n-Tetradecyl, n-Pentadecyl, n-Hexadecyl, 2-Propenyl, Butenyl, Pentenyl, Hexenyl, Propargyl, Butinyl, Pentinyl, -CF₃, -CHF₂, -CClF₂, -CF₂CHFCl, -CF₂CH₂F, -CF₂CHF₂, -CF₂CCl₃, -CH₂CF₃, -CF₂CHFCF₃, -CH₂CF₂CHF₂, -CH₂CF₂CF₃, für jeweils gegebenenfalls einfach bis dreifach durch Fluor, Chlor, Brom, Methyl, Ethyl, n-Propyl, Isopropyl, n-Butyl, Isobutyl, sec.-Butyl, tert.-Butyl, Ethenyl, 1-Propenyl, 2,2-Dimethylethenyl, -CH=CCl₂, Phenyl, Styryl, jeweils durch Fluor, Chlor oder Brom substituiertes Phenyl oder 4-Chlorstyryl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, für jeweils gegebenenfalls durch Fluor, Chlor, Methyl, Ethyl, n-Propyl, Isopropyl, n-Butyl, Isobutyl, sec.-Butyl oder tert.-Butyl substituiertes Cyclopentenyl, Cyclohexenyl, Cyclohexenylmethyl oder Cyclopentenylmethyl, für jeweils gegebenenfalls einfach oder zweifach durch Nitro, Fluor, Chlor, Brom, Methyl, Ethyl, n-Propyl, Isopropyl, n-Butyl, Isobutyl, sec.-Butyl, tert.-Butyl, Methoxy, Ethoxy, n-Propoxy, Isopropoxy, n-Butoxy, Isobutoxy, sec.-Butoxy, tert.-Butoxy, Trifluormethyl, Trifluormethoxy, Difluormethoxy oder Chlordifluormethoxy substituiertes Benzyl, Phenethyl, Naphthylmethyl, Tetrahydronaphthylmethyl, Furylmethyl, Thienylmethyl, Pyrrolylmethyl, Oxazolylmethyl, Isoxazolylmethyl, Thiazolylmethyl oder Pyridylmethyl, für -CO-R⁶, -CO-NR⁷R⁸ oder die Gruppierung

- (CH₂)ₚ - (CR⁹R¹⁰)_{q} - (CH₂)ᵣ - G .
- Z und D: stehen auch besonders bevorzugt gemeinsam für gegebenenfalls einfach oder zweifach durch Nitro, Fluor, Chlor, Brom, Methyl, Ethyl, n-Propyl, i-Propyl, Methoxy, Ethoxy, n-Propoxy, i-Propoxy, Trifluormethyl, Trifluormethoxy, Difluormethoxy oder Chlordifluormethoxy substituiertes Phenoxymethyl.
- Y: steht besonders bevorzugt für eine direkte Bindung, Sauerstoff, Schwefel, Carbonyl, -CH₂-, -(CH₂)₂-, -CH=CH- (E oder Z), -C C-, -CH₂O-, -(CH₂)₂O-, -CH(CH₃)O-, -OCH₂-, -O(CH₂)₂-, -SCH₂-, -S(CH₂)₂-, -SCH(CH₃)-, C₁-C₄-Alkylendioxy, insbesondere -OCH₂O- oder -O(CH₂)₂O- oder für gegebenenfalls einfach durch einen Rest aus der Liste W¹ substituiertes p-Phenylen.
- E: steht besonders bevorzugt für Wasserstoff, Methyl, Ethyl, n-Propyl, Isopropyl, n-Butyl, Isobutyl, sec.-Butyl, tert.-Butyl, die isomeren Pentyle, die isomeren Hexyle, n-Heptyl, n-Octyl, n-Isooctyl, n-Nonyl, n-Decyl, n-Undecyl, n-Dodecyl, n-Tridecyl, n-Tetradecyl, n-Pentadecyl, n-Hexadecyl, 2-Propenyl, Butenyl, Pentenyl, Hexenyl, Propargyl, Butinyl, Pentinyl, -CF₃, -CHF₂, -CClF₂, -CF₂CHFCl, -CF₂CH₂F, -CF₂CHF₂, -CF₂CCl₃, -CH₂CF₃, -CF₂CHFCF₃, -CH₂CF₂CHF₂, -CH₂CF₂CF₃, für jeweils gegebenenfalls einfach bis dreifach durch Fluor, Chlor, Brom, Methyl, Ethyl, n-Propyl, Isopropyl, n-Butyl, Isobutyl, sec.-Butyl, tert.-Butyl, Ethenyl, 1-Propenyl, 2,2-Dimethylethenyl, -CH=CCl₂, Phenyl, Styryl, jeweils durch Fluor, Chlor oder Brom substituiertes Phenyl oder durch 4-Chlorstyryl substituiertes Cyclopropyl, Cyclobutyl, Cyclopentyl oder Cyclohexyl, für jeweils gegebenenfalls durch Fluor, Chlor, Methyl, Ethyl, n-Propyl, Isopropyl, n-Butyl, Isobutyl, sec.-Butyl oder tert.-Butyl substituiertes Cyclopentenyl oder Cyclohexenyl, für gegebenenfalls einfach oder zweifach durch Reste aus der Liste W¹ substituiertes Phenyl, für jeweils gegebenenfalls einfach oder zweifach durch Reste aus der Liste W² substituiertes Furyl, Thienyl, Pyrrolyl, Oxazolyl, Isoxazolyl, Thiazolyl oder Pyridyl, oder für die Gruppierung

- (CH₂)ₚ - (CR⁹R¹⁰)_{q} - (CH₂)ᵣ - G.
- R⁶: steht besonders bevorzugt für Methyl, Ethyl, n-Propyl, Isopropyl, n-Butyl, Isobutyl, sec.-Butyl, tert.-Butyl, Methoxy, Ethoxy, n-Propoxy, Isopropoxy, n-Butoxy, Isobutoxy, sec.-Butoxy, tert.-Butoxy, Cyclopropyl, Cyclohexyl, Cyclohexyloxy, Cyclohexylmethyloxy, Phenyl, 2-Chlorphenyl, 3-Chlorphenyl, 2,6-Difluorphenyl, 2,4-Dichlorphenyl, 3,4-Dichlorphenyl, 2-Trifluormethoxyphenyl oder 4-Trifluormethoxyphenyl.
- R⁷: steht besonders bevorzugt für Wasserstoff.
- R⁸: steht besonders bevorzugt für Methyl, Ethyl oder gegebenenfalls einfach durch Chlor substituiertes Phenyl.
- p, q und r: stehen besonders bevorzugt unabhängig voneinander für 0, 1, 2 oder 3, wobei deren Summe kleiner 4 ist.
- R⁹ und R¹⁰: stehen unabhängig voneinander besonders bevorzugt für Wasserstoff, Methyl, Ethyl, n-Propyl, Isopropyl, n-Butyl, Isobutyl, sec.-Butyl, tert.-Butyl.
- G: steht besonders bevorzugt für Cyano, für jeweils gegebenenfalls einfach bis dreifach durch Fluor, Chlor, Brom, Methyl, Ethyl, n-Propyl, Isopropyl oder Trifluormethyl und gegebenenfalls an der Verknüpfungsstelle durch den Rest R¹¹ substituiertes 5,6-Dihydrodioxazin-2-yl, 3-Pyridyl, 3-Furyl, 3-Thienyl, 2-Thiazolyl, 5-Thiazolyl, 2-Dioxolanyl, 1,3-Dioxan-2-yl, 2-Dithiolanyl, 1,3-Dithian-2-yl oder 1,3-Thioxan-2-yl oder eine der folgenden Gruppierungen:

(a) ―CO―R¹¹

(b) ―CO―OR¹²

(c) ―CO―NR¹³R¹⁴

(d) ―CS―NR¹³R¹⁴ 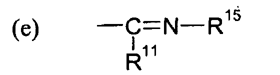 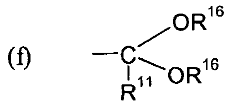 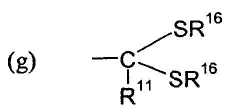 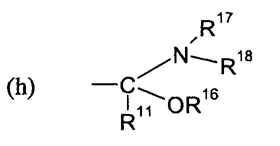 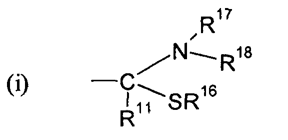
- R¹¹: steht besonders bevorzugt für Wasserstoff, Methyl, Ethyl, n-Propyl, Isopropyl, n-Butyl, Isobutyl, sec.-Butyl, tert.-Butyl, die isomeren Pentyle, die isomeren Hexyle -CF₃, -CHF₂, -CClF₂, -CF₂CHFCl, -CF₂CH₂F, -CF₂CHF₂, -CF₂CCl₃, -CH₂CF₃, C₃-C₆-Alkenyl, einfach bis dreifach durch Fluor oder Chlor substituiertes C₃-C₆-Alkenyl, für jeweils gegebenenfalls einfach oder zweifach durch Fluor, Chlor, Methyl, Ethyl, n-Propyl, Isopropyl, -CF₃, -CHF₂, -CClF₂, -CF₂CHFCl, -CF₂CH₂F, -CF₂CHF₂, -CF₂CCl₃ oder -CH₂CF₃ substituiertes Cyclopropyl, Cyclopentyl oder Cyclohexyl oder für gegebenenfalls einfach oder zweifach durch Methylcarbonylamino, Ethylcarbonylamino, Methylcarbonyl-methylamino und/oder Reste aus der Liste W³ substituiertes Phenyl.
- R¹²: steht besonders bevorzugt für Wasserstoff, Methyl, Ethyl, n-Propyl, Isopropyl, n-Butyl, Isobutyl, sec.-Butyl, tert.-Butyl, -CH₂CF₃, Allyl, jeweils gegebenenfalls einfach oder zweifach durch Fluor, Chlor, Methyl, Ethyl, n-Propyl, Isopropyl -CF₃, -CHF₂, -CClF₂, -CF₂CHFCl, -CF₂CH₂F, -CF₂CHF₂, -CF₂CCl₃ oder -CH₂CF₃ substituiertes Cyclopropyl, Cyclopentyl, Cyclohexyl, Cyclopropylmethyl, Cyclopentylmethyl, Cyclohexylmethyl, Cyclopropylethyl, Cyclopentylethyl oder Cyclohexylethyl oder für jeweils gegebenenfalls einfach oder zweifach durch Reste aus der Liste W³ substituiertes Benzyl oder Phenethyl.
- R¹³ und R¹⁴: stehen unabhängig voneinander besonders bevorzugt für Wasserstoff, Methyl, Ethyl, n-Propyl, Isopropyl, n-Butyl, Isobutyl, sec.-Butyl, tert.-Butyl, - CH₂CF₃, Methoxy, Ethoxy, Allyl, für jeweils gegebenenfalls einfach oder zweifach durch Fluor, Chlor, Methyl, Ethyl, n-Propyl, Isopropyl oder Trifluormethyl substituiertes Cyclopropyl, Cyclopentyl, Cyclohexyl, Cyclopropylmethyl, Cyclopentylmethyl oder Cyclohexylmethyl, für jeweils gegebenenfalls einfach oder zweifach durch Reste aus der Liste W³ substituiertes Phenyl, Benzyl oder Phenethyl, für -OR¹² oder -NR¹¹R¹².
- R¹⁵: steht besonders bevorzugt für -OR¹², -NR¹¹R¹² oder -N(R¹¹)-COOR¹².
- R¹⁶, R¹⁷ und R¹⁸: stehen unabhängig voneinander besonders bevorzugt für Methyl, Ethyl, n-Propyl oder Isopropyl.
- W¹: steht besonders bevorzugt für Wasserstoff, Fluor, Chlor, Brom, Cyano, Formyl, Nitro, Methyl, Ethyl, n-Propyl, Isopropyl, n-Butyl, Isobutyl, sec.-Butyl, tert.-Butyl, Methoxy, Ethoxy, n-Propoxy, Isopropoxy, n-Butoxy, Isobutoxy, sec.-Butoxy, tert.-Butoxy, -CF₃, -CHF₂, -CClF₂, -CF₂CHFCl, -CF₂CH₂F, -CF₂CHF₂, -CF₂CCl₃, -CH₂CF₃, -CF₂CHFCF₃, -CH₂CF₂CHF₂, -CH₂CF₂CF₃, Trifluormethoxy, Difluormethoxy, Chlordifluormethoxy, Acetyl, Propionyl, Butyryl, Isobutyryl, Methoxycarbonyl, Ethoxycarbonyl, n-Propoxycarbonyl, Isopropoxycarbonyl, n-Butoxycarbonyl, Isobutoxycarbonyl, sec.-Butoxycarbonyl, tert.-Butoxycarbonyl oder -S(O)ₒR³.
- W²: steht besonders bevorzugt für Fluor, Chlor, Brom, Cyano, Methyl, Ethyl, n-Propyl, Isopropyl, Trifluormethyl, Trifluormethoxy, Difluormethoxy, Chlordifluormethoxy, Acetyl, Trifluormethylthio, -CH=N-OCH₃, -CH=N-OC₂H₅, -CH=N-OC₃H₇, -C(CH₃)=N-OCH₃, -C(CH₃)=N-OC₂H₅, -C(CH₃)=N-OC₃H₇, -C(C₂H₅)=N-OCH₃, -C(C₂H₅)=N-OC₂H₅ oder -C(C₂H₅)=N-OC₃H₇.
- W³: steht besonders bevorzugt für Fluor, Chlor, Cyano, Nitro, Methyl, Ethyl, Methoxy, Ethoxy, Methylthio, Trifluormethyl, Trifluormethoxy, Trifluormethylthio, Dimethylamino, Diethylamino, -COOR¹⁹ oder -CONR²⁰R²¹.
- R¹⁹: steht besonders bevorzugt für Wasserstoff, Methyl, Ethyl, n-Propyl, Isopropyl, tert.-Butyl, -CH₂CF₃, für jeweils gegebenenfalls einfach oder zweifach durch Fluor, Chlor, Methyl, Ethyl, n-Propyl, Isopropyl oder -CF₃ substituiertes Cyclopropyl, Cyclopentyl oder Cyclohexyl oder für gegebenenfalls einfach oder zweifach durch Reste aus der Liste W⁴ substituiertes Phenyl.
- R²⁰ und R²¹: stehen unabhängig voneinander besonders bevorzugt für Wasserstoff, Methyl, Ethyl, n-Propyl, Isopropyl, n-Butyl, Isobutyl, sec.-Butyl, tert.-Butyl, - CH₂CF₃, Methoxy, Ethoxy, Allyl, für jeweils gegebenenfalls einfach oder zweifach durch Fluor oder Chlor substituiertes Cyclopropyl, Cyclopentyl, Cyclohexyl, Cyclopropylmethyl, Cyclopentylmethyl oder Cyclohexylmethyl, für jeweils gegebenenfalls einfach oder zweifach durch Reste aus der Liste W⁴ substituiertes Phenyl, Benzyl oder Phenethyl, für -OR¹⁶ oder -NR¹⁷R¹⁸.
- W⁴: steht besonders bevorzugt für Fluor, Chlor, Brom, Cyano, Nitro, Methyl, Ethyl, tert.-Butyl, Methoxy, Ethoxy, Methylthio, Trifluormethyl, Trifluormethoxy oder Trifluormethylthio.

Weiterhin bevorzugt sind Verbindungen der Formel (I-a) 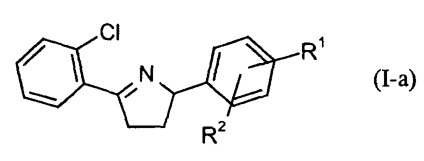 in welcher
- R²: die oben genannten allgemeinen, bevorzugten, besonders bevorzugten oder ganz besonders bevorzugten Bedeutungen hat,
- R¹: für Wasserstoff oder einfach oder zweifach durch Reste aus der Liste W¹ substituiertes Phenyl oder eine der folgenden Gruppierungen

(m-b) -B―O―D

(l) -Y―E

steht,
- B: für gegebenenfalls einfach durch einen Rest aus der Liste W¹ substituiertes p-Phenylen steht,
- Y: für eine direkte Bindung oder für gegebenenfalls einfach oder zweifach durch Reste aus der Liste W¹ substituiertes p-Phenylen steht und
- D und E: die oben genannten ganz besonders bevorzugten Bedeutungen haben, wobei
G für Cyano oder eine der folgenden Gruppierungen

(a) ―CO―R¹¹

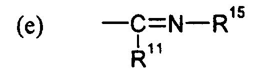

steht, in denen
R¹¹ und R¹⁵ die oben genannten allgemeinen, bevorzugten, besonders bevorzugten oder ganz besonders bevorzugten Bedeutungen haben und
- W¹: die obengenannte allgemeine, bevorzugte, besonders bevorzugte oder ganz besonders bevorzugte Bedeutung hat.

Weiterhin bevorzugt sind Verbindungen der Formel (I-f) 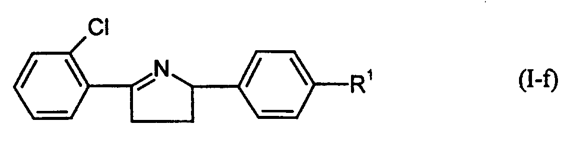 in welcher
- R¹: für Wasserstoff oder
a) einfach oder zweifach durch Reste aus der Liste W² substituiertes Phenyl oder
b) einfach oder zweifach durch Reste aus der Liste W² substituiertes Hetaryl (insbesondere Furyl, Thienyl, Pyrrolyl, Oxazolyl, Isoxazolyl, Thiazolyl oder Pyridyl, speziell Thienyl) steht.

Weiterhin bevorzugt sind Verbindungen der Formel (I-g) 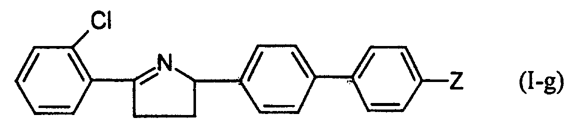 in welcher
- Z: für Wasserstoff, Fluor, Brom, Cyano, Methyl, Ethyl, n-Propyl, Isopropyl, Trifluormethyl, Trifluormethoxy, Difluormethoxy, Chlordifluormethoxy, Acetyl, Trifluormethylthio, -CH=N-OCH₃, -CH=N-OC₂H₅, -CH=N-OC₃H₇, -C(CH₃)=N-OCH₃, -C(CH₃)=N-OC₂H₅, -C(CH₃)=N-OCt3H₇, -C(C₂H₅)=N-OC₂H₅ oder - C(C₂H₅)=N-OC₃H₇ steht.

Weiterhin bevorzugt sind die in Tabelle 1 aufgeführten Verbindungen der Formel (I-f)

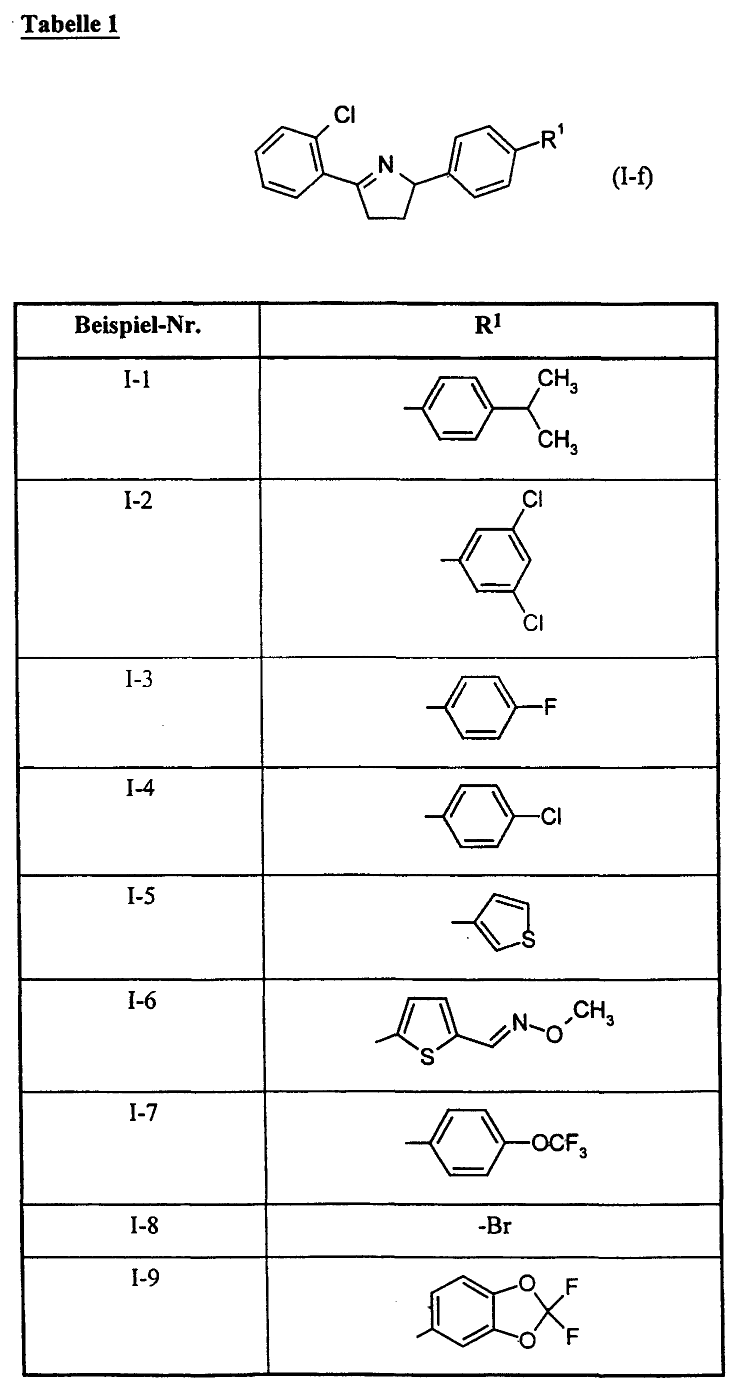

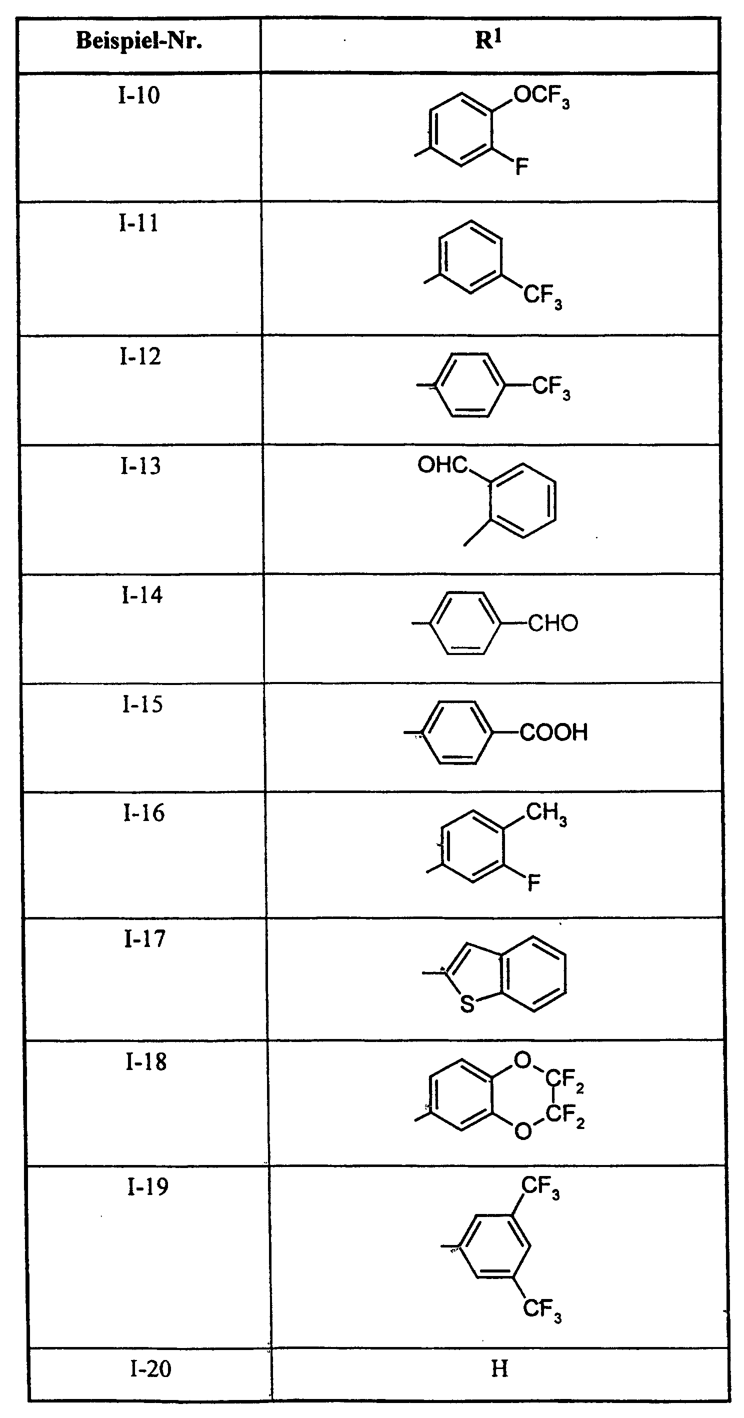

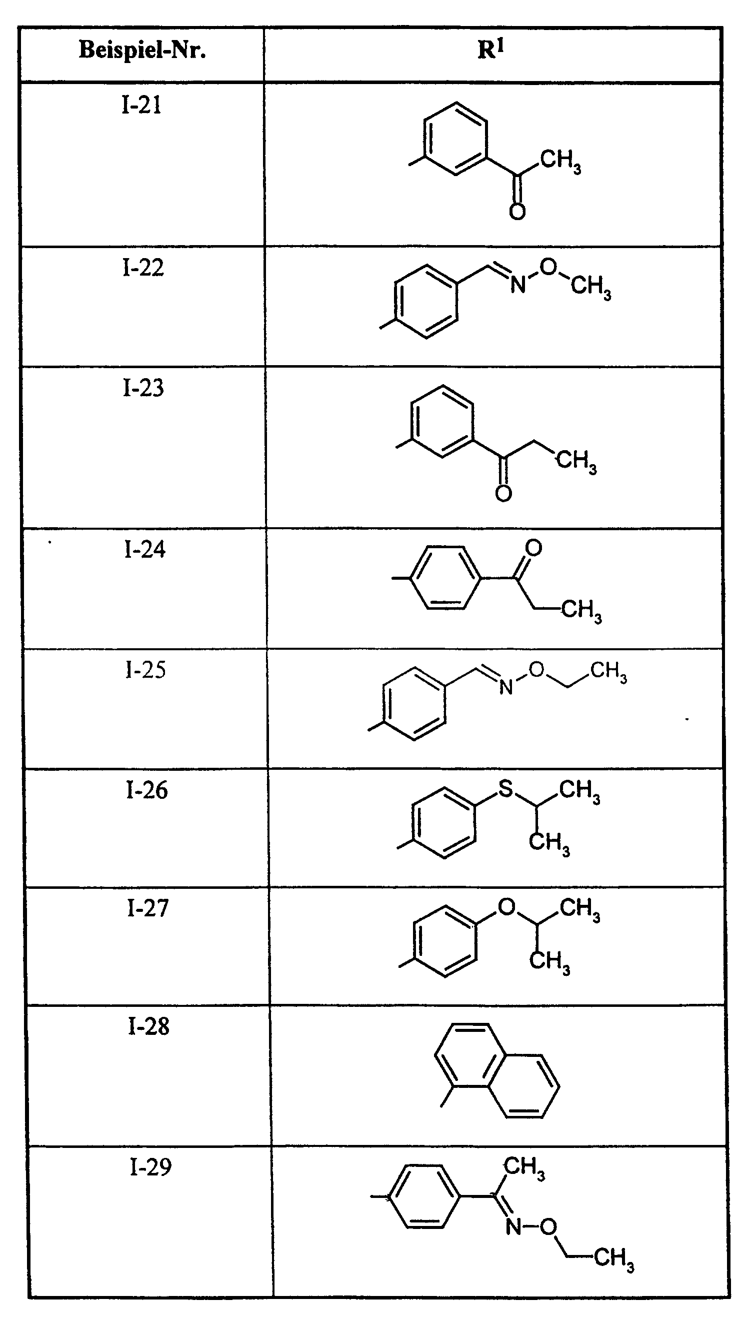

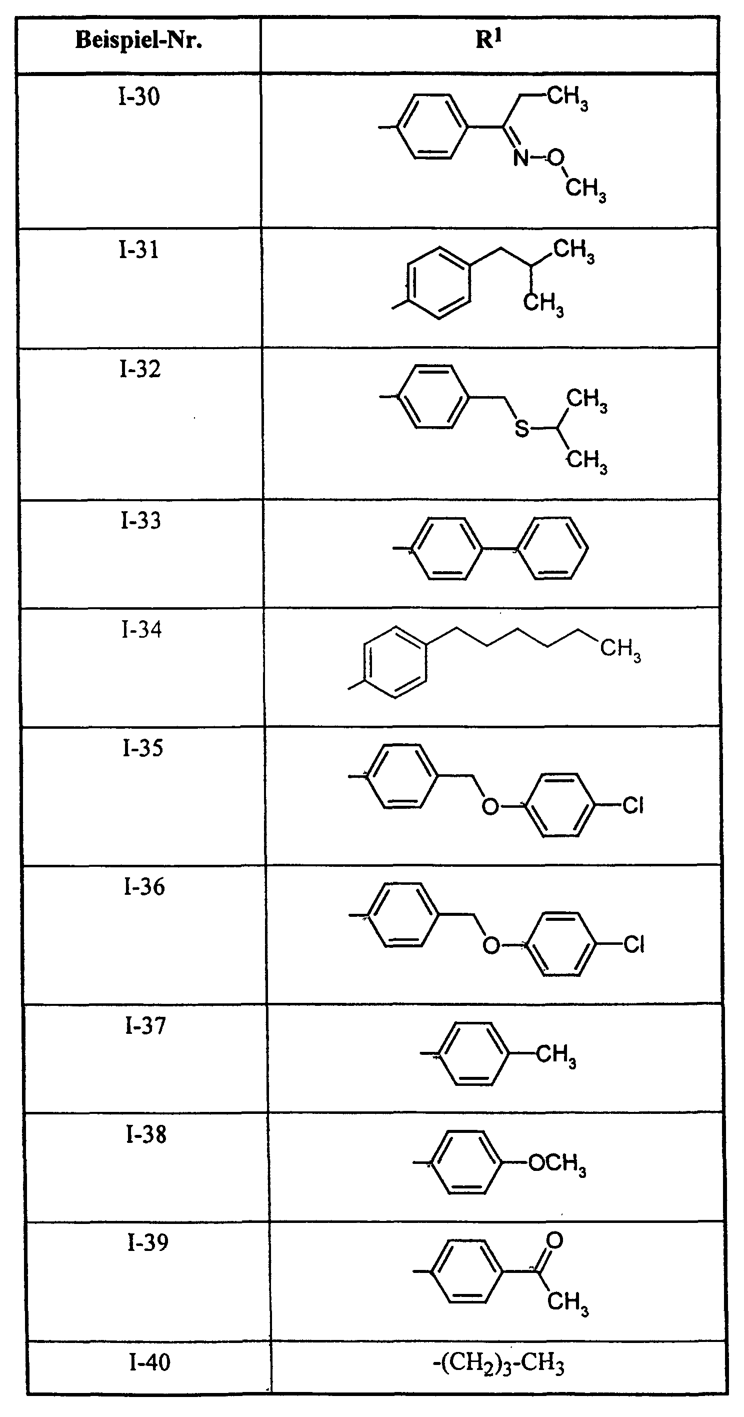

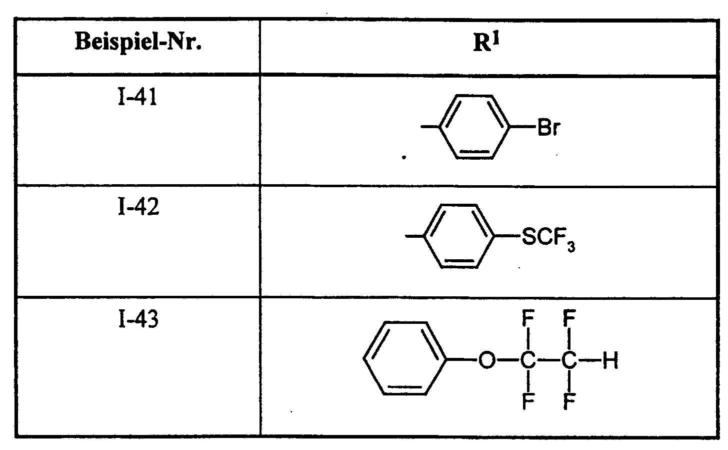

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können. Mehrere Reste mit denselben Indizes wie beispielsweise m Reste R² für m > 1, können gleich oder verschieden sein.

Verwendet man beispielsweise tBOC-[1-(4-Ethyl-2-Methyl-phenyl)-3-(2-chlor-phenylcarboxyl)-1-propyl]-amin als Ausgangsstoff, so kann der Reaktionsablauf des erfindungsgemäßen Verfahrens (A) a) durch das folgende Formelschema wiedergegeben werden:

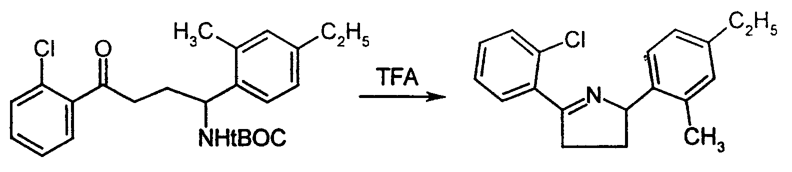

Verwendet man beispielsweise 1-(4-Ethyl-2-Methyl-phenyl)-1-Nitro-3-(2-chlorphenylcarboxyl)-propan als Ausgangsstoffe, so kann der Reaktionsablauf des erfindungsgemäßen Verfahrens (A) b) durch das folgende Formelschema wiedergegeben werden:

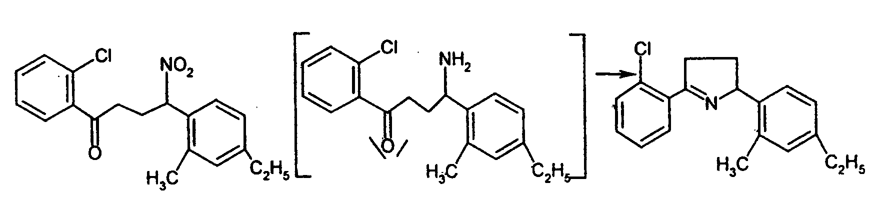

Verwendet man beispielsweise 1-(4-Ethyl-2-Methyl-phenyl)-1-(Diphenylmethylenimino)-3-(2-chlor-phenylcarboxyl)-propan als Ausgangsstoff, so kann der Reaktionsablauf des erfindungsgemäßen Verfahrens (A) c) durch das folgende Formelschema wiedergegeben werden:

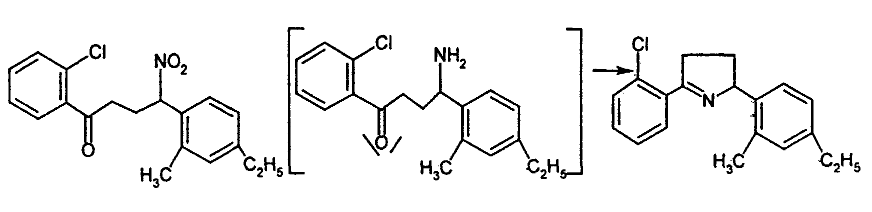

Verwendet man beispielsweise 2-(4-Methoxyphenyl)-cyclobutan-O-methansulfonyloxim und 2-Chlorphenylmagnesiumbromid als Ausgangsstoffe, so kann der Reaktionsablauf des erfindungsgemäßen Verfahrens (B) durch das folgende Formelschema wiedergegeben werden:

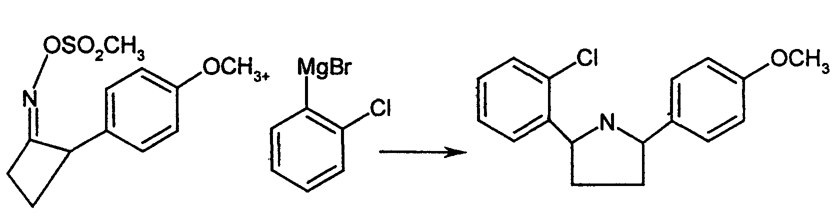

Verwendet man beispielsweise 2-(2-Chlorphenyl)-5-(4-iodphenyl)-3,4-dihydro-2H-pyrrol und 4-Cyanomethoxyphenylboronsäure als Ausgangsstoffe, so kann der Reaktionsablauf des erfindungsgemäßen Verfahrens (C) durch das folgende Formelschema wiedergegeben werden:

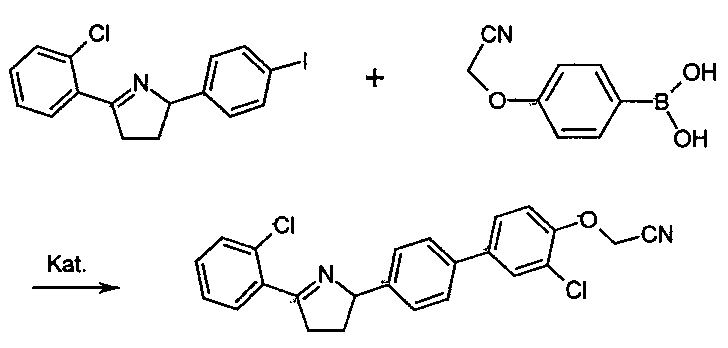

Verwendet man beispielsweise 2-(2-Chlor-phenyl)-5-(3'-chlor-4'-hydroxybiphenyl-4-yl)-3,4-dihydro-2H-pyrrol und α-Bromvaleriansäuremethylester als Ausgangsstoffe, so kann der Reaktionsablauf des erfindungsgemäßen Verfahrens (D) durch das folgende Formelschema wiedergegeben werden:

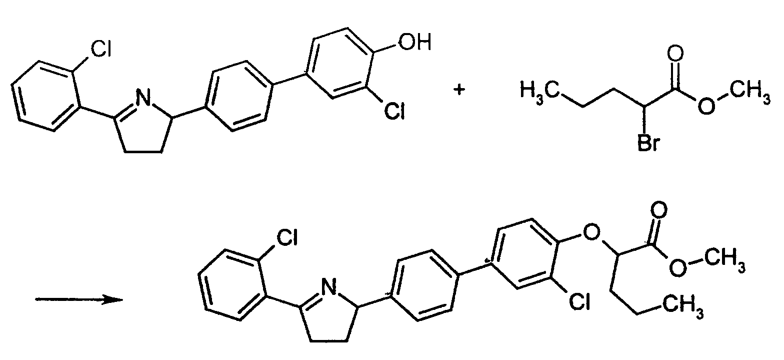

Verwendet man beispielsweise 5-(4'-Cyclopropylcarbonylmethoxy-3-trifluormethoxy-biphenyl-4-yl)-2-(2-chlorphenyl)-3,4-dihydro-2H-pyrrol und *O*-Methylhydroxylamin als Ausgangsstoffe, so kann der Reaktionsablauf des erfindungsgemäßen Verfahrens (E) durch das folgende Formelschema wiedergegeben werden:

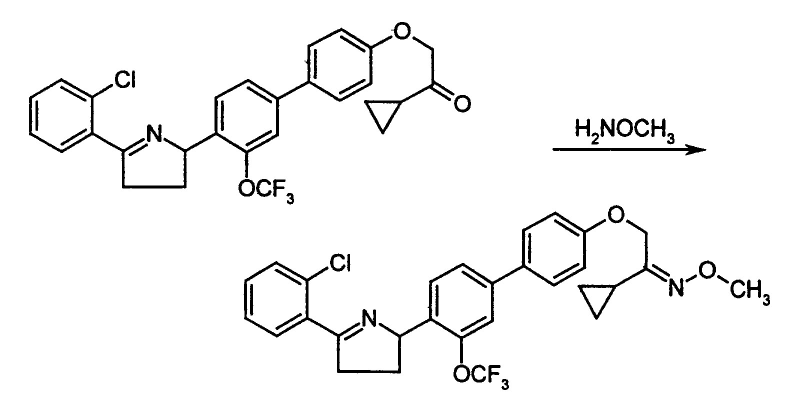

Verwendet man beispielsweise 4-Brombenzylcyanid, so läßt sich der Reaktionsablauf des erfindungsgemäßen Verfahrens (F) durch das folgende Formelschema wiedergeben:

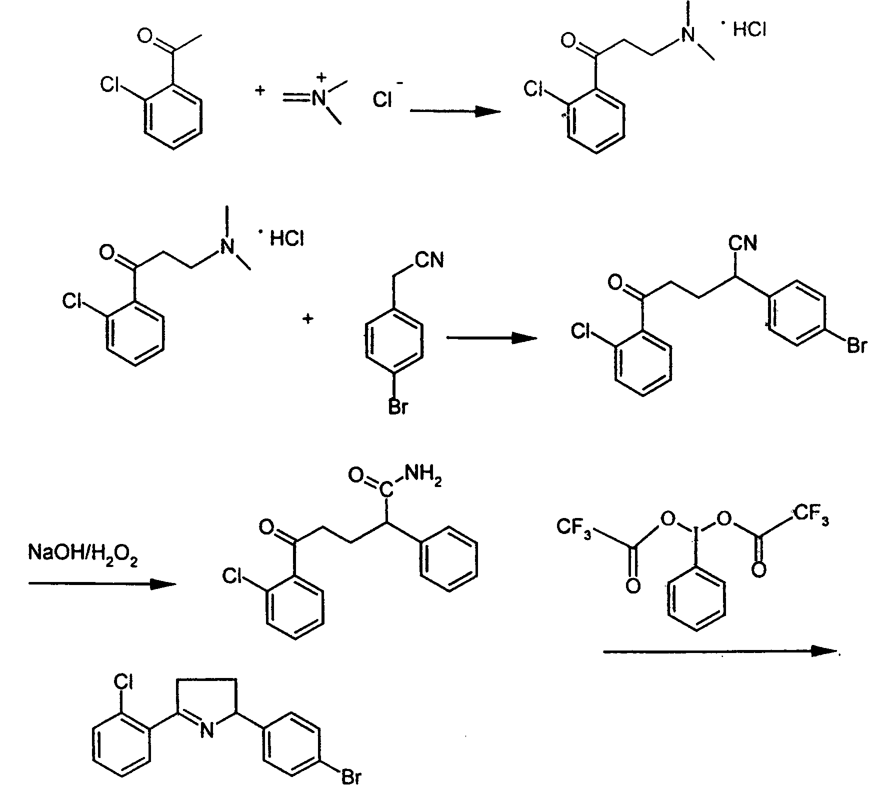

Verwendet man beispielsweise 5-Phenylpyrrolidin-2-on, so läßt sich der Verlauf des erfindungsgemäßen Verfahrens G) durch das folgende Formelschema wiedergeben:

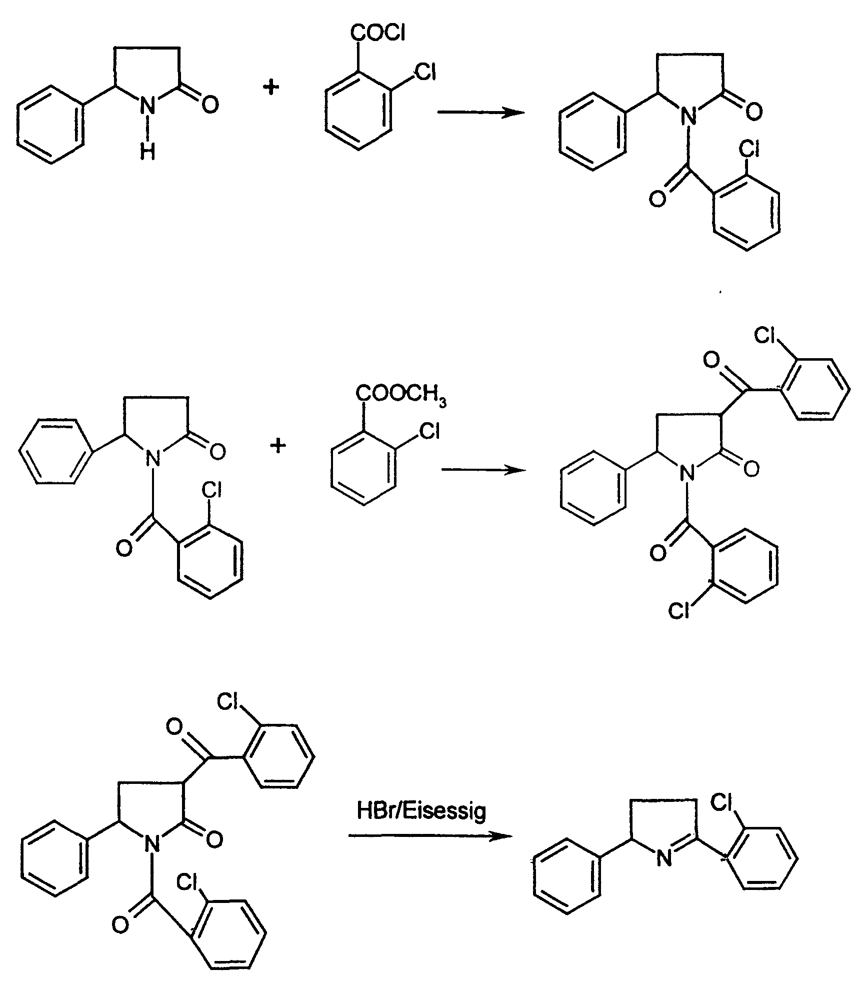

Die zur Durchführung des erfindungsgemäßen Verfahrens (A) a) benötigten Aminoketonderivate sind durch die Formel (VIII) allgemein definiert. In dieser Formel hat Ar vorzugsweise diejenigen Bedeutungen, die bereits im Zusammenhang mit der Beschreibung der cyclischen Imine der Formel (I) als bevorzugt genannt wurden. Die Aminoketonderivate der Formel (VIII) sind neu.

Aminoketon-Derivate der Formel (VIII) lassen sich z.B. herstellen, indem man BOC-geschützte Lactame der Formel (IX) mit metallierten Aromaten der Formel (X) bei Temperaturen zwischen 0°C und 80°C gemäß dem folgenden Reaktionsschema umsetzt:

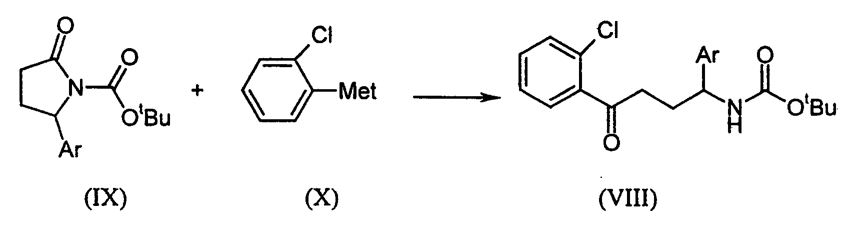

In der Formel (X) steht Met für einen einwertigen Metallrest wie Li, MgI, MgBr oder MgCl.

Metallierte Aromaten der Formel (X) sind zum Teil bekannt oder können nach bekannten Methoden wie z.B. Lithiierung oder Grignard-Reaktion aus den entsprechenden Aromaten oder Halogenaromaten hergestellt werden.

Geschützte Lactame der Formel (IX) erhält man beispielsweise, indem man Lactame der Formel (XI) nach üblichen Methoden wie z.B. Metallierung mit Butyllithium und Umsetzung mit Di-tert.-butyldicarbonat BOC-schützt (vgl. z.B. T. W. Greene, P. G. M. Wuts, Protective Groups in Organic Synthesis, 2. Ed., John Wiley & Sons, New York 1991).

Lactame der Formel (XI) lassen sich z.B. von ω-Alkoxylactamen der Formel (XII) ausgehend nach zwei Methoden herstellen. Man kann diese mit Aromaten der Formel (XIII) in Gegenwart -eines sauren Katalysators, wie beispielsweise Schwefelsäure, Essigsäure oder Aluminiumchlorid und gegebenenfalls in Gegenwart eines Verdünnungsmittels, wie beispielsweise Dichlormethan oder Acetonitril gemäß dem folgenden Reaktionsschema umsetzen:

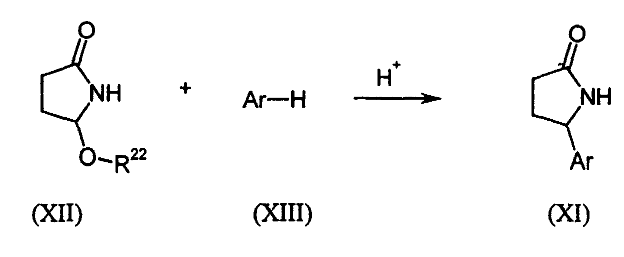

Alternativ kann man mit Aryl-Grignard-Verbindungen der Formel (XIV) in Gegenwart eines Verdünnungsmittels, wie beispielsweise Tetrahydrofuran gemäß dem folgenden Reaktionsschema umsetzen [vgl. Org. Prep. Proced. Int. **25**, 255 (1993)]:

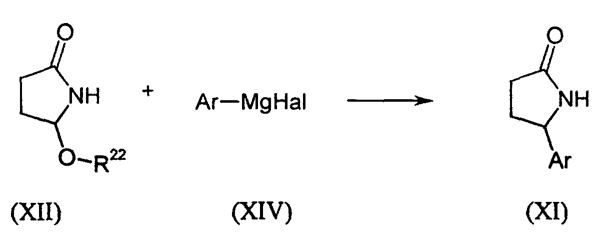

In Formel (XII) steht R²² für Methyl oder Ethyl. In Formel (XIV) steht Hal für Chlor, Brom oder Iod.

Die ω-Alkoxylactame der Formel (XII) sind bekannt, z.T. kommerziell erhältlich und lassen sich z.B. aus den entsprechenden unsubstituierten Imiden durch kathodische oder Natriumboranat-Reduktion oder aus den unsubstituierten Lactamen durch anodische Oxidation, jeweils in Gegenwart von Methanol oder Ethanol herstellen (vgl. z.B. J. Org. Chem. **56**, 1822 (1991); Synthesis **1980**, 315).

Die Aromaten der Formel (XIII) sind Benzolderivate, die allgemein bekannt sind oder nach einer weiten Palette allgemein bekannter Methoden der organischen Chemie hergestellt werden können.

Die Aryl-Grignard-Verbindungen der Formel (XIV) können nach üblicher Weise aus den entsprechenden Arylhalogeniden und Magnesium hergestellt werden. Arylhalogenide sind allgemein bekannte Verbindungen der organischen Chemie.

Lactame der Formel (XI) lassen sich z.B. auch herstellen, indem man substituierte ω-Benzoylcarbonsäuren der Formel (XV) mit einem Reagenz, bereitet aus Ammoniumcarbonat und Ameisensäure bei Siedetemperatur gemäß folgendem Reaktionsschema cyclisiert [vgl. Recl. Trav. Chim. Bays-Bas **81**, 788 (1962)]:

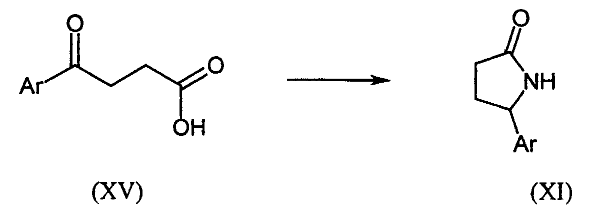

Die hierfür benötigten ω-Benzoylcarbonsäuren der Formel (XV) lassen sich z.B. herstellen, indem man die Dicarbonsäureanhydride der Formel (XVI) mit Aromaten der Formel (XIII) in Gegenwart einer Lewis-Säure wie beispielsweise Aluminiumchlorid und gegebenenfalls in Gegenwart eines Verdünnungsmittels wie beispielsweise Benzol gemäß dem folgenden Reaktionsschema umsetzt [vgl. Recl. Trav. Chim. Bays-Bas **81**, 788 (1962)]:

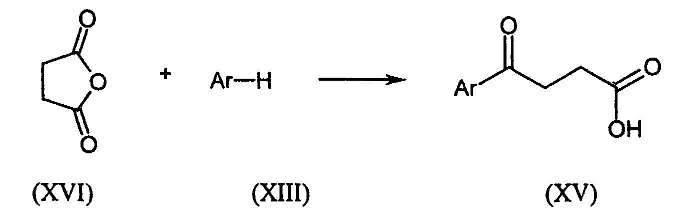

Das hierfür benötigte Anhydrid (Bernsteinsäureanhydrid) ist kommerziell erhältlich.

Ausgehend von den Lactamen der Formel (XI) können alle Verfahrensschritte bis zur Darstellung der cyclischen Imine der Formel (I), einschließlich der Cyclokondensation analog Verfahren A a) auch als "Eintopfreaktion" durchgeführt werden.

Für den Fall, daß Ar im erfindungsgemäßen Wirkstoff der Formel (I) wie in der weiter oben angegebenen Formel (I-b) für ein gegebenenfalls substituiertes Biphenylyl steht, kann man die entsprechenden Biphenyllactame der Formel (XI-a) in einer vorteilhaften Variante des hier beschriebene Verfahrens herstellen, indem man analog zum oben und weiter unten beschriebenen Verfahren (C) bestimmte Phenyllactame der Formel (XVII) mit Boronsäuren der Formel (VI) gemäß dem folgenden Reaktionsschema umsetzt:

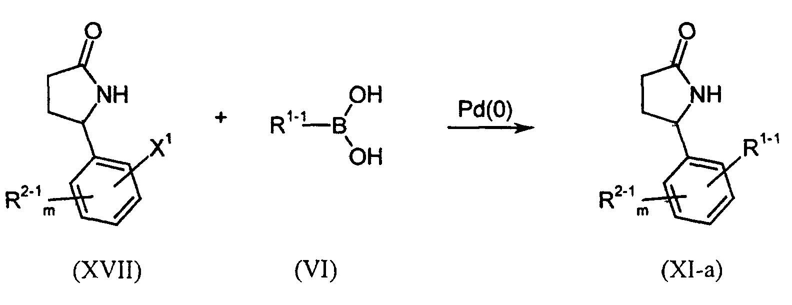

Die Phenyllactame der Formel (XVII), in denen X¹ für Brom oder Iod steht, sind eine Teilmenge der Verbindungen der Formel (XI), deren Herstellung oben angegeben ist. Die Phenyllactame der Formel (XVII), in denen X¹ für Trifluormethansulfonyl steht, lassen sich analog wie bei Verfahren (C) beschrieben aus den entsprechenden Verbindungen der Formel (XI), in denen Ar durch R¹ = Hydroxy substituiert ist, herstellen.

Die zur Durchführung des Verfahrens A) b) benötigten Nitroketone sind durch die Formel (XVIII) allgemein definiert. In dieser Formel hat Ar vorzugsweise diejenigen Bedeutungen, die bereits im Zusammenhang mit der Bechreibung der cyclischen Imine der Formel (I) als bevorzugt genannt wurden. Die Nitroketone der Formel (XVIII) sind neu.

Nitroketone der Formel (XVIII) lassen sich z.B. herstellen, indem man das ω-Chloralkylphenylketon der Formel (XXI) in Gegenwart eines Verdünnungsmittels wie z.B.

Methanol, Ethanol, einem anderen niederen aliphatischen Alkohol oder auch Tetrahydrofuran und in Gegenwart eines Säurebindemittels wie z.B. Natriumhydrid oder eines Alkalialkoholates, vorzugsweise des entsprechenden als Verdünnungsmittel eingesetzten Alkohols, gemäß dem folgenden Reaktionsschema kondensiert:

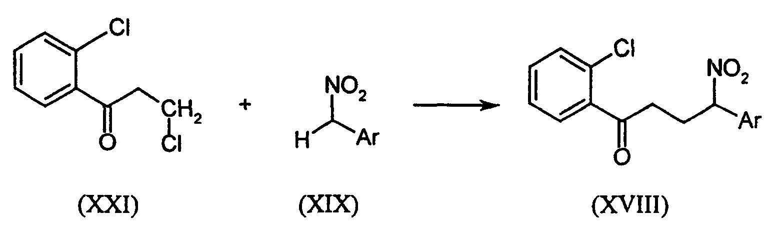

Das ω-Chloralkylphenylketon der Formel (XXI) läßt sich auf bekannte Weise herstellen, wie z.B. durch Friedel-Crafts-Acylierung von Chlorbenzol der Formel (XXII) (s. weiter unten) mit 3-Chlorpropionsäurechlorid.

Die Nitromethylbenzole der Formel (XIX) sind bekannt oder lassen sich auf bekannter Weise herstellen, wie z.B. durch Nitrieren entsprechender Toluole in der Seitenkette oder Umsetzung entsprechender Benzylhalogenide mit Silbernitrit [vgl. hierzu z.B. J. Am. Chem. Soc. **77,** 6269 (1955); J. Am. Chem. Soc **86,** 2681 (1964); Houben-Weyl, Methoden der Organischen Chemie, Georg Thieme Verlag Stuttgart, Band **10/1**, 46-57 (Halogensubstitution), Band **E16**, 145-154 (Beide Methoden)]. Die hierfür benötigten Toluole oder Benzylhalogenide sind allgemein bekannte Verbindungen der organischen Chemie.

Die Nitroketone der Formel (XVIII) lassen sich z.B. durch Michael-Additionen von Nitromethylbenzolen der Formel (XIX) an Phenylvinylketon der Formel (XX) in Gegenwart eines Verdünnungsmittels wie z.B. Methanol, Ethanol oder einem anderen niederen aliphatischen Alkohol und in Gegenwart eines Säurebindemittels wie z.B. vorzugsweise eines Alkalialkoholates des entsprechenden als Verdünnungsmittel eingesetzten Alkohols gemäß dem folgenden Reaktionsschema herstellen (vgl. z.B. J. Prakt. Chem., Serie 4, **1**, 57 (1955); Houben-Weyl, Methoden der Organischen Chemie, Georg Thieme Verlag Stuttgart, Band **10/1**, 199-206):

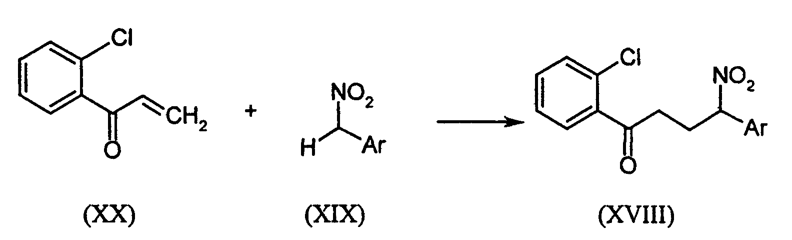

Das Phenylvinylketon der Formel (XX) läßt sich z.B. herstellen, indem man Chlorwasserstoff aus β-Chlorpropiophenon der Formel (XXI), welches sich z.B. durch Friedel-Crafts-Acylierung von Chlorbenzol der Formel (XXII) mit 3-Chlorpropionsäurechlorid erhalten läßt, in Gegenwart eines Säurebindemittels wie beispielsweise Kaliumacetat in Gegenwart eines Verdünnungsmittels wie beispielsweise Methanol gemäß folgendem Reaktionsschema abspaltet [vgl. z.B. J. Prakt. Chem., Serie 4, **1**, 57 (1955)]:

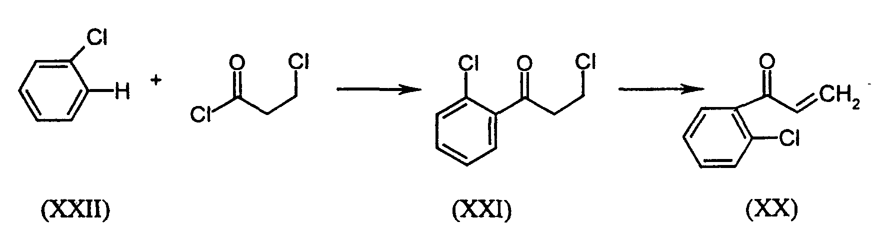

Chlorbenzol der Formel (XXII) ist kommerziell erhältlich.

Das Phenylvinylketon der Formel (XX) läßt sich auch herstellen, indem man *O*-Methyl-methyl-2-Chlor-benzohydroxamat der Formel (XXIII) mit Vinylmagnesiumbromid gemäß folgendem Reaktionsschema umsetzt:

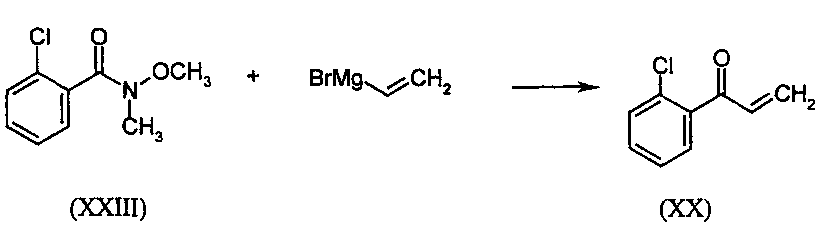

Das *O*-Methyl-methyl-2-Chlor-benzohydroxamat der Formel (XXIII) kann nach bekannten Methoden z.B. aus den entsprechenden Benzoesäure-Derivaten hergestellt werden [vgl. z.B. Tetrahedron Lett. **22**, 3815 (1981)].
Da das Phenylvinylketone der Formel (XX) empfindlich ist, setzt man es in einer bevorzugten Variante zur Herstellung der Nitroketone der Formel (XVIII), direkt mit Nitromethylbenzolen der Formel (XIX) weiter um.

Nitroketone der Formel (XVIII) lassen sich auch herstellen, indem man gemäß nachfolgendem Reaktionsschema Enamine von Methylphenylketonen der Formel (XXVI) an α-Nitrostyrole der Formel (XXVII) addiert und das Reaktionprodukt sauer hydrolisiert:

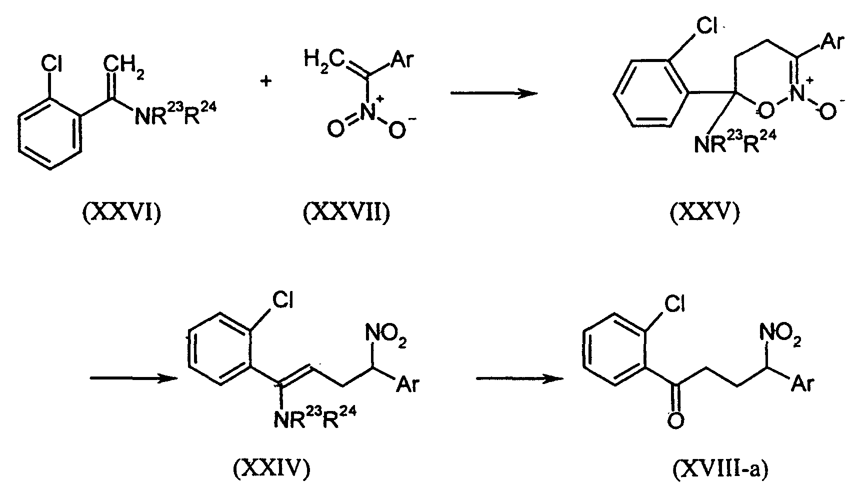

In den Formeln (XXIV), (XXV) und (XXVI) stehen R²³ und R²⁴ gemeinsam mit dem Stickstoffatom, an das sie gebunden sind für einen cyclischen Aminorest, wie z.B. 1-Pyrrolidino, 1-Piperidino oder 4-Morpholino.

Die Addition verläuft zumeist in einer [4+2]-Cycloaddition zu isolierbaren 1,2-Oxazin-N-oxid-Derivaten der Formel (XXV) und wird gegebenenfalls in Gegenwart eines unpolaren Verdünnungsmittel wie z.B. Diethylether bei beispielsweise -80° bis +20°C durchgeführt. Zur Hydrolyse verwendet man z.B wäßrige Mineralsäuren wie Salzsäure gegebenenfalls in Gegenwart von Methanol oder Ethanol [vgl. z.B. Helv. Chim. Acta **68,** 162 (1985); Tetrahedron **45,** 2099 (1989)]. In vielen Fällen ist es vorteilhaft, zunächst die Ringöffnung zu Verbindungen der Formel (XXIV) durch einfaches Lösen des 1,2-Oxazin-N-oxid-Derivates in Methanol oder Ethanol durchzuführen, da sonst unerwünschte Nef-Reaktion zu der entsprechenden Diketo-Verbindung als Konkurrenzreaktion auftritt [vgl. z.B. Tetrahedron **45,** 2099 (1989)].

Die Enamine der Formel (XXVI) sind teilweise bekannt oder lassen sich z.B aus den entsprechend substituierten Acetophenonen und den cyclischen Aminen nach Standardverfahren herstellen (z.B. Org. Syntheses Vol. **58**, 56, John Wiley & Sons, New York). Die hierfür benötigten Acetophenone sind teilweise käuflich, bekannt oder lassen sich nach bekannten Methoden der Aromatenchemie herstellen.

Die Nitrostyrole der Formel (XXVII) sind teilweise bekannt oder lassen sich z.B. durch Formylierung der Nitromethylbenzole der oben angegebenen Formel (XIX) herstellen (vgl. z.B. Houben-Weyl, Methoden der Organischen Chemie. Georg Thieme Verlag, Stuttgart, Band **E16**, 215).

Die zur Durchführung des Verfahrens A) c) benötigten Imine sind durch die Formel (XXVIII) allgemein definiert. In dieser Formel hat Ar vorzugsweise diejenigen Bedeutungen, die bereits im Zusammenhang mit der Beschreibung der cyclischen Imine der Formel (I) als bevorzugt genannt werden.

Die Imine der Formel (XXVIII) lassen sich z.B. herstellen, indem man Michael-Additionen von *N*-Diphenylmethylenbenzylaminen der Formel (XXIX) an das Phenylvinylketon der Formel (XX) gemäß dem folgendem Reaktionsschema durchführt:

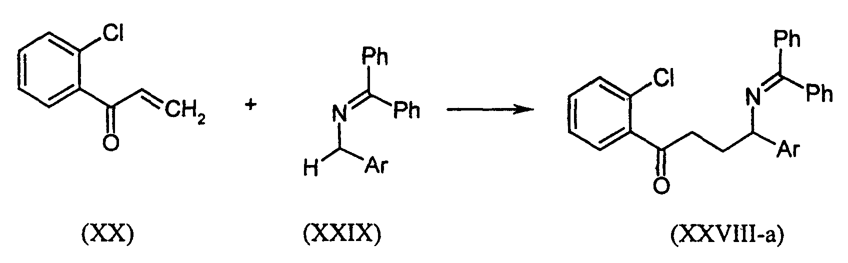

Die Addition wird in Gegenwart eines Säurebindemittels und in Gegenwart eines Verdünnungsmittels wie z.B. Acetonitril oder Dichlormethan und gegebenenfalls in Gegenwart eines Reaktionshilfsmittels z.B. bei Raumtemperatur durchgeführt. Als Säurebindemittel dient vorzugsweise wäßriges Alkali wie 50%-ige Natronlauge in Gegenwart eines Phasentransferkatalysators wie z.B. Triethylbenzylammoniumchlorid als Reaktionshilfsmittel [vgl. z.B. Synth. Commun.**17**, 211 (1987)].

Die Herstellung des Phenylvinylketons der Formel (XX) ist weiter oben beschrieben. Die *N*-Diphenylmethylenbenzylamine der Formel (XXIX) erhält man z.B. durch Umsetzung der entsprechenden Benzylamine mit Benzophenon (vgl. z.B. Tetrahedron. Lett. **1978**, 2641). Die hierfür benötigten Benzylamine sind bekannt oder können nach bekannten Methoden wie z.B. Aminolyse der entsprechenden Benzylhalogenide (siehe oben) hergestellt werden.

Die zur Durchführung des erfindungsgemäßen Verfahrens (B) benötigten cyclischen *O*-Methansulfonyloxime sind durch die Formel (III) allgemein definiert. In dieser Formel hat Ar vorzugsweise diejenigen Bedeutungen, die bereits im Zusammenhang mit der Beschreibung der cyclischen Imine der Formel (I) als bevorzugt genannt wurden. Die *O*-Methansulfonyloxime der Formel (III) sind neu.

Die *O*-Methylsulfonyloxime der Formel (III) lassen sich herstellen, indem man z.B. gemäß dem nachfolgenden Reaktionsschema cyclische α-Arylketone der Formel (XXXI) zunächst nach allgemein bekannten Methoden in ihre Oxime der Formel (XXX) überführt und diese anschließend mit Methansulfonylchlorid analog der Mesylierung von Alkoholen umsetzt:

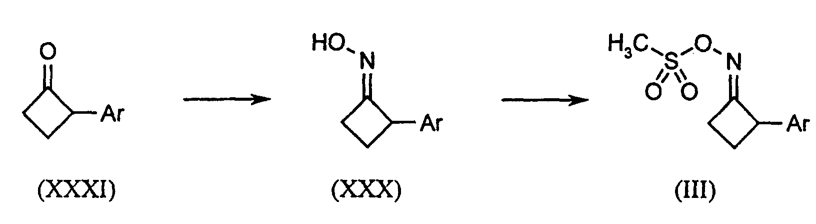

Cyclische α-Arylketone der Formel (XXXI) lassen sich z.B. herstellen, indem man gemäß nachfolgendem Reaktionsschema 1-Arylcycloalkene der Formel (XXXIII) nach üblichen Methoden, wie beispielweise mit m-Chlorperbenzoesäure, zu Oxiranen der Formel (XXXII) epoxidiert und diese anschließend durch saure Aufarbeitung isomerisiert [vgl. z.B. Tetrahedron **30**, 2027 (1974)]:

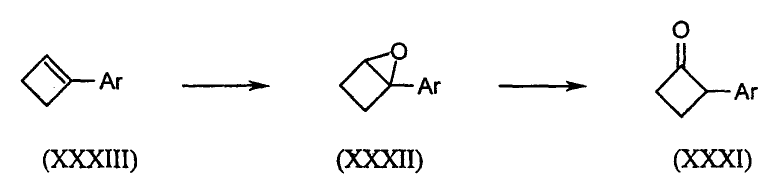

Natürlich kann man auch auf anderen Wegen erhaltene Oxirane der Formel (XXXII) z.B. durch Schütteln einer Lösung in Chloroform mit 20%-iger Schwefelsäure zu cyclischen α-Arylketonen der Formel (XXXI) isomerisieren.

1-Arylcycloalkene der Formel (XXXIII) lassen sich z.B. herstellen, indem man gemäß dem nachfolgenden Reaktionsschema weiter vorne beschriebene Aryl-Grignard-Verbindungen der Formel (XIV) mit Cyclobutanon der Formel (XXXV) unter üblichen Grignard-Bedingungen umsetzt und die z.B. auf diese Weise erhaltenen cyclischen Benzylalkohole der Formel (XXXIV) dehydratisiert:

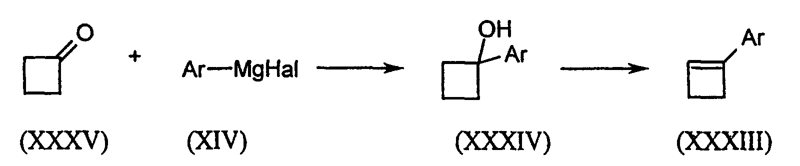

Die Dehydratisierung kann man beispielsweise durchführen, indem man den Alkohol in einem wenig polaren Lösungsmittel wie Hexan löst und bei z.B. 0°C bis 20°C mit halbkonzentrierter Schwefelsäure verrührt [vgl. z.B. Tetrahedron **30**, 2027 (1974)].

Cyclobutanon der Formel (XXXV) ist kommerziell erhältlich. Die weiterhin zur Durchführung des erfindungsgemäßen Verfahrens (B) benötigten Aryl-Gignard-Verbindungen sind durch die Formel (IV) definiert.

Aryl-Grignard-Verbindungen der Formel (IV) lassen sich durch Grignard-Reaktion aus entsprechenden Arylhalogeniden und Magnesium herstellen. Arylhalogenide sind allgemein bekannte Verbindungen der organischen Chemie.

Die zur Durchführung des erfindungsgemäßen Verfahrens (C) benötigten cyclischen Imine der Formel (V) sind, soweit X¹ für Brom oder Iod steht, Teilmengen der erfindungsgemäßen Verbindungen der allgemeinen Formel (I) und lassen sich beispielsweise nach den Verfahren (A) oder (B) herstellen. Für den Fall, daß X¹ für Trifluormethansulfonyl steht, lassen sie sich die Verbindungen der Formel (V-a) durch Umsetzung von Hydroxyverbindungen der Formel (I-f), welche ebenfalls nach den Verfahren (A) oder (B) hergestellt werden können, mit Trifluormethansulfonylchlorid oder Trifluormethansulfonsäureanhydrid in Gegenwart eines Säurebindemittels wie z.B. Pyridin und gegebenenfalls in Gegenwart eines Verdünnungsmittels gemäß folgendem Reaktionsschema herstellen:

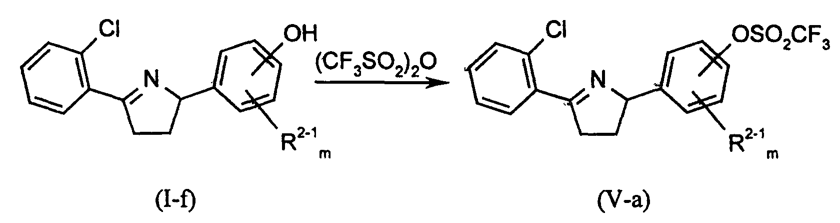

Die ebenfalls zur Durchführung des erfindungsgemäßen Verfahrens (C) benötigten Boronsäuren sind durch die Formel (VI) allgemein definiert. In dieser Formel hat R¹⁻¹ vorzugsweise diejenigen Bedeutungen, die bereits im Zusammenhang mit der Beschreibung der cyclischen Imine der Formel (I-b) als bevorzugt genannt wurden.

Aromatische Boronsäuren der Formel (VI) sind bekannt oder können nach bekannten Methoden hergestellt werden [vgl. Chem. Rev. **45**, 2457 (1995); Pure Appl. Chem. **66**, 213 (1994)].

Die zur Durchführung des erfindungsgemäßen Verfahrens (D) benötigten cyclischen Imine der Formel (I-d) sind Teilmengen der erfindungsgemäßen Verbindungen der allgemeinen Formel (I) und lassen sich beispielsweise nach den Verfahren (A) bis (C) herstellen.

Die weiteren zur Durchführung des erfindungsgemäßen Verfahrens (D) benötigten Verbindungen sind durch die Formel (VII) definiert. In dieser Formel haben R⁹, R¹⁰, G, p, q und r vorzugsweise diejenigen Bedeutungen, die bereits im Zusammenhang mit der Beschreibung der cyclischen Imine der Formel (I) als bevorzugt genannt wurden. Ab steht für eine übliche Abgangsgruppe wie z.B. Halogen, insbesondere Chlor oder Brom; Alkylsulfonyloxy, insbesondere Methylsulfonyloxy; oder gegebenenfalls substituiertes Arensulfonyloxy, insbesondere Phenylsulfonyloxy, p-Chlorsulfonyloxy oder p-Tolylsulfonyloxy.

Die Verbindungen der Formel (VII) sind allgemein bekannte Verbindungen der Organischen Chemie.

Die weiteren zur Durchführung des erfindungsgemäßen Verfahrens (F) benötigten Verbindungen der Formeln (F-I), (F-II), (F-IV) und (F-VII), sind allgemein bekannte Verbindungen der Organischen Chemie.

Die zur Durchführung des erfindungsgemäßen Verfahrens (G) benötigten o-Chlorbenzoesäurechlorid und -methylester sind kommerziell erhältlich.

Zur Durchführung des erfindungsgemäßen Verfahrens A) a) eignen sich organische oder anorganische Brønstedt-Säure wie beispielsweise Fluorwasserstoff, Chlorwasserstoff, Schwefelsäure, Phosphorsäure, Ameisensäure, Essigsäure, Benzoesäure, Citronensäure, Trifluoressigsäure, Methansulfonsäure, Trifluormethansulfonsäure oder Toluolsulfonsäure.

Insbesondere eignet sich die zur Abspaltung der tert.-Butoxycarbonyl-Aminoschutzgruppe üblicherweise eingesetzte Acidolyse mit Trifluoressigsäure (vgl. z.B. T.W. Greene, P.G.M. Wuts, Protective Groups in Organic Synthesis, 2. Ed., John Wiley & Sons, New York 1991).

Das erfindungsgemäße Verfahren (A) a) wird gegebenenfalls in Gegenwart eines geeigneten Säureakzeptors durchgeführt. Als solche kommen alle üblichen anorganischen oder organischen Basen in Frage. Hierzu gehören vorzugsweise Erdalkalimetall- oder Alkalimetallhydroxide, -amide, -alkoholate, -acetate, -carbonate oder - hydrogencarbonate, wie beispielsweise Natrium-, Kalium- oder Ammoniumhydroxid, Natriumamid, Lithiumdiisopropylamid, Natrium-methylat, Natriumethylat, Kalium-tert.-butylat, Natrium-, Kalium-, Calcium- oder Ammoniumacetat. Natrium-, Kalium- oder Ammoniumcarbonat, Natriumhydrogen- oder Kaliumhydrogencarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Das erfindungsgemäße Verfahren (A) a) wird gegebenenfalls in Gegenwart eines Verdünnungsmittels durchgeführt. Hierfür kommen Wasser, organische Lösungsmittel und Mischungen davon in Betracht. Beispielhaft seien genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-amylether, Dioxan, Tetrahydrofuran, 1,2-Dimethoxyethan, 1,2-Diethoxyethan, Diethylenglykoldimethylether oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie Formamid, N,N-Dimethylformamid, N,N-Dimethylacetamid, N-Methylformanilid, N-Methylpyrrolidon oder Hexamethylphosphorsäuretriamid; N-Oxide wie N-Methylmorpholin-N-oxid; Ester wie Methyl-, Ethyl- oder Butylacetat; Sulfoxide, wie Dimethylsulfoxid; Sulfone, wie Sulfolan; Alkohole, wie Methanol, Ethanol, n- oder i-Propanol, n-, iso-, sek- oder tert-Butanol, Ethandiol, Propan-1,2-diol, Ethoxyethanol, Methoxyethanol, Diethylenglykolmonomethylether, Diethylenglykolmonoethylether.

Die Reaktionstemperatur kann bei dem erfindungsgemäßen Verfahren (A) a) innerhalb eines größeren Bereiches variiert werden. Im allgemeinen arbeitet man bei Temperaturen zwischen -50°C und +150°C, bevorzugt zwischen -20°C und +100°C.

Bei der Durchführung des Verfahrens A) a) wird die Säure im allgemeinen im Überschuß eingesetzt.

Das erfindungsgemäße Verfahren A) b) wird als katalytische Hydrierung oder nach anderen allgemein bekannten Methoden zur Reduktion von Nitrogruppen durchgeführt (vgl. z.B. Houben-Weyl, Methoden der Organischen Chemie, Georg Thieme Verlag Stuttgart, Band **11/1**, 394 409 und Band **4/1c**, 490 506).

Das erfindungsgemäße Verfahren A) c) wird als Hydrolyse nach allgemein bekannten Methoden z.B. mit wäßriger Salzsäure durchgeführt.

Als Verdünnungsmittel zur Durchführung der Verfahren A) b) und A) c) kommen die oben für das Verfahren A) a) genannten Verdünnungsmittel in Frage.

Als Verdünnungsmittel zur Durchführung des erfindungsgemäßen Verfahrens (B) kommen inerte organische Lösungsmittel und Mischungen davon in Betracht. Beispielhaft seien genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-amylether, Dioxan, Tetrahydrofuran, 1,2-Dimethoxyethan, 1,2-Diethoxyethan, Diethylenglykoldimethylether oder Anisol.

Bevorzugt setzt man eine Lösung der Grignard-Verbindung der Formel (IV) in einem Ether und eine Lösung des *O*-Methylsulfonyloxim der Formel (III) in einem Kohlenwasserstoff ein.

Die Reaktionstemperatur kann bei dem erfindungsgemäßen Verfahren (B) innerhalb eines größeren Bereiches variiert werden. Im allgemeinen arbeitet man bei Temperaturen zwischen -100°C und +50°C, bevorzugt zwischen -80°C und +30°C.

Bei der Durchführung des erfindungsgemäßen Verfahrens (B) werden Grignard-Verbindung der Formel (IV) und *O*-Methylsulfonyloxim der Formel (III) im molaren Verhältnis von 1:1 bis 3:1, vorzugsweise 1:1 bis 2:1 eingesetzt.

Zur Durchführung des erfindungsgemäßen Verfahrens (C) sind Palladium(0)-Komplexe als Katalysator geeignet. Bevorzugt wird beispielsweise Tetrakis(triphenylphosphin)palladium.

Als Säureakzeptors zur Durchführung des erfindungsgemäßen Verfahrens (C) kommen anorganische oder organische Basen in Frage. Hierzu gehören vorzugsweise Erdalkalimetall- oder Alkalimetallhydroxide, -acetate, -carbonate oder -hydrogencarbonate, wie beispielsweise Natrium-, Kalium-, Barium- oder Ammoniumhydroxid, Natrium-, Kalium-, Calcium- oder Ammoniumacetat, Natrium-, Kalium- oder Ammoniumcarbonat, Natriumhydrogen- oder Kaliumhydrogencarbonat, Alkalifluoride, wie beispielsweise Cäsiumfluorid, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Als Verdünnungsmittel zur Durchführung des erfindungsgemäßen Verfahrens (C) kommen Wasser, organische Lösungsmittel und Mischungen davon in Betracht. Beispielhaft seien genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-amylether, Dioxan, Tetrahydrofuran, 1,2-Dimethoxyethan, 1,2-Diethoxyethan, Diethylenglykoldimethylether oder Anisol; Alkohole, wie Methanol, Ethanol, n- oder i-Propanol, n-, iso-, sek- oder tert-Butanol, Ethandiol, Propan-1,2-diol, Ethoxyethanol, Methoxyethanol, Diethylenglykolmonomethylether, Diethylenglykolmonoethylether.

Die Reaktionstemperatur kann bei dem erfindungsgemäßen Verfahren (C) innerhalb eines größeren Bereiches variiert werden. Im allgemeinen arbeitet man bei Temperaturen zwischen 0°C und +140°C, bevorzugt zwischen 50°C und +100°C.

Bei der Durchführung des erfindungsgemäßen Verfahrens (C) werden Boronsäuren der Formel (VI) und Verbindungen der Formel (V) im molaren Verhältnis von 1:1 bis 3:1, vorzugsweise 1:1 bis 2:1 eingesetzt. Vom Katalysator setzt man im allgemeinen 0,005 bis 0,5 Mol, vorzugsweise 0,01 mol bis 0,1 Mol pro Mol der Verbindung der Formel (V) ein. Die Base setzt man im allgemeinen in einem Überschuß ein.

Das erfindungsgemäße Verfahren (D) wird vorzugsweise in Gegenwart eines geeigneten Säureakzeptors durchgeführt. Als solche kommen alle üblichen anorganischen oder organischen Basen in Frage. Hierzu gehören vorzugsweise Erdalkalimetall- oder Alkalimetallhydride, -hydroxide, -amide, -alkoholate, -acetate, -carbonate oder - hydrogencarbonate, wie beispielsweise Natriumhydrid, Natrium-, Kalium- oder Ammoniumhydroxid, Natriumamid, Lithiumdiisopropylamid, Natrium-methylat, Natrium-ethylat, Kalium-tert.-butylat, Natrium-, Kalium-, Calcium- oder Ammoniumacetat, Natrium-, Kalium- oder Ammoniumcarbonat, Natriumhydrogen- oder Kaliumhydrogencarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Das erfindungsgemäße Verfahren (D) kann in Gegenwart eines geeigneten Phasentransferkatalysators durchgeführt werden. Als Beispiele für solche Katalysatoren seien genannt: Tetrabutylammoniumiodid, -bromid oder -chlorid, Tributylmethylphosphoniumbromid, Trimethyl-C₁₃/C₁₅-alkylammoniumchlorid oder -bromid, Dibenzyldimethylammonium-methylsulfat, Dimethyl-C₁₂/C₁₄-alkylbenzylammoniumchlorid, 15-Krone-5, 18-Krone-6 oder Tris-[2-(2-methoxyethoxy)-ethyl]-amin.

Das erfindungsgemäße Verfahren (D) wird vorzugsweise in Gegenwart eines Verdünnungsmittels durchgeführt. Hierfür kommen z.B. alle bei Verfahren (A) aufgelisteten Lösungsmittel in Frage.

Die Reaktionstemperatur kann bei dem erfindungsgemäßen Verfahren (D) innerhalb eines größeren Bereiches variiert werden. Im allgemeinen arbeitet man bei Temperaturen zwischen -20°C und +100°C, bevorzugt zwischen 0°C und 60°C.

Bei der Durchführung des erfindungsgemäßen Verfahrens (D) arbeitet man im allgemeinen mit ungefähr äquimolaren Mengen der Edukte. Man kann jedoch auch einen Überschuß der Verbindung der Formel (VII) verwenden.

Die Umsetzungen gemäß dem erfindungsgemäßen Verfahren E) sind dem Fachmann bekannte Derivatisierungsreaktionen insbesondere von Carbonsäureestern und Ketonen (vgl. z.B. Houben-Weyl, Methoden der Organischen Chemie, Georg Thieme Verlag, Stuttgart, Bd. VII/2b, insbesonders 1912 ff; Bd. VIII zu Carbonsäureestern und deren Derivaten; Bd. E5, insbesondere S. 812 ff und die darin zitierte Literatur).

Die Schritte des erfindungsgemäßen Verfahren (F) werden gegebenenfalls in Gegenwart eines Verdünnungsmittels durchgeführt. Hierfür kommen Wasser (nicht für F. α), organische Lösungsmittel und Mischungen davon in Betracht. Beispielhaft seien genannt: aliphatische, alicyclische oder aromatische Kohlenwasserstoffe, wie beispielsweise Petrolehter, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-amylether, Dioxan, Tetrahydrofuran, 1,2-Dimethoxyethan, 1,2-Diethoxyethan, Diethylenglykoldimethylether oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie Formamid, N,N-Dimethylformamid, N,N-Dimethylacetamid, N-Methylformanilid, N-Methylpyrrolidon oder Hexamethylphosphorsäuretriamid; N-Oxide wie N-Methylmorpholin-N-oxid; Ester wie Methyl-, Ethyl- oder Butylacetat; Sulfoxide, wie dimethylsulfoxid; Sulfone, wie Sulfolan; Alkohole, wie Methanol, Ethanol, n- oder i-Propanol, n-, iso-, sek- oder tert-Butanol, Ethandiol, Propan-1,2-diol, Ethoxyethanol, Methoxyethanol, Diethylenglykolmonomethylether, Diethylenglykolmonoethylether; Wasser.

Die Reaktionstemperatur kann bei den einzelnen Schritten des erfindungsgemäßen Verfahrens (F) innerhalb eines größeren Bereiches variiert werden. Im allgemeinen arbeitet man bei Temperaturen zwischen -50°C und 250°C, bevorzugt zwischen - 20°C und +100°C.

Bei der Durchführung des Verfahrens (F) werden das Chloracetophenon (F-I) und das Iminiumsalz (F-II) in molaren Verhältnis 1:1 bis 2:1, vorzugsweise 1,1:1 eingesetzt.

Bei der Durchführung des Verfahrens (F) werden die Verbindungen der Formel (F-III) und das Benzylcyanid der Formel (F-IV) im molaren Verhältnis 1:1 bis 1:2, vorzugsweise 1:1,05 eingesetzt.

Bei der Durchführung des Verfahrens (F) werden die Verbindungen der Formel (F-V) zu NaOH bzw. H₂O₂ im Verhältnis 1:2,5:5 bis 1:5:10, vorzugsweise 1:2,5:5 bezogen auf die Äquivalente eingesetzt.

Bei der Durchführung des Verfahrens (F) werden PIFA (F-VII) und die Verbindungen der Formel (F-VI) im molaren Verhältnis 1:1 bis 3:1, vorzugsweise 1:1 bis 2: 1 eingesetzt.

Die Schritte des erfindungsgemäßen Verfahren (G) werden gegebenenfalls in Gegenwart eines Verdünnungsmittels durchgeführt. Hierfür kommen Wasser (nicht für G. α und β), organische Lösungsmittel und Mischungen davon in Betracht. Beispielhaft seien genannt: aliphatische, alicyclische oder aromatische Kohlenwasserstoffe, wie beispielsweise Petrolehter, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-amylether, Dioxan, Tetrahydrofuran, 1,2-Dimethoxyethan, 1,2-Diethoxyethan, Diethylenglykoldimethylether oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie Formamid, N,N-Dimethylformamid, N,N-Dimethylacetamid, N-Methylformanilid, N-Methylpyrrolidon oder Hexamethylphosphorsäuretriamid; N-Oxide wie N-Methylmorpholin-N-oxid; Ester wie Methyl-, Ethyl- oder Butylacetat; Sulfoxide, wie dimethylsulfoxid; Sulfone, wie Sulfolan; Alkohole, wie Methanol, Ethanol, n- oder i-Propanol, n-, iso-, sek- oder tert-Butanol, Ethandiol, Propan-1,2-diol, Ethoxyethanol, Methoxyethanol, Diethylenglykolmonomethylether, Diethylenglykolmonoethylether; Wasser.

Die Reaktionstemperatur kann bei den einzelnen Schritten des erfindungsgemäßen Verfahrens (F) innerhalb eines größeren Bereiches variiert werden. Im allgemeinen arbeitet man bei Temperaturen zwischen -50°C und 250°C, bevorzugt zwischen -20°C und +100°C.

Die Schritte α) und β) des erfindungsgemäßen Verfahrens (F) werden vorzugsweise in Gegenwart eines geeigneten Säureakzeptors durchgeführt. Als solche kommen alle üblichen anorganischen oder organischen Basen in Frage. Hierzu gehören vorzugsweise Erdalkalimetall- oder Alkalimetallhydride, -hydroxide, -amide, -alkoholate, - acetate, -carbonate oder -hydrogencarbonate, wie beispielsweise Natrium-, Kalium-, Calcium- oder Ammoniumacetat, Natrium-, Kalium- oder Ammoniumcarbonat, Natriumhydrogen- oder Kaliumhydrogencarbonat, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU). O-Chlorbenzoesäurechlorid bzw. Methylester werden im Überschuß eingesetzt.

Die Umsetzungen der erfindungsgemäßen Verfahren können bei Normaldruck oder unter erhöhtem Druck durchgeführt werden, vorzugsweise wird bei Normaldruck gearbeitet. Die Aufarbeitung geschieht nach üblichen Methoden der Organischen Chemie. Die Endprodukte werden vorzugsweise durch Kristallisation, chromatographische Reinigung oder durch Entfernung der flüchtigen Bestandteile gegebenenfalls im Vakuum gereinigt.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium comi, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp.

Die erfindungsgemäßen Wirkstoffe der Formel (I) zeichnen sich insbesondere durch hervorragende Wirkung gegen Larven des Meerettichblattkäfers (Phaedon cochleariae), Raupen des Eulenfalters (Spodoptera frugiperda), Larven der Grünen Reiszikade (Nephotettix cincticeps), Pfirsichblattläuse (Myzus persicae) und alle Stadien der gemeinen Spinnmilbe (Tetranychus urticae).

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoff-imprägnierte Natur- und synthetische Stoffe sowie feinstverkapselungen in polymeren Stoffen.

Diese Formulierungen werden in bekannter Weise hergestellt, z. B. und bevorzugt durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

### Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgierund/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate. Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

### Besonders günstige Mischpartner sind z.B. die folgenden:

**Fungizide:**
   2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino[alpha-(otolyloxy)-o-tolyl]acetat; 2-Phenylphenol (OPP), Aldimorph, Ampropylfos, Anilazin, Azaconazol,
   Benalaxyl, Benodanil, Benomyl, Binapacryl, Biphenyl, Bitertanol, Blasticidin-S, Bromuconazole, Bupirimate, Buthiobate,
   Calciumpolysulfid, Captafol, Captan, Carbendazim, Carboxin, Chinomethionat (Quinomethionat), Chloroneb, Chloropicrin, Chlorothalonil, Chlozolinat, Cuiraneb,
   Cymoxanil, Cyproconazole, Cyprofuram,
   Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
   Edifenphos, Epoxyconazole, Ethirimol, Etridiazol,
   Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
   Guazatine,
   Hexachlorobenzol, Hexaconazol, Hymexazol,
   Imazalil, Imibenconazol, Iminoctadin, Iprobenfos (IBP), Iprodion, Isoprothiolan,
   Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
   Mancopper, Mancozeb, Maneb, Mepanipyrim, Mepronil, Metalaxyl, Metconazol, Methasulfocarb, Methfuroxam, Metiram, Metsulfovax, Myclobutanil,
   Nickel-dimethyldithiocarbamat, Nitrothal-isopropyl, Nuarimol,
   Ofurace, Oxadixyl, Oxamocarb, Oxycarboxin,
   Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
   Quintozen (PCNB),
   Schwefel und Schwefel-Zubereitungen,
   Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
   Validamycin A, Vinclozolin,
   Zineb, Ziram.
**Bakterizide:**
   Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, AC 303 630, Acephat, Acrinathrin, Alanycarb, Aldicarb, Alphamethrin, Amitraz, Avermectin. AZ 60541, Azadirachtin, Azinphos A, Azinphos M, Azocyclotin,
   Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxim, Butylpyridaben,
   Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
   Deltamethrin, Demeton M, Demeton S, Demeton-S-methyl, Diafenthiuron, Diazinon. Dichlofenthion, Dichlorvos, Dicliphos, Dicrotophos, Diethion, Diflubenzuron, Dimethoat, Dimethylvinphos, Dioxathion, Disulfoton,
   Edifenphos, Emamectin, Esfenvalerat, Ethiofencarb, Ethion, Ethofenprox, Ethoprophos, Etrimphos,
   Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
   HCH, Heptenophos, Hexaflumuron, Hexythiazox,
   Imidacloprid, Iprobenfos, Isazophos, Isofenphos, Isoprocarb, Isoxathion, Ivermectin, Lamda-cyhalothrin, Lufenuron,
   Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
   Naled, NC 184, NI 25, Nitenpyram,
   Omethoat, Oxamyl, Oxydemeton M, Oxydeprofos,
   Parathion A, Parathion M, Permethrin, Phenthoat, Phorat, Phosalon, Phosmet, Phosphamidon, Phoxim, Pirimicarb, Pirimiphos M, Primiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,
   Quinalphos,
   RH 5992,
   Salithion, Sebufos, Silafluofen, Sulfotep, Sulprofos,
   Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiometon, Thionazin, Thuringiensin, Tralomethrin, Triarathen, Triazophos, Triazuron, Trichlorfon, Triflumuron, Trimethacarb,
   Vamidothion, XMC, Xylylcarb, YI 5301 / 5302, Zetamethrin.

Auch eine Mischung mit anderen bekannten Wirkstoffen, wie Herbiziden oder mit Düngemitteln und Wachstumsregulatoren ist möglich.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp.
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp.. Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.
Aus der Ordnung der Siphonapterida z.B. Pulex spp., Ctenocephalides spp., Xenopsylla spp., Ceratophyllus spp.
Aus der Ordnung der Heteropterida z.B. Cimex spp., Triatoma spp., Rhodnius spp., Panstrongylus spp.
Aus der Ordnung der Blattarida z.B. Blatta orientalis, Periplaneta americana. Blattela gernianica, Supella spp.
Aus der Unterklasse der Acaria (Acarida) und den Ordnungen der Meta- sowie Mesostigmata z.B. Argas spp., Omithodorus spp., Otabius spp., Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp., Sternostoma spp., Varroa spp.
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Beispielsweise zeigen sie eine hervorragende Wirksamkeit gegen alle larvalen Stadien der Fliege *Lucilia cuprina* und alle Entwicklungsstadien der Zecke *Amblyomma variegatum*.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändem, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie
   Hylotrupes bajulus, Chlorophorus pilosis, Anobium punctatum, Xestobium rufovillosum, Ptilinus pecticornis, Dendrobium pertinex, Emobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus
Hautflügler wie
   Sirex juvencus, Urocerus gigas, Urocerus gigas taignus, Urocerus augur
Termiten wie
   Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

Borstenschwänze, wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege.

Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/ oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindelöl und/oder Monochlomaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glykolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. dem Ausfällen vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

- Als ganz besonders bevorzugte Zumischpartner seien Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron, sowie Fungizide, wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on genannt.

Die Herstellung und die Verwendung der erfindungsgemäßen Wirkstoffe gehen aus den nachfolgenden Beispielen hervor.

### Herstellungsbeispiele

### Beispiel I-8:

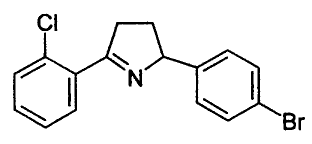

3,22 g (7,5 mmol) PIFA (1,1-Bis-(trifluoracetoxy)iodbenzol wurden in 25 ml absolutem Acetonitril und 10 ml deionisiertem Wasser vorgelegt. Anschließend wurden bei Raumtemperatur 1,9 g (5,0 mmol) F-VI-1 zugegeben und ca. 4 h bei Raumtemperatur gerührt. Dann wurde mit 100 ml 1N HCl versetzt und ca. 3 h unter Rühren auf 50°C erwärmt. Die Reaktionsmischung wurde mit Methyl-t-Butylether extrahiert und die organischen Phasen vereinigt. Die organischen Phasen wurden 1N HCl extrahiert und die vereinigten HCl Phasen mit 20 %iger NaOH-Lösung alkalisch gestellt. Die so erhaltene Lösung wurde erneut mit Methyl-t-Butylether extrahiert, die organischen Phasen vereinigt, getrocknet und eingeengt.
Es wurden 0,9 g (54 % d.Th.) 2-(2-Chlorphenyl)-5-(4-Bromphenyl)-3,4-dihydro-2H-pyrrol erhalten.
¹H-NMR (500 MHz, d₆-DMSO [ppm]: 1,7/2,5/3,0-3,1) (m, 4H, 2xCH₂); 5,2 (m, 1H,CH); 7,2-7,7 (m, 8H, ArH).

### Beispiel I-7:

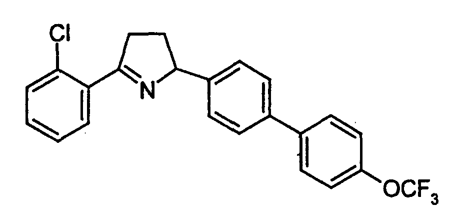

1,0 g (3,0 mmol) 2-(2-Chlorphenyl)-5-(4-Bromphenyl)-3,4-dihydro-2H-pyrrol (1-8) wurden in 8,0 ml Dimethoxyethan und 6,4 ml 1 N Na₂CO₃-Lösung vorgelegt. 0,93 g (4,5 mmol) Trifluormethoxyphenylboronsäure wurden bei Raumtemperatur zugesetzt und anschließend wurde ca. 20 min lang Argon durch die Reaktionsmischung geleitet. Dann wurden 105 mg (0,15 mmol; 5 mol%) Pd (PPh₃)₂Cl₂ zugegeben und über Nacht refluxiert. Nach DC-Kontrolle wurde das auf Raumtemperatur abgekühlte Reaktionsgemisch mit ca. 30 ml H₂O und 50 ml Essigester versetzt und filtriert. Die Phasen wurden getrennt, die wäßrige Phase wurde mehrfach mit Essigester extrahiert und die organischen Phasen vereinigt, getrocknet und eingeengt. Das Rohprodukt wurde an Kieselgel mit Cyclohexan/Essigester 10:1 chromatographiert. Es wurde 0,14 g (11,2 % d.Th.) 2-(2-Chlorphenyl)-5-(4-Trifluormethoxy-4,4'-Biphenyl-1yl)-3,4-dihydro-2H-pyrrol erhalten.
¹-NMR(500 MHz, CDCl₃) [ppm]: 2,0/2,7/3.2/3.3) (m, 4H, 2xCH₂); 5,4 (m, 1H,CH); 7,3-7,5 (m, 12H, Ar-H).

### Beispiel I-20

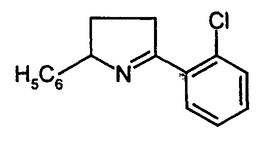

3,7 g (8,4 mmol) 1,3-(2-Chlorbenzoyl)-5-phenyl-pyrrolidin-2-on wurden in 20 ml HBr/Eisessig (33 %ig) 4 Stunden am Rückfluß gekocht. Anschließend wurde die Reaktionsmischung auf Wasser gegossen und 3 mal mit Essigester extrahiert. Die organischen Phasen wurden vereinigt, mit Natriumhydrogencarbonat-Lösung gewaschen, getrocknet und eingeengt. Das dunkle Öl wurde durch Säulenchromatographie im System Cyclohexan/Essigester (2:1) gereinigt. Es wurden 0,45 g (Ausbeute: 21 % der Theorie) zähes, öliges 3,4-Dihydro-2-(2-chlorrphenyl)-5-phenyl-2H-pyrrolin vom log P* (pH 7,5) = 3,61 erhalten.
- log P* =: negativer dekadischer Logarithmus des Alkan/Wasser Verteilungsko- effizienten, bestimmt durch HPLC Analytik mit Wasser/Acetonitril als Laufmittel auf 125 x 4.0 mm Kromasil 120 C 18 (5µm); Fluß: 1,5 ml/min.

### Beispiel I-43:

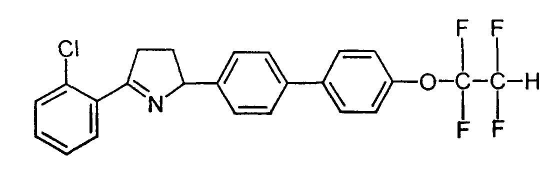

100 mg der Verbindung (VIII-43) wurden in 2 ml CH₂Cl₂ vorgelegt und auf 0°C gekühlt. Trifluoressigsäure (0,139 ml, 200 mg) wurden zugegeben und anschließend wurde der Ansatz über Nacht bei Raumtemperatur gerührt. Trifluoressigsäure wurde im Vakuum einrotiert und der Rückstand in Essigester aufgenommen. Mit 1 N NaOH wurde der pH-Wert auf 11 gestellt. Die organische Phase wurde mit Wasser gewaschen, über MgSO₄ getrocknet, filtriert und eingeengt. Es wurden 30 mg der Verbindung I-43 erhalten.
HPLC: Logp (pH 2,3 = 4,01
LC-MS: M⁺+H 448

### Herstellung der Ausgangsprodukte

### γ-Ethoxy-γ-butyrolactam

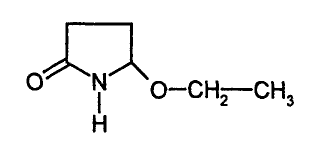

9,91 g Succinimid wurden bei 0°C in 415 ml Ethanol vorgelegt und portionsweise mit insgesamt 5,53 g Natriumboranat versetzt. Es wurden bei dieser Temperatur über 4½ Stunden alle 15 Minuten 2 bis 3 Tropfen 2N ethanolischer Chlorwasserstoff zugetropft. Anschließend wurde mit weiterer Säure auf pH 3 angesäuert. Nach 1 Stunde Rühren bei 0°C wurde mit 1%-iger ethanolischer Kalilauge neutralisiert, weitere 15 Minuten gerührt und eingedampft. Der Rückstand wurde in Wasser aufgenommen und dreimal mit Dichlormethan extrahiert. Nach Trocknen über Natriumsulfat und Einengen wurden 7,16 g (55 % d.Th.) γ-Ethoxy-γ-butyrolactam erhalten.

### Beispiel XI-1

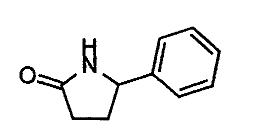

6,45 g γ-Ethoxy-γ-butyrolactam und 50 ml konz. Schwefelsäure wurden bei 0°C vorgelegt und 18,8 ml Benzol zugesetzt. Nach dem Auftauen wurde 4 Tage bei Raumtemperatur gerührt. Zur Aufarbeitung wurde auf Eis gegossen, dreimal mit Ethylacetat extrahiert, die vereinigten Extrakte je einmal mit Wasser und gesättigter Kochsalzlösung gewaschen, getrocknet und eingedampft. Es wurden 8,1 g (100 % d.Th.) γ-Phenyl-γ-butyrolactam erhalten.
¹H-NMR (400 MHz, d₆-DMSO) [ppm]: 1,75 (m, 1H); 2,23 (t, 2H); 2,45 (m, 1H); 4,67 (t, 1H); 7,26-7,39 (m, 5H); 8,08 (br, 1H)

### Beispiel XI-2

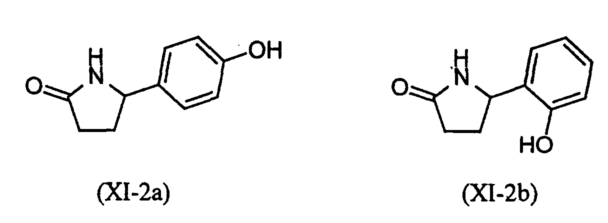

12,9 g γ-Ethoxy-γ-butyrolactam, 10 ml konz. Schwefelsäure und 90 ml Eisessig wurden bei 0°C vorgelegt und portionsweise mit insgesamt 18,8 g Phenol versetzt. Nach dem Auftauen wurde 2 Tage bei Raumtemperatur gerührt. Zur Aufarbeitung wurde auf Eis gegossen, dreimal mit Ethylacetat extrahiert, die vereinigten Extrakte je einmal mit Wasser und gesättigter Kochsalzlösung gewaschen, getrocknet und eingedampft. Aus der wäßrigen Phase kristallisierte nach einiger Zeit γ-2-Hydroxyphenyl-γ-butyrolactam (XI-2b) vom Schmelzpunkt 220°C aus (6,4 g 36 % d.Th). Der Eindampfrückstand wurde mit einem 1:1-Gemisch Cyclohexan/Ethylacetat verrührt und lieferte nach Absaugen 4,65 g γ-4-Hydroxyphenyl-γ-butyrolactam (XI-2a) vom Schmelzpunkt 183°C. Das Filtrat wurde eingedampft. Durch Umkristallisieren aus Dichlormethan/Hexan wurden weitere 3,35 g (gesamt: 45 % d.Th.) γ-4-Hydroxyphenyl-γ-butyrolactam erhalten.

### Beispiel XVII-2

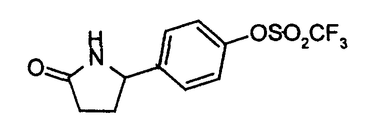

Zu 5,23 g γ-4-Hydroxyphenyl-γ-butyrolactam (z.B. aus Bsp. XI-2) in 60 ml Pyridin wurden bei 0°C 10 g Trifluormethansulfonsäureanhydrid getropft. Nach Rühren über Nacht bei Raumtemperatur wurde auf Eis gegossen, mit 10%-iger Salzsäure angesäuert und dreimal mit Ethylacetat extrahiert. Nach Trocknung und Abdampfen des Lösungsmittels wurden 6,4 g (70 % d.Th.) γ-4-Trifluormethylsulfonyloxyphenyl-γ-butyrolactam vom Schmelzpunkt 127°C erhalten.

### Beispiel XI-a-2

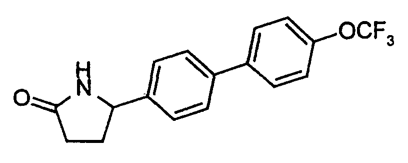

5,4 g γ-4-Trifluormethylsulfonyloxyphenyl-γ-butyrolactam (z.B. aus Bsp. XVII-2) wurden unter Argon in 43 ml Dimethoxyethan vorgelegt. Nacheinander gab man 5,87 g 4-Trifluormethoxyboronsäure sowie 1,01 g Tetrakis(triphenylphosphin)palladium zu. Nach 15 Minuten wurden 28 ml 2M Sodalösung zugegeben, auf 80°C erwärmt und über Nacht gerührt. Nach Beendigung der Reaktion wurde in Wasser/Ethylacetat aufgenommen, die Phasen getrennt und die wäßrige Phase zweimal mit Ethylacetat extrahiert. Die vereinigten organischen Phasen wurden mit gesättigter Kochsalzlösung gewaschen und getrocknet. Durch Eindampfen wurden 5.5 g (98 % d.Th.) γ-4'-Trifluormethoxybiphenyl-4-yl-γ-butyrolactam vom Schmelzpunkt 128°C erhalten.

### Beispiel XI-3

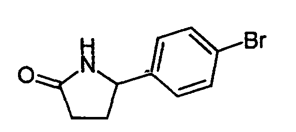

In einem 3 1-Dreihalskolben mit Rührer und Destillationsbrücke wurden 199,3 g Ammoniumformiat in 127,9 g Ameisensäure vorgelegt und 210 g aus Toluol umkristallisierte 4-Brombenzoylpropionsäure zugegeben. Darauf wurde der Kolben in ein 200°C heißes Ölbad getaucht. Bei 60°C beginnt der Kolbeninhalt unter Gasentwicklung in Lösung zu gehen. Über ca. 2 h wird bei von 140 bis auf 167°C steigender Sumpftemperatur wird ausdestilliert. Nach Abkühlen auf unter 60°C wurden vorsichtig 1 l Dichlormethan zugegeben und ausgefallenes Salz durch Absaugen über eine Filtemutsche abgetrennt. Die organische Phase wurde mit 1 l Wasser gewaschen, über Magnesiumsulfat getrocknet und im Vakuum eingeengt. Zur Reinigung wurde das Rohprodukt wurde über 1 kg Kieselgel mit Dichlormethan/Ethanol/Triethylamin (95:5:3) filtriert und anschließend aus Methyl-tert.-butyl-ether kristallisiert. Es wurden 38 g (19 % d.Th.) γ-4-Bromphenyl-γ-butyrolactam vom Schmelzpunkt 142°C erhalten.

### Beispiel XI-43:

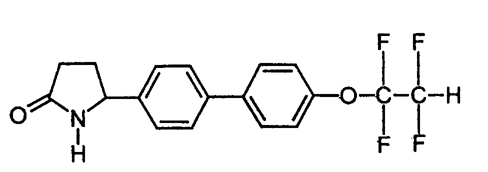

In einem Autoklav wurden 400 ml HF einkondensiert. Dann wurden 38,7 g γ-ethoxy-γ-butyrolactam und 40,6 g Tetrafluorethoxybiphenyl gemeinsam in 100 ml CH₂Cl₂ gelöst und zugegeben. Das Reaktionsgemisch wurde über Nacht bei Raumtemperatur gerührt. HF wurde abgezogen, der Rückstand in CH₂Cl₂ aufgenommen und mit wässriger Natriumhydrogencarbonat-Lösung gewaschen. Die organische Phase wurde über MgSO₄ getrocknet, filtriert, und eingeengt. Die Rohausbeute wurde aus 500 ml Toluol umkristallisiert. 20,9 g weißer Feststoff wurde erhalten.
HPLC: LogP (pH 2,3) = 2,79

### Beispiel IX-1

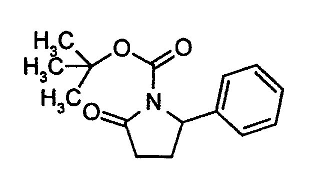

3,4 g γ-Phenyl-γ-butyrolactam (z.B aus Bsp. XI-1) wurden in 63 ml Tetrahydrofuran (THF) vorgelegt und bei -78°C mit 9,24 ml 2,4N Butyllithium-Lösung in n-Hexan versetzt. Nach halbstündigem Rühren bei dieser Temperatur wurde eine Lösung von 5,04 g Di-tert.-Butyldicarbonat in 20 ml THF unter weiterer Kühlung zugetropft, weitere 3 Stunden bei -78°C und dann über Nacht ohne Kühlung gerührt. Danach wurde mit gesättigter wäßriger Ammoniumchlorid-Lösung hydrolisiert, mit Wasser verdünnt und dreimal mit Ethylacetat extrahiert. Die vereinigten organischen Phasen wurden mit gesättigter Kochsalzlösung gewaschen und über Magnesiumsulfat getrocknet. Durch Eindampfen wurden 1,54 g (28 % d.Th.) *N*-^{t}Butoxycarbonyl-γ-phenyl-γ-butyrolactam erhalten.
¹H-NMR (400 MHz, d₆-DMSO) [ppm]: 1,18 (s, 9H); 1,73 (m, 1H); 2,40-2,60 (m, 3H); 5,10 (m, 1H), 7,24 (m, 2H); 7,30 (m, 1H); 7,38 (m, 2H)

### Beispiel IX-2

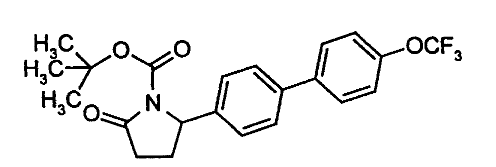

1,7 g γ-4'-Trifluormethoxybiphenyl-4-yl-γ-butyrolactam (z.B. aus Bsp. XI-a-2) wurden in 30 ml Tetrahydrofuran (THF) vorgelegt und bei -78°C mit 2,42 ml 2,4N Butyllithium-Lösung in n-Hexan versetzt. Nach halbstündigem Rühren bei dieser Temperatur wurde eine Lösung von 1,27 g Di-tert.-Butyldicarbonat in 10 ml THF unter weiterer Kühlung zugetropft. Dann wird die Kühlung entfernt und bei Raumtemperatur über Nacht gerührt. Danach wurde mit gesättigter wäßriger Ammoniumchlorid-Lösung hydrolisiert, mit 2N Salzsäure angesäuert und dreimal mit Dichlormethan extrahiert. Nach Trocknung und Eindampfen wurde das Produkt durch Säulenchromatographie gereinigt (stationäre Phase: Kieselgel; mobile Phase Gradient Cyclohexan:Ethylacetat = 5:1,3 bis 1,1:1). Es wurden 1,14 g (47 % d.Th.) teilkristallines *N*-^{t}Butoxycarbonyl-γ-4'-trifluormethoxybiphenyl-4-yl-γ-butyrolactam erhalten.
¹H-NMR (400 MHz, CDCl₃) [ppm]: 1,22 (s, 9H); 1,79 (m, 1H); 2,48-2,60 (m, 3H); 5,17 (m, 1H); 7,36 (d, 2H); 7,46 (d, 2H); 7,71 (d, 2H); 7,80 (d, 2H)

### Beispiel IX-3

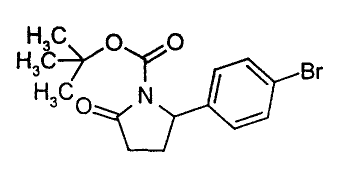

3,24 ml Diisopropylamin wurden in 90 ml THF bei -78°C vorgelegt und mit 9,24 ml 2,4N Butyllithium-Lösung in n-Hexan versetzt. Nach ½ h Rühren bei dieser Temperatur wurden eine Lösung von 5,02 g γ-4-Bromphenyl-γ-butyrolactam (z.B. aus Beispiel XI-3) in 20 ml THF zugetropft. Nach weiterer ½ h Rühren bei -78°C wurden 5,04 g Di-tert.-Butyldicarbonat in 20 ml THF zugetropft, auftauen gelassen und bei Raumtemperatur über Nacht gerührt. Danach wurde mit gesättigter wäßriger Ammoniumchlorid-Lösung hydrolisiert, mit 2N Salzsäure angesäuert und mit 150 ml Dichlormethan dreimal extrahiert. Nach Trocknung über Magnesiumsulfat und Eindampfen wurde das Produkt durch Kristallisieren aus Dichlormethan/Hexan gereinigt. Es wurden insgesamt 7,61 g (97 % d.Th.) kristallines *N*-^{t}Butoxycarbonyl-γ-4-bromphenyl-γ-butyrolactam erhalten. Die reinste Kristallfraktion (2,34 g) schmolz bei 122-124°C.

### Beispiel IX-43:

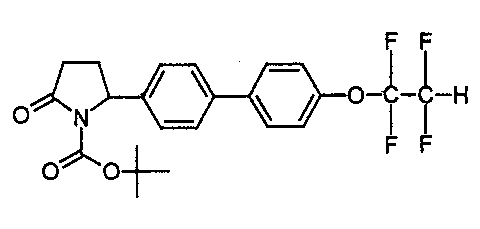

8,8 g der Verbindung (XI-43) wurden mit 8,1 g Pyrokohlensäuretertiärbutylester und 0,2 g Kaliumfluorid in 80 ml Toluol 6 Stunden auf 108°C erhitzt, anschließend über Nacht bei Raumtemperatur stehen gelassen. 6,3 g ausgefallener Feststoff* wurde abgesaugt. Die Toluol-Lösung wurde mit Wasser gewaschen, die organische Phase über MgSO₄ getrocknet, filtriert und einrotiert. Es wurden 6,1 g weißer Feststoff isoliert.
Fp. 134°C; LogP [pH 2,3] = 3,96
(* Der ausgefallene Feststoff entspricht auch dem gewünschten Produkt.)

### Beispiel VIII-43

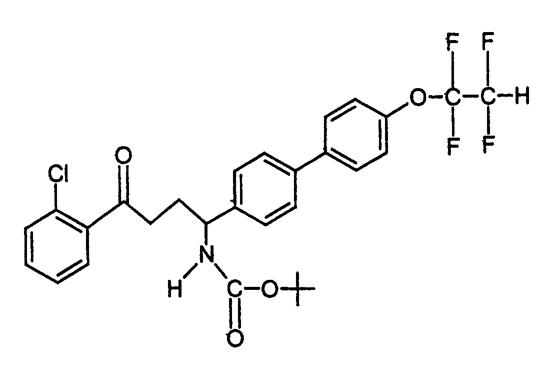

0,36 g Bromchlorbenzol wurden in 10 ml Hexan vorgelegt. Bei -78°C wurden 1,19 ml nBuLi (1,6 M in Hexan) zugetropft. Nach 15 Minuten wurde 0,6 g der Verbindung (IX-43) in 2 ml THF zugetropft. Das Kühlbad wurde entfernt und der Ansatz wurde über Nacht bei Raumtemperatur gerührt. Das Reaktionsgemisch wurde auf 100 ml Wasser gegossen und zweimal mit Essigester extrahiert. Die organische Phase wurde über MgSO₄ getrocknet, filtriert und einrotiert. Das Rohprodukt wurde mittels Säulenchromatographie gereinigt.

| | |
|---|---|
| Ausbeute | 0,12 g |
| HPLC:logP (pH 2,3) | 4,94 |
| Fp. | 130-131°C |

### Beispiel F-III

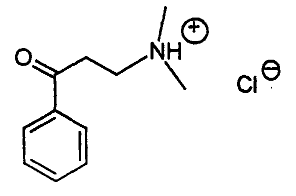

23,4 g (0,25 mol) Dimethylmethylenammoniumchlorid wurden in 150 ml absolutem Acetonitril vorgelegt. Anschließend wurden bei Raumtemperatur 42,5 g (0,275 mol) o-Chloracetophenon zugegeben und abschließend bei Raumtemperatur gerührt. Das Dimethylenammoniumchlorid ging dabei sukzessive in Lösung, das Produkt fiel aus. Es wurde unter Wasserausschluß filtriert und getrocknet. Es wurden 54 g (87 % der Theorie) des Ammoniumchlorid F-III erhalten.
¹H-NMR (400 MHz, CDCl₃) [ppm]: 2,9 (s, 6H, CH₃); 3,5/3,7 (t, 4H, 2xCH₂); 7,4/7,7 (m, 4H, ArH).

### Beispiel F-V-1

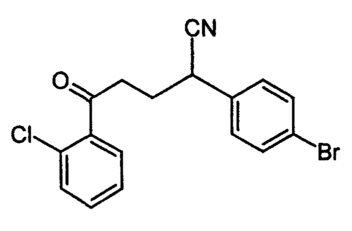

43,5 g (0,175 mol) F-III werden in 60 ml Ethanol p.a. vorgelegt, anschließend ließ man bei Raumtemperatur 36,0 g (0,184 mol) 4-Brombenzylcyanid in 50,7 ml Ethanol p.a. gelöst, zulaufen. Dann wurde 11,2 g (0,2 mol) KOH in 40,0 ml Wasser zugetropft, wobei sich die Lösung leicht erwärmte. Es wurde noch 20 min bei Raumtemperatur nachgerührt und abschließend 16 h zum Rückfluß erhitzt. Nachdem die Reaktionsmischung abgekühlt war, wurde sie auf Wasser gegossen und die Mischung mit Methyl-tert.-Butylether extrahiert. Die organischen Phasen wurden vereinigt, mit 2N HCl, 2N NaOH-Lösung und H₂O gewaschen, getrocknet und eingeengt. Das Rohprodukt wurde an Kieselgel mit Methylenchlorid/Petrolether 2:1 chromatographisch gereinigt. Es wurden 12,8 g (20,2 % d.Th.) der Verbindung erhalten.
¹H-NMR (400 MHz, DMSO-d₆) [ppm]: 2,25 (m, 2H, CHCH₂); 3,1 (t, 2H, COCH₂); 4,4 (t, 1 H, CH); 7,9/7,7 (m, 8H, ArH).

### Beispiel F-VI-1

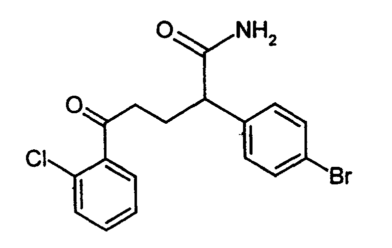

7,5 g (20,7 mmol) F-V-1 wurden 75 ml Ethanol p.a. gelöst und bei Raumtemperatur zu einer Mischung aus 17,7 ml 3N NaOH-Lösung und 11,8 ml 30 % H₂O₂-Lösung getropft, dabei war Gasentwicklung zu beobachten. Es wurde bei Raumtemperatur gerührt, bis die dünnschichtchromatographische Reaktionskontrolle das Ende der Reaktion anzeigte. Anschließend wurde mit Salzsäure neutralisiert, die Lösung mit Methylenchlorid extrahiert und evtl. noch vorhandenes Peroxid mit NaHSO₂-Lösung zerstört. Die organischen Phasen wurden vereinigt, getrocknet und eingeengt. Das Rohprodukt wurde abschließend säulenchromatographisch an Kieselgel mit Cyclohexan/Essigester 4:1 → 1:1 gereinigt. Es wurden 3,1 g (39,3 % der Theorie) der Verbindung F-VI-1 vom Schmelzpunkt 105 bis 107°C erhalten.
¹H-NMR (400 MHz, DMSO-d₆) [ppm]: 1,9/2,2 (m, 2H, CHCH₂); 2,7 (m, 2H, COCH₂); 3,5 (m, 1H, CH); 6,95 (bs, 1H, NH); 7,3/7,6 (m, 9H, ArH+NH).

### Beispiel G(I)-20

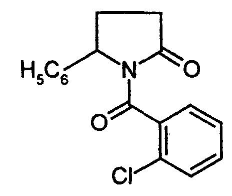

Zu einer Mischung von 25 g (0,155 mol) 5-Phenylpyrrolidin-2-on, 18,3 g (0,23 mol) Pyridin sowie 0,5 g 4-Dimethylaminopyridin wurden unter Rühren bei Raumtemperatur 28,6 g (0,163 mol) 2-Chlorbenzoylchlorid getropft und die Mischung anschließend 1,5 Stunden am Rückfluß erwärmt. Nach dem Erkalten wurde auf Eiswasser gegossen und mit verdünnter Salzsäure pH "5 eingestellt. Die Reaktionsmischung wurde anschließend mit Dichlormethan extrahiert. Die organischen Phasen wurden vereinigt, mit Wasser gewaschen, getrocknet und im Vakuum einrotiert. Der ölige Rückstand wurde mit Cyclohexan verrührt und das Kristallisat abgesaugt. Es wurden 19,5 g (42 % der Theorie) 1-(2-Chlorbenzoyl)-5-phenypyrrolidin-2-on vom Schmelzpunkt 81-83°C erhalten.

### Beispiel G(II)-20

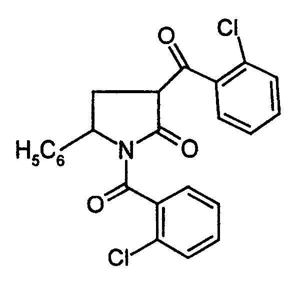

Eine Lösung von 11,4g (67mmol) 2-Chlorbenzoesäuremethylester und 20 g (67 mmol) 1-(2-Chlorbenzoyl)-5-phenyl-pyrrolidin-2-on in 40 ml abs. Tetrahydrofuran wurde unter Rühren bei Raumtemperatur zu einem Gemisch aus 3 g (74 mmol) 60 %igen Natriumhydrid in 150 ml Tetrahydrofuran getropft. Anschließend wurde 3 Stunden auf 50°C erhitzt, anschließend abgekühlt, mit Essigsäure angesäuert und mit Wasser verdünnt. Die organische Phase wurde abgetrennt, getrocknet und im Vakuum eingeengt. Das zähe Öl wurde durch Säulenchromatographie im System Cyclohexan/Essigester (2:1) gereinigt. Es wurden 9,9 g (Ausbeute 33,7 % der Theorie) 1,3-(2-Chlorbenzoyl)-5-phenyl-pyrrolidin-2-on als glasartiges und sprödes Produkt vom log p (pH 2,5) = 3,91 erhalten.

### Biologische Beispiele

### Beispiel A

### Heliothis armigera-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Sojatriebe (Glycine max) werden durch Tauchen in die Wirkstoffzubereitung der gewünschten Konzentration behandelt und mit Raupen des Baumwollkapselwurms (Heliothis armigera) ersetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigt z.B. die Verbindung gemäß Herstellungsbeispiel I-7 in einer beispielhaften Wirkstoffkonzentration von 0,004 % eine Abtötung von 100 % nach 6 Tagen.

### Beispiel B

### Phaedon-Larven-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Larven des Meerettichkäfers (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käferlarven abgetötet wurden.

Bei diesem Test zeigt z.B. die Verbindung gemäß Herstellungsbeispiel I-7 bei einer beispielhaften Wirkstoffkonzentration von 0,004 % eine Abtötung von 100 % nach 6 Tagen.

### Beispiel C

### Plutella-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella xylostella) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigt z.B. die Verbindung gemäß Herstellungsbeispiel I-7 bei einer beispielhaften Wirkstoffkonzentration von 0,004 % eine Abtötung von 100 % nach 6 Tagen.

### Beispiel D

### Spodoptera frugiperda-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen des Heerwurms (Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigt z.B. die Verbindung gemäß Herstellungsbeispiele I-7 und I-8 bei einer beispielhaften Wirkstoffkonzentration von höchstens 0,1 % eine Abtötung von 100 % nach 6 Tagen.

### Beispiel E

### Tetranychus-Test (OP-resistent / Tauchbehandlung)

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenpflanzen (Phaseolus vulgaris), die stark von allen Stadien der gemeinen Spinnmilbe (Tetranychus urticae) befallen sind, werden in eine Wirkstoffzubereitung der gewünschten Konzentration getaucht.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigt z.B. die Verbindung gemäß Herstellungsbeispiele I-7, I-8 und I-20 bei einer beispielhaften Wirkstoffkonzentration von höchstens 0,1 % eine Abtötung von 100 % nach 6 Tagen.

## Patentansprüche

1. Verbindungen der Formel (I) 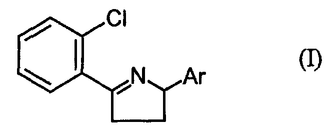 in welcher
Ar für den Rest 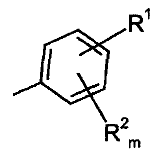 steht, wobei
m für 0, 1, 2, 3 steht,
R¹ für einen Substituenten in meta- oder para-Position aus der Reihe Wasserstoff, Halogen, Cyano, Tri-(C₁-C₆-alkyl)-silyl, -CO-NR⁴R⁵, Tetrahydropyranyl oder eine der folgenden Gruppierungen
(l) -X - A
(m) -B - Z - D
(n) -Y - E
steht,
R² für Wasserstoff, Halogen, Cyano, Nitro, C₁-C₁₆-Alkyl, C₁-C₁₆-Alkoxy, C₁-C₆-Halogenalkyl, C₁-C₆-Halogenalkoxy, C₁-C₈-Alkoxy-C₁-C₈-alkoxy oder -S(O)ₒR³ steht,
o für 0, 1 oder 2 steht,
R³ für gegebenenfalls durch Fluor oder Chlor substituiertes C₁-C₆-Alkyl steht,
R⁴ und R⁵ unabhängig voneinander für Wasserstoff, C₁-C₆-Alkyl, C₁-C₆-Halogenalkyl oder für jeweils gegebenenfalls einfach bis dreifach durch Reste aus der Liste W¹ substituiertes Phenyl oder Phenyl-C₁-C₄-alkyl stehen,
X für eine direkte Bindung, Sauerstoff, Schwefel, Carbonyl, Carbonyloxy, Oxycarbonyl, C₁-C₄-Alkylen, C₂-C₄-Alkenylen, C₂-C₄-Alkinylen, C₁-C₄-Alkylenoxy, C₁-C₄-Oxyalkylen, C₁-C₄-Thioalkylen, C₁-C₄-Alkylendioxy oder Di-C₁-C₄-alkylsilylen steht,
A für jeweils gegebenenfalls einfach bis vierfach durch Reste aus der Liste W¹ substituiertes Phenyl, Naphthyl oder Tetrahydronaphthyl oder für jeweils gegebenenfalls einfach bis vierfach durch Reste aus der Liste W² substituiertes 5- bis 10-gliedriges, 1 oder 2 aromatische Ringe enthaltendes Heterocyclyl mit 1 bis 4 Heteroatomen, welches 0 bis 4 Stickstoffatome, 0 bis 2 Sauerstoffatome und 0 bis 2 Schwefelatome enthält, (insbesondere Furyl, Benzofuryl, Thienyl, Benzothienyl, Oxazolyl, Benzoxazolyl, Thiazolyl, Benzthiazolyl, Pyrrolyl, Pyridyl, Pyrimidyl, 1,3,5-Triazinyl, Chinolinyl, Isochinolinyl, Indolyl, Purinyl, Benzodioxolyl, Indanyl, Benzodioxanyl oder Chromanyl) steht,
B für gegebenenfalls einfach oder zweifach durch Reste aus der Liste W¹ substituiertes p-Phenylen steht,
Z für Sauerstoff oder Schwefel steht,
D für Wasserstoff, C₁-C₁₆-Alkyl, C₂-C₁₆-Alkenyl, C₂-C₆-Alkinyl, C₁-C₁₆-Halogenalkyl, C₂-C₁₆-Halogenalkenyl, jeweils gegebenenfalls durch Halogen, C₁-C₄-Alkyl, C₂-C₄-Alkenyl, C₂-C₄-Halogenalkenyl, Phenyl, Styryl, Halogenphenyl oder Halogenstyryl substituiertes C₃-C₈-Cycloalkyl oder C₃-C₈-Cycloalkyl-C₁-C₆-alkyl, für jeweils gegebenenfalls durch Halogen oder C₁-C₄-Alkyl substituiertes C₅-C₈-Cycloalkenyl oder C₅-C₈-Cycloalkenyl-C₁-C₄-alkyl, für jeweils gegebenenfalls durch Nitro, Halogen, C₁-C₆-Alkyl, C₁-C₆-Alkoxy, C₁-C₆-Halogenalkyl oder C₁-C₆-Halogenalkoxy substituiertes Phenyl-C₁-C₆-alkyl, Naphthyl-C₁-C₆-alkyl, Tetrahydronaphthyl-C₁-C₆-alkyl oder 5- oder 6-ringgliedriges Hetaryl-C₁-C₆-alkyl mit 1 oder 2 Heteroatomen aus der Reihe Stickstoff, Sauerstoff und Schwefel (insbesondere Furylmethyl, Thienylmethyl, Pyrrolylmethyl, Oxazolylmethyl, Isoxazolylmethyl, Thiazolylmethyl oder Pyridylmethyl), für -CO-R⁶, -CO-NR⁷R⁸ oder für die Gruppierung
-(CH₂)ₚ - (CR⁹R¹⁰)_{q} - (CH₂)ᵣ - G
steht,
Z und D können auch gemeinsam für jeweils gegebenenfalls durch Nitro, Halogen, C₁-C₆-Alkyl, C₁-C₆-Alkoxy, C₁-H₆-Halogenalkyl oder C₁-C₆-Halogenalkoxy substituiertes Phenoxy-C ₁-C₄-alkyl, stehen,
Y für eine direkte Bindung, Sauerstoff, Schwefel, Carbonyl, Carbonyloxy, Oxycarbonyl, C₁-C₄-Alkylen, C₂-C₄-Alkenylen, C₂-C₄-Alkinylen, C₁-C₄-Alkylenoxy, C₁-C₄-Oxyalkylen, C₁-C₄-Thioalkylen, C₁-C₄-Alkylendioxy oder für gegebenenfalls einfach oder zweifach durch Reste aus der Liste W¹ substituiertes p-Phenylen steht,
E für Wasserstoff, C₁-C₁₆-Alkyl, C₂-C₁₆-Alkenyl, C₂-C₆-Alkinyl, C₁-C₁₆-Halogenalkyl, C₂-C₁₆-Halogenalkenyl, gegebenenfalls durch Halogen, C₁-C₄-Alkyl, C₂-C₄-Alkenyl, C₂-C₄-Halogenalkenyl, Phenyl, Styryl, Halogenphenyl oder Halogenstyryl substituiertes C₃-C₈-Cycloalkyl, für gegebenenfalls durch Halogen oder C₁-C₄-Alkyl substituiertes C₅-C₈-Cycloalkenyl, für gegebenenfalls einfach bis vierfach durch Reste aus der Liste W¹ substituiertes Phenyl oder für jeweils gegebenenfalls einfach bis vierfach durch Reste aus der Liste W² substituiertes 5- oder 6-gliedriges Hetaryl mit 1 oder 2 Heteroatomen aus der Reihe Stickstoff, Sauerstoff und Schwefel (insbesondere Furyl, Thienyl, Pyrrolyl, Oxazolyl, Isoxazolyl, Thiazolyl oder Pyridyl) oder für die Gruppierung
-(CH₂)ₚ - (CR⁹R¹⁰)_{q} - (CH₂)ᵣ - G
steht,
R⁶ für C₁-C₁₂-Alkyl, C₁-C₁₂-Alkoxy, C₂-C₁₂-Alkenyl, C₂-C₁₂-Alkenyloxy, jeweils gegebenenfalls durch Halogen, C₁-C₄-Alkyl, C₂-C₄-Alkenyl, C₁-C₄-Halogenalkyl oder C₂-C₄-Halogenalkenyl substituiertes C₃-C₈-Cycloalkyl, C₃-C₈-Cycloalkyloxy oder C₃-C₈-Cycloalkyl-C₁-C₆-alkyloxy oder für jeweils gegebenenfalls einfach bis vierfach durch Nitro, Halogen, C₁-C₁₂-Alkyl, C₁-C₁₂-Alkoxy, C₁-C₁₂-Halogenalkyl oder C₁-C₁₂-Halogenalkoxy substituiertes Phenyl oder Naphthyl steht,
R⁷ für Wasserstoff oder C₁-C₁₂-Alkyl steht,
R⁸ für C₁-C₁₂-Alkyl, C₁-C₁₂-Halogenalkyl, jeweils gegebenenfalls durch Halogen, C₁-C₄-Alkyl, C₂-C₄-Alkenyl, C₁-C₄-Halogenalkyl oder C₂-C₄-Halogenalkenyl substituiertes C₃-C₈-Cycloalkyl oder C₃-C₈-Cycloalkyl-C₁-C₆-alkyl oder für jeweils gegebenenfalls einfach bis vierfach durch Halogen, C₁-C₁₂-Alkyl, C₁-C₁₂-Alkoxy, C₁-C₁₂-Halogenalkyl oder C₁-C₁₂-Halogenalkoxy substituiertes Phenyl oder Phenyl-C₁-C₆-alkyl steht,
p, q und r unabhängig voneinander für 0, 1, 2 oder 3, wobei deren Summe kleiner 6 ist stehen,
R⁹ und R¹⁰ unabhängig voneinander für Wasserstoff oder C₁-C₄-Alkyl stehen,
G für Cyano, für einen gegebenenfalls einfach bis dreifach durch Halogen, C₁-C₄-Alkyl oder C₁-C₄-Halogenalkyl und gegebenenfalls an der Verknüpfungsstelle durch den Rest R¹¹ substituierten 5- oder 6-gliedrigen Heterocyclus mit 1 bis 3 gleichen oder verschiedenen Heteroatomen aus der Reihe Stickstoff, Sauerstoff und Schwefel (insbesondere 5,6-Dihydrodioxazin-2-yl, 3-Pyridyl, 3-Furyl, 3-Thienyl, 2-Thiazolyl, 5-Thiazolyl, 2-Dioxolanyl, 1,3-Dioxan-2-yl, 2-Dithiolanyl, 1,3-Dithian-2-yl oder 1,3-Thioxan-2-yl) oder eine der folgenden Gruppierungen steht:
(a) ―CO―R¹¹
(b) ―CO―OR¹²
(c) ―CO―NR¹³R¹⁴
(d) ―CS―NR¹³R¹⁴ 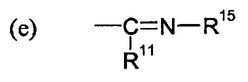 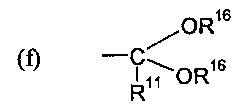 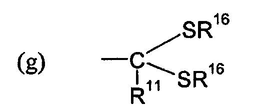 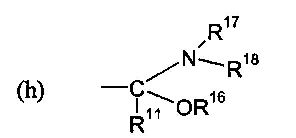 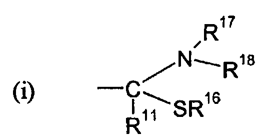 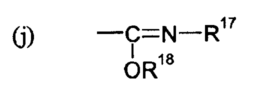 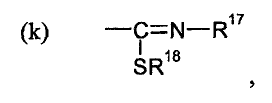
R¹¹ für Wasserstoff, C₁-C₆-Alkyl, C₂-C₆-Alkenyl, C₁-C₄-Halogenalkyl, C₂-C₆-Halogenalkenyl, gegebenenfalls durch Halogen, C₁-C₄-Alkyl oder C₁-C₄-Halogenalkyl substituiertes C₃-C₆-Cycloalkyl oder für gegebenenfalls einfach bis fünffach durch C₁-C₄-Alkylcarbonylamino, C₁-C₄-Alkylcarbonyl-C₁-C₄-alkylamino und/oder Reste aus der Liste W³ substituiertes Phenyl steht,
R¹² für Wasserstoff, C₁-C₄-Alkyl, C₂-C₆-Alkenyl, C₁-C₄-Halogenalkyl, C₂-C₆-Halogenalkenyl, jeweils gegebenenfalls durch Halogen, C₁-C₄-Alkyl oder C₁-C₄-Halogenalkyl substituiertes C₃-C₆-Cycloalkyl oder C₃-C₆-Cycloalkyl-C₁-C₄-alkyl oder für gegebenenfalls einfach bis vierfach durch Reste aus der Liste W³ substituiertes C₆-C₁₀-Aryl-C₁-C₄-alkyl (insbesondere Phenyl-C₁-C₄-alkyl oder Naphthyl-C₁-C₄-alkyl) steht,
R¹³ und R¹⁴ unabhängig voneinander für Wasserstoff, C₁-C₄-Alkyl, C₃-C₆-Alkenyl, C₁-C₄-Halogenalkyl, C₃-C₆-Halogenalkenyl, C₁-C₄-Alkoxy, jeweils gegebenenfalls durch Halogen, C₁-C₄-Alkyl oder C₁-C₄-Halogenalkyl substituiertes C₃-C₆-Cycloalkyl oder C₃-C₆-Cycloalkyl-C₁-C₄-alkyl, für jeweils gegebenenfalls einfach bis fünffach durch Reste aus der Liste W³ substituiertes Phenyl oder Phenyl-C₁-C₄-alkyl, für -OR¹² oder -NR¹¹R¹² oder gemeinsam für eine Alkylenkette mit 4 bis 6 Gliedern, in welcher gegebenenfalls eine Methylengruppe durch Sauerstoff ersetzt ist stehen,
R¹⁵ für -OR¹², -NR¹¹R¹² oder -N(R¹¹)-COOR¹² steht,
R¹⁶, R¹⁷ und R¹⁸ unabhängig voneinander für C₁-C₆-Alkyl stehen,
W¹ für Wasserstoff, Halogen, Cyano, Formyl, Nitro, C₁-C₆-Alkyl, Tri-C₁-C₄-alkylsilyl, C₁-C₁₆-Alkoxy, C₁-C₆-Halogenalkyl, C₁-C₆-Halogenalkoxy, C₂-C₆-Halogenalkenyloxy, C₁-C₆-Alkylcarbonyl, C₁-C₁₆-Alkoxycarbonyl, Pentafluorthio oder -S(O)ₒR³ steht,
W² für Halogen, Cyano, Formyl, Nitro, C₁-C₆-Alkyl, Tri-C₁-C₄-alkylsilyl, C₁-C₁₆-Alkoxy, C₁-C₆-Halogenalkyl, C₁-C₆-Halogenalkoxy, C₁-C₆-Alkylcarbonyl, C₁-C₁₆-Alkoxycarbonyl, Pentafluorthio, -S(O)ₒR³ oder -C(R¹¹)=N-R¹⁵ steht,
W³ für Halogen, Cyano, Nitro, C₁-C₄-Alkyl, C₁-C₄-Alkoxy, C₁-C₄-Halogenalkyl, C₁-C₄-Halogenalkoxy, Di-C₁-C₄-alkylamino, -S(O)ₒR³, -COOR¹⁹ oder -CONR²⁰R²¹ steht,
R¹⁹ für Wasserstoff, C₁-C₄-Alkyl, C₁-C₄-Halogenalkyl, gegebenenfalls durch Halogen, C₁-C₄-Alkyl oder C₁-C₄-Halogenalkyl substituiertes C₃-C₇-Cycloalkyl oder für gegebenenfalls einfach bis fünffach durch Reste aus der Liste W⁴ substituiertes Phenyl steht,
R²⁰ und R²¹ unabhängig voneinander für Wasserstoff, C₁-C₄-Alkyl, C₃-C₆-Alkenyl, C₁-C₄-Halogenalkyl, C₃-C₆-Halogenalkenyl, C₁-C₄-Alkoxy, jeweils gegebenenfalls durch Halogen, C₁-C₄-Alkyl oder C₁-C₄-Halogenalkyl substituiertes C₃-C₆-Cycloalkyl oder C₃-C₆-Cycloalkyl-C₁-C₄-alkyl oder für jeweils gegebenenfalls einfach bis fünffach durch Reste aus der Liste W⁴ substituiertes Phenyl oder Phenyl-C₁-C₄-alkyl, für -OR¹⁶ oder -NR¹⁷R¹⁸ oder gemeinsam für eine Alkylenkette mit 4 bis 6 Gliedern, in welcher gegebenenfalls eine Methylengruppe durch Sauerstoff ersetzt ist stehen, und
W⁴ für Halogen, Cyano, Nitro, C₁-C₆-Alkyl, C₁-C₆-Alkoxy, C₁-C₆-Halogenalkyl, C₁-C₆-Halogenalkoxy, Di-C₁-C₄-alkylamino, C₁-C₆-Alkoxycarbonyl, Di-C₁-C₆-alkylaminocarbonyl oder -S(O)ₒR³ steht,

2. Verbindungen der Formel (I) gemäß Anspruch 1, in welcher
Ar für den Rest 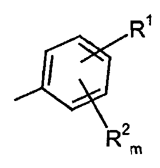 steht,
m für 0, 1 oder 2 steht,
R¹ für einen Substituenten in meta- oder para-Position aus der Reihe Wasserstoff, Fluor, Chlor, Brom, Iod, Cyano, Tri-(C₁-C₄-alkyl)-silyl, - CO-NR⁴R⁵, Tetrahydropyranyl oder eine der folgenden Gruppierungen
(l) - X - A
(m) - B - Z - D
(n) - Y - E
steht,
R² für Wasserstoff, Fluor, Chlor, Brom, Iod, Cyano, Nitro, C₁-C₁₆-Alkyl, C₁-C₁₆-Alkoxy, jeweils durch Fluor oder Chlor substituiertes C₁-C₆-Alkyl oder C₁-C₆-Alkoxy, für C₁-C₈-Alkoxy-C₁-C₈-alkoxy, oder -S(O)ₒR³ steht,
o für 0, 1 oder 2 steht,
R³ für C₁-C₄-Alkyl oder jeweils durch Fluor oder Chlor substituiertes Methyl oder Ethyl steht
R⁴ und R⁵ unabhängig voneinander für Wasserstoff, C₁-C₆-Alkyl, durch Fluor oder Chlor substituiertes C₁-C₆-Alkyl oder für jeweils gegebenenfalls einfach oder zweifach durch Reste aus der Liste W¹ substituiertes Phenyl oder Benzyl stehen,
X für eine direkte Bindung, Sauerstoff, Schwefel, Carbonyl, Carbonyloxy, Oxycarbonyl, C₁-C₄-Alkylen, C₂-C₄-Alkenylen, C₂-C₄-Alkinylen, C₁-C₄-Alkylenoxy, C₁-C₄-Oxyalkylen, C₁-C₄-Thioalkylen, C₁-C₄-Alkylendioxy oder Di-C₁-C₄-alkylsilylen steht,
A für jeweils gegebenenfalls einfach bis dreifach durch Reste aus der Liste W¹ substituiertes Phenyl, Naphthyl oder Tetrahydronaphthyl oder für jeweils gegebenenfalls einfach bis dreifach durch Reste aus der Liste W² substituiertes 5- bis 10-gliedriges, 1 oder 2 aromatische Ringe enthaltendes Heterocyclyl mit 1 bis 4 Heteroatomen, welches 0 bis 4 Stickstoffatome, 0 bis 2 Sauerstoffatome und 0 bis 2 Schwefelatome enthält, (insbesondere Furyl, Benzofuryl, Thienyl, Benzothienyl, Oxazolyl, Benzoxazolyl, Thiazolyl, Benzthiazolyl, Pyrrolyl, Pyridyl, Pyrimidyl, 1,3,5-Triazinyl, Chinolinyl, Isochinolinyl, Indolyl, Purinyl, Benzodioxolyl, Indanyl, Benzodioxanyl oder Chromanyl) steht,
B für gegebenenfalls einfach oder zweifach durch Reste aus der Liste W¹ substituiertes p-Phenylen steht,
Z für Sauerstoff oder Schwefel steht,
D für Wasserstoff, C₁-C₁₆-Alkyl, C₂-C₁₆-Alkenyl, C₂-C₆-Alkinyl, jeweils durch Fluor oder Chlor substituiertes C₁-C₄-Alkyl oder C₂-C₄-Alkenyl, für jeweils gegebenenfalls durch Fluor, Chlor, Brom, C₁-C₄-Alkyl, C₂-C₄-Alkenyl, durch Fluor oder Chlor substituiertes C₂-C₄-Alkenyl, durch Phenyl, Styryl, jeweils durch Fluor, Chlor oder Brom substituiertes Phenyl oder Styryl substituiertes C₃-C₆-Cycloalkyl oder C₃-C₆-Cycloalkyl-C₁-C₄-alkyl, für jeweils gegebenenfalls durch Fluor, Chlor, Brom oder C₁-C₄-Alkyl substituiertes C₅-C₆-Cycloalkenyl oder C₅-C₆-Cycloalkenyl-C₁-C₄-alkyl, für jeweils gegebenenfalls durch Nitro, Fluor, Chlor, Brom, C₁-C₆-Alkyl, C₁-C₆-Alkoxy, jeweils durch Fluor oder Chlor substituiertes C₁-C₄-Alkyl oder C₁-C₄-Alkoxy substituiertes Phenyl-C₁-C₄-alkyl, Naphthyl-C₁-C₄-alkyl, Tetrahydronaphthyl-C₁-C₆-alkyl oder 5- oder 6-ringgliedriges Hetaryl-C₁-C₄-alkyl mit 1 oder 2 Heteroatomen aus der Reihe Stickstoff, Sauerstoff und Schwefel, insbesondere Furylmethyl, Thienylmethyl, Pyrrolylmethyl, Oxazolylmethyl, Isoxazolylmethyl, Thioazolylmethyl oder Pyridylmethyl) für -CO-R⁶, -CO-NR⁷R⁸ oder die Gruppierung
- (CH₂)ₚ - (CR⁹R¹⁰)_{q} - (CH₂)ᵣ - G
steht,
Z und D können auch gemeinsam für gegebenenfalls durch Nitro, Fluor, Chlor, Brom, C₁-C₄-Alkyl, C₁-C₄-Alkoxy, jeweils durch Fluor oder Chlor substituiertes C₁-C₄-Alkyl oder C₁-C₄-Alkoxy substituiertes Phenoxy-C₁-C₃-alkyl stehen,
Y für eine direkte Bindung, Sauerstoff, Schwefel, Carbonyl, Carbonyloxy, Oxycarbonyl, C₁-C₄-Alkylen, C₂-C₄-Alkenylen, C₂-C₄-Alkinylen, C₁-C₄-Alkylenoxy, C₁-C₄-Oxyalkylen, C₁-C₄-Thioalkylen, C₁-C₄-Alkylendioxy oder für gegebenenfalls einfach oder zweifach durch Reste aus der Liste W¹ substituiertes p-Phenylen steht,
E für Wasserstoff, C₁-C₁₆-Alkyl, C₂-C₁₆-Alkenyl, C₂-C₆-Alkinyl, jeweils durch Fluor oder Chlor substituiertes C₁-C₄-Alkyl oder C₂-C₄-Alkenyl, für gegebenenfalls durch Fluor, Chlor, Brom, C₁-C₄-Alkyl, C₂-C₄-Alkenyl, durch Fluor oder Chlor substituiertes C₂-C₄-Alkenyl, durch Phenyl, Styryl oder jeweils durch Fluor, Chlor oder Brom substituiertes Phenyl oder Styryl substituiertes C₃-C₆-Cycloalkyl, für gegebenenfalls durch Fluor, Chlor; Brom oder C₁-C₄-Alkyl substituiertes C₅-C₆-Cycloalkenyl, für gegebenenfalls einfach bis dreifach durch Reste aus der Liste W¹ substituiertes Phenyl oder für jeweils gegebenenfalls einfach oder zweifach durch Reste aus der Liste W² substituiertes 5- oder 6-gliedriges Hetaryl mit 1 oder 2 Heteroatomen aus der Reihe Stickstoff, Sauerstoff und Schwefel (insbesondere Furyl, Thienyl, Pyrrolyl, Oxazolyl, Isoxazolyl, Thiazolyl oder Pyridyl) oder für die Gruppierung
- (CH₂)ₚ - (CR⁹R¹⁰)_{q} - (CH₂)ᵣ - G
steht,
R⁶ für C₁-C₆-Alkyl, C₁-C₆-Alkoxy, C₂-C₆-Alkenyl, C₂-C₆-Alkenyloxy, jeweils gegebenenfalls durch Fluor, Chlor, C₁-C₃-Alkyl oder jeweils durch Fluor oder Chlor substituiertes C₁-C₂-Alkyl oder C₂-C₃-Alkenyl substituiertes C₃-C₆-Cycloalkyl, C₃-C₆-Cycloalkyloxy oder C₃-C₆-Cycloalkyl-C₁-C₂-alkyloxy oder für gegebenenfalls einfach oder zweifach durch Fluor, Chlor, Brom, Iod, C₁-C₄-Alkyl, C₁-C₄-Alkoxy oder jeweils durch Fluor oder Chlor substituiertes C₁-C₃-Alkyl oder C₁-C₄-Alkoxy substituiertes Phenyl steht,
R⁷ für Wasserstoff oder C₁-C₄-Alkyl steht,
R⁸ für C₁-C₄-Alkyl oder für jeweils gegebenenfalls einfach oder zweifach durch Fluor, Chlor, Brom, C₁-C₄-Alkyl oder jeweils durch Fluor oder Chlor substituiertes C₁-C₄-Alkyl oder C₁-C₄-Alkoxy substituiertes Phenyl oder Benzyl steht,
p, q und r unabhängig voneinander für 0, 1, 2 oder 3, wobei deren Summe kleiner 6 ist stehen,
R⁹ und R¹⁰ unabhängig voneinander für Wasserstoff oder C₁-C₄-Alkyl stehen,
G für Cyano, für einen gegebenenfalls einfach bis dreifach durch Fluor, Chlor, Brom, C₁-C₄-Alkyl oder durch Fluor oder Chlor substituiertes C₁-C₄-Alkyl und gegebenenfalls an der Verknüpfungsstelle durch den Rest R¹¹ substituierten 5- oder 6-gliedrigen Heterocyclus mit 1 bis 3 gleichen oder verschiedenen Heteroatomen aus der Reihe Stickstoff, Sauerstoff und Schwefel (insbesondere 5,6-Dihydrodioxazin-2-yl, 3-Pyridyl, 3-Furyl, 3-Thienyl, 2-Thiazolyl, 5-Thiazolyl, 2-Dioxolanyl, 1,3-Dioxan-2-yl, 2-Dithiolanyl, 1,3-Dithian-2-yl oder 1,3-Thioxan-2-yl) oder eine der folgenden Gruppierungen:
(a) ―CO―R¹¹
(b) ―CO―OR¹²
(c) ―CO―NR¹³R¹⁴
(d) ―CS―NR¹³R¹⁴ 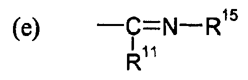 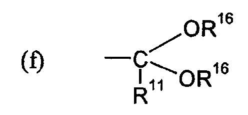 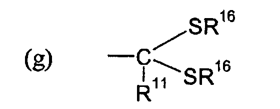 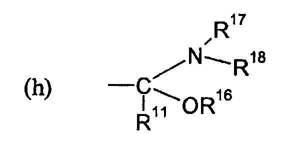 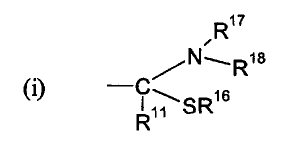 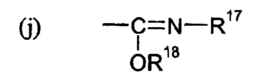 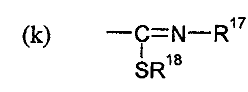
steht,
R¹¹ für Wasserstoff, C₁-C₆-Alkyl, C₂-C₆-Alkenyl, jeweils durch Fluor oder Chlor substituiertes C₁-C₄-Alkyl oder C₂-C₆-Alkenyl, gegebenenfalls durch Fluor, Chlor, C₁-C₄-Alkyl oder durch Fluor oder Chlor substituiertes C₁-C₄-Alkyl substituiertes C₃-C₆-Cycloalkyl oder für gegebenenfalls einfach bis dreifach durch C₁-C₄-Alkylcarbonylamino, C₁-C₄-Alkylcarbonyl-C₁-C₄-alkylamino und/oder Reste aus der Liste W³ substituiertes Phenyl steht,
R¹² für Wasserstoff, C₁-C₄-Alkyl, C₃-C₆-Alkenyl, jeweils durch Fluor oder Chlor substituiertes C₁-C₄-Alkyl oder C₃-C₆-Alkenyl, jeweils gegebenenfalls durch Fluor, Chlor, C₁-C₄-Alkyl oder durch Fluor oder Chlor substituiertes C₁-C₄-Alkyl substituiertes C₃-C₆-Cycloalkyl oder C₃-C₆-Cycloalkyl-C₁-C₄-alkyl oder für jeweils gegebenenfalls einfach bis dreifach durch Reste aus der Liste W³ substituiertes Phenyl-C₁-C₄-alkyl oder Naphthyl-C₁-C₄-alkyl steht,
R¹³ und R¹⁴ unabhängig voneinander für Wasserstoff, C₁-C₄-Alkyl, C₃-C₆-Alkenyl, jeweils durch Fluor oder Chlor substituiertes C₁-C₄-Alkyl oder C₃-C₆-Alkenyl, für C₁-C₄-Alkoxy, jeweils gegebenenfalls durch Fluor, Chlor, C₁-C₄-Alkyl oder durch Fluor oder Chlor substituiertes C₁-C₄-Alkyl substituiertes C₃-C₆-Cycloalkyl oder C₃-C₆-Cycloalkyl-C₁-C₄-alkyl, für jeweils gegebenenfalls einfach bis dreifach durch Reste aus der Liste W³ substituiertes Phenyl oder Phenyl-C₁-C₄-alkyl, für -OR¹² oder -NR¹¹R¹² oder gemeinsam für -(CH₂)₅-, -(CH₂)₆- oder -(CH₂)₂-O-(CH₂)₂- stehen,
R¹⁵ für -OR¹², -NR¹¹R¹² oder -N(R¹¹)-COOR¹² steht,
R¹⁶, R¹⁷ und R¹⁸ unabhängig voneinander für C₁-C₄-Alkyl stehen,
W¹ für Wasserstoff, Fluor, Chlor, Brom, Iod, Cyano, Formyl, Nitro, C₁-C₄-Alkyl, C₁-C₄-Alkoxy, jeweils durch Fluor oder Chlor substituiertes C₁-C₄-Alkyl oder C₁-C₄-Alkoxy, für C₁-C₄-Alkylcarbonyl, C₁-C₄-Alkoxycarbonyl oder -S(O)ₒR³ steht,
W² für Fluor, Chlor, Brom, Cyano, Formyl, Nitro, C₁-C₄-Alkyl, C₁-C₄-Alkoxy, jeweils durch Fluor oder Chlor substituiertes C₁-C₄-Alkyl oder C₁-C₄-Alkoxy, für C₁-C₄-Alkylcarbonyl, C₁-C₄-Alkoxycarbonyl, -S(O)ₒR³ oder -C(R¹¹)=N-R¹⁵ steht,
W³ steht für Fluor, Chlor, Brom, Cyano, Nitro, C₁-C₄-Alkyl, C₁-C₄-Alkoxy, jeweils durch Fluor oder Chlor substituiertes C₁-C₄-Alkyl oder C₁-C₄-Alkoxy, für Di-C₁-C₄-alkylamino, -S(O)ₒR³, -COOR¹⁹ oder -CONR²⁰R²¹.
R¹⁹ für Wasserstoff, C₁-C₄-Alkyl, durch Fluor oder Chlor substituiertes C₁-C₄-Alkyl, gegebenenfalls durch Fluor, Chlor, C₁-C₄-Alkyl oder durch Fluor oder Chlor substituiertes C₁-C₄-Alkyl substituiertes C₃-C₆-Cycloalkyl oder für gegebenenfalls einfach bis dreifach durch Reste aus der Liste W⁴ substituiertes Phenyl steht,
R²⁰ und R²¹ unabhängig voneinander für Wasserstoff, C₁-C₄-Alkyl, C₃-C₆-Alkenyl, jeweils durch Fluor oder Chlor substituiertes C₁-C₄-Alkyl oder C₃-C₆-Alkenyl, für C₁-C₄-Alkoxy, jeweils gegebenenfalls durch Fluor, Chlor, C₁-C₄-Alkyl oder durch Fluor oder Chlor substituiertes C₁-C₄-Alkyl substituiertes C₃-C₆-Cycloalkyl oder C₃-C₆-Cycloalkyl-C₁-C₄-alkyl oder für jeweils gegebenenfalls einfach bis dreifach durch Reste aus der Liste W⁴ substituiertes Phenyl oder Phenyl-C₁-C₄-alkyl, für -OR¹⁶ oder -NR¹⁷R¹⁸ oder gemeinsam für -(CH₂)₅-, -(CH₂)₆- oder -(CH₂)₂-O-(CH₂)₂- stehen, und
W⁴ für Fluor, Chlor, Brom, Cyano, Nitro, C₁-C₄-Alkyl, C₁-C₄-Alkoxy, jeweils durch Fluor oder Chlor substituiertes C₁-C₄-Alkyl oder C₁-C₄-Alkoxy, Di-C₁-C₄-alkylamino, C₁-C₄-Alkoxycarbonyl, Di-C₁-C₆-alkylaminocarbonyl oder -S(O)ₒR³ steht.

3. Verbindungen der Formel (I) gemäß Anspruch 1, in welcher
Ar für den Rest steht, 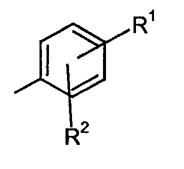
R¹ für einen Substituenten in meta- oder para-Position aus der Reihe Wasserstoff, Fluor, Chlor, Brom, Iod, Cyano, -CO-NR⁴R⁵, Tetrahydropyranyl oder eine der folgenden Gruppierungen
(l) - X - A 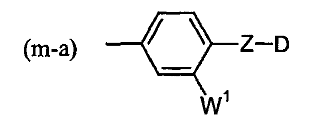
(n) - Y - E
steht,
R² für Wasserstoff, Fluor, Chlor, Brom, Methyl, Ethyl, Methoxy, Ethoxy, Methylthio, Ethylthio, Trifluormethyl, Difluormethoxy, Trifluormethoxy oder Trifluormethylthio steht,
o für 0 oder 2 steht,
R³ für Methyl, Ethyl, n-Propyl, Isopropyl, Difluormethyl oder Trifluormethyl steht,
R⁴ und R⁵ unabhängig voneinander für Wasserstoff, Methyl, Ethyl, n-Propyl, Isopropyl, n-Butyl, Isobutyl, sec.-Butyl, tert.-Butyl oder für jeweils gegebenenfalls einfach durch einen Rest aus der Liste W¹ substituiertes Phenyl oder Benzyl stehen,
X steht für eine direkte Bindung, Sauerstoff, Schwefel, Carbonyl, -CH₂-, -(CH₂)₂-, -CH=CH- (E oder Z), -C≡C-, -CH₂O-, -(CH₂)₂O-, -CH(CH₃)O-, -OCH₂-, -O(CH₂)₂-, -SCH₂-, -S(CH₂)₂-, -SCH(CH₃)-, C₁-C₄-Alkylendioxy, insbesondere -OCH₂O-, -O(CH₂)₂O- oder -OCH(CH₃)O-.
A für gegebenenfalls einfach oder zweifach durch Reste aus der Liste W¹ substituiertes Phenyl oder für jeweils gegebenenfalls einfach oder zweifach durch Reste aus der Liste W² substituiertes Furyl, Benzofuryl, Thienyl, Benzothienyl, Oxazolyl, Benzoxazolyl, Thiazolyl, Benzthiazolyl, Pyrrolyl, Pyridyl, Pyrimidyl, 1,3,5-Triazinyl, Chinolinyl, Isochinolinyl, Indolyl, Purinyl, Benzodioxolyl, Indanyl, Benzodioxanyl oder Chromanyl steht,
Z für Sauerstoff oder Schwefel steht,
D für Wasserstoff, Methyl, Ethyl, n-Propyl, Isopropyl, n-Butyl, Isobutyl, sec.-Butyl, tert.-Butyl, die isomeren Pentyle, die isomeren Hexyle, n-Heptyl, n-Octyl, n-Isooctyl, n-Nonyl, n-Decyl, n-Undecyl, n-Dodecyl, n-Tridecyl, n-Tetradecyl, n-Pentadecyl, n-Hexadecyl, 2-Propenyl, Butenyl, Pentenyl, Hexenyl, Propargyl, Butinyl, Pentinyl, -CF₃, -CHF₂, -CClF₂, -CF₂CHFCl, -CF₂CH₂F, -CF₂CHF₂, -CF₂CCl₃, -CH₂CF₃, -CF₂CHFCF₃, -CH₂CF₂CHF₂, -CH₂CF₂CF₃, für jeweils gegebenenfalls einfach bis dreifach durch Fluor, Chlor, Brom, Methyl, Ethyl, n-Propyl, Isopropyl, n-Butyl, Isobutyl, sec.-Butyl, tert.-Butyl, Ethenyl, 1-Propenyl, 2,2-Dimethylethenyl, -CH=CCl₂, Phenyl, Styryl, jeweils durch Fluor, Chlor oder Brom substituiertes Phenyl oder 4-Chlorstyryl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, für jeweils gegebenenfalls durch Fluor, Chlor, Methyl, Ethyl, n-Propyl, Isopropyl, n-Butyl, Isobutyl, sec.-Butyl oder tert.-Butyl substituiertes Cyclopentenyl, Cyclohexenyl, Cyclohexenylmethyl oder Cyclopentenylmethyl, für jeweils gegebenenfalls einfach oder zweifach durch Nitro, Fluor, Chlor, Brom, Methyl, Ethyl, n-Propyl, Isopropyl, n-Butyl, Isobutyl, sec.-Butyl, tert.-Butyl, Methoxy, Ethoxy, n-Propoxy, Isopropoxy, n-Butoxy, Isobutoxy, sec.-Butoxy, tert.-Butoxy, Trifluormethyl, Trifluormethoxy, Difluormethoxy oder Chlordifluormethoxy substituiertes Benzyl, Phenethyl, Naphthylmethyl, Tetrahydronaphthylmethyl, Furylmethyl, Thienylmethyl, Pyrrolylmethyl, Oxazolylmethyl, Isoxazolylmethyl, Thiazolylmethyl oder Pyridylmethyl, für -CO-R⁶, -CO-NR⁷R⁸ oder die Gruppierung
- (CH₂)ₚ - (CR⁹R¹⁰)_{q} - (CH₂)ᵣ - G
steht,
Z und D können auch ganz besonders bevorzugt gemeinsam für gegebenenfalls einfach oder zweifach durch Nitro, Fluor, Chlor, Brom, Methyl, Ethyl, n-Propyl, i-Propyl, Methoxy, Ethoxy, n-Propoxy, i-Propoxy, Trifluormethyl, Trifluormethoxy, Difluormethoxy oder Chlordifluormethoxy substituiertes Phenoxymethyl stehen.
Y für eine direkte Bindung, Sauerstoff, Schwefel, Carbonyl, -CH₂-, -(CH₂)₂-, -CH=CH- (E oder Z), -C≡C-, -CH₂O-, -(CH₂)₂O-, -CH(CH₃)O-, -OCH₂-, -O(CH₂)₂-, -SCH₂-, -S(CH₂)₂-, -SCH(CH₃)-, C₁-C₄-Alkylendioxy, insbesondere -OCH₂O- oder -O(CH₂)₂O- oder für gegebenenfalls einfach durch einen Rest aus der Liste W¹ substituiertes p-Phenylen steht,
E für Wasserstoff, Methyl, Ethyl, n-Propyl, Isopropyl, n-Butyl, Isobutyl, sec.-Butyl, tert.-Butyl, die isomeren Pentyle, die isomeren Hexyle, n-Heptyl, n-Octyl, n-Isooctyl, n-Nonyl, n-Decyl, n-Undecyl, n-Dodecyl, n-Tridecyl, n-Tetradecyl, n-Pentadecyl, n-Hexadecyl, 2-Propenyl, Butenyl, Pentenyl, Hexenyl, Propargyl, Butinyl, Pentinyl, -CF₃, -CHF₂, -CClF₂, -CF₂CHFCl, -CF₂CH₂F, -CF₂CHF₂, -CF₂CCl₃, -CH₂CF₃, -CF₂CHFCF₃, -CH₂CF₂CHF₂, -CH₂CF₂CF₃, für jeweils gegebenenfalls einfach bis dreifach durch Fluor, Chlor, Brom, Methyl, Ethyl, n-Propyl, Isopropyl, n-Butyl, Isobutyl, sec.-Butyl, tert.-Butyl, Ethenyl, 1-Propenyl, 2,2-Dimethylethenyl, -CH=CCl₂, Phenyl, Styryl, jeweils durch Fluor, Chlor oder Brom substituiertes Phenyl oder durch 4-Chlorstyryl substituiertes Cyclopropyl, Cyclobutyl, Cyclopentyl oder Cyclohexyl, für jeweils gegebenenfalls durch Fluor, Chlor, Methyl, Ethyl, n-Propyl, Isopropyl, n-Butyl, Isobutyl, sec.-Butyl oder tert.-Butyl substituiertes Cyclopentenyl oder Cyclohexenyl, für gegebenenfalls einfach oder zweifach durch Reste aus der Liste W¹ substituiertes Phenyl, für jeweils gegebenenfalls einfach oder zweifach durch Reste aus der Liste W² substituiertes Furyl, Thienyl, Pyrrolyl, Oxazolyl, Isoxazolyl, Thiazolyl oder Pyridyl, oder für die Gruppierung
- (CH₂)ₚ - (CR⁹R¹⁰)_{q} - (CH₂)ᵣ - G
steht,
R⁶ für Methyl, Ethyl, n-Propyl, Isopropyl, n-Butyl, Isobutyl, sec.-Butyl, tert.-Butyl, Methoxy, Ethoxy, n-Propoxy, Isopropoxy, n-Butoxy, Isobutoxy, sec.-Butoxy, tert.-Butoxy, Cyclopropyl, Cyclohexyl, Cyclohexyloxy, Cyclohexylmethyloxy, Phenyl, 2-Chlorphenyl, 3-Chlorphenyl, 2,6-Difluorphenyl, 2,4-Dichlorphenyl, 3,4-Dichlorphenyl, 2-Trifluormethoxyphenyl oder 4-Trifluormethoxyphenyl steht,
R⁷ für Wasserstoff steht,
R⁸ für Methyl, Ethyl oder gegebenenfalls einfach durch Chlor substituiertes Phenyl steht,
p, q und r unabhängig voneinander für 0, 1, 2 oder 3, wobei deren Summe kleiner 4 ist stehen,
R⁹ und R¹⁰ unabhängig voneinander für Wasserstoff, Methyl, Ethyl, n-Propyl, Isopropyl, n-Butyl, Isobutyl, sec.-Butyl, tert.-Butyl stehen,
G für Cyano, für jeweils gegebenenfalls einfach bis dreifach durch Fluor, Chlor, Brom, Methyl, Ethyl, n-Propyl, Isopropyl oder Trifluormethyl und gegebenenfalls an der Verknüpfungsstelle durch den Rest R¹¹ substituiertes 5,6-Dihydrodioxazin-2-yl, 3-Pyridyl, 3-Furyl, 3-Thienyl, 2-Thiazolyl, 5-Thiazolyl, 2-Dioxolanyl, 1,3-Dioxan-2-yl, 2-Dithiolanyl, 1,3-Dithian-2-yl oder 1,3-Thioxan-2-yl oder eine der folgenden Gruppierungen:
(a) ―CO―R¹¹
(b) ―CO―OR¹²
(c) ―CO―NR¹³R¹⁴
(d) ―CS―NR¹³R¹⁴ 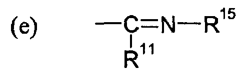 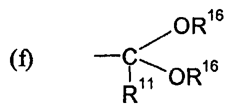 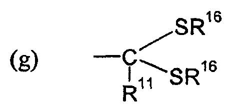 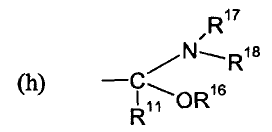 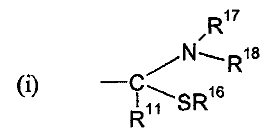
steht,
R¹¹ für Wasserstoff, Methyl, Ethyl, n-Propyl, Isopropyl, n-Butyl, Isobutyl, sec.-Butyl, tert.-Butyl, die isomeren Pentyle, die isomeren Hexyle -CF₃, -CHF₂, -CClF₂, -CF₂CHFCl, -CF₂CH₂F, -CF₂CHF₂, -CF₂CCl₃, -CH₂CF₃, C₃-C₆-Alkenyl, einfach bis dreifach durch Fluor oder Chlor substituiertes C₃-C₆-Alkenyl, für jeweils gegebenenfalls einfach oder zweifach durch Fluor, Chlor, Methyl, Ethyl, n-Propyl, Isopropyl, -CF₃, -CHF₂, -CClF₂, -CF₂CHFCl, -CF₂CH₂F, -CF₂CHF₂, -CF₂CCl₃ oder -CH₂CF₃ substituiertes Cyclopropyl, Cyclopentyl oder Cyclohexyl oder für gegebenenfalls einfach oder zweifach durch Methylcarbonylamino, Ethylcarbonylamino, Methylcarbonyl-methylamino und/oder Reste aus der Liste W³ substituiertes Phenyl steht,
R¹² für Wasserstoff, Methyl, Ethyl, n-Propyl, Isopropyl, n-Butyl, Isobutyl, sec.-Butyl, tert.-Butyl, -CH₂CF₃, Allyl, jeweils gegebenenfalls einfach oder zweifach durch Fluor, Chlor, Methyl, Ethyl, n-Propyl, Isopropyl -CF₃, -CHF₂, -CClF₂, -CF₂CHFCl, -CF₂CH₂F, -CF₂CHF₂, -CF₂CCl₃ oder -CH₂CF₃ substituiertes Cyclopropyl, Cyclopentyl, Cyclohexyl, Cyclopropylmethyl, Cyclopentylmethyl, Cyclohexylmethyl, Cyclopropylethyl, Cyclopentylethyl oder Cyclohexylethyl oder für jeweils gegebenenfalls einfach oder zweifach durch Reste aus der Liste W³ substituiertes Benzyl oder Phenethyl steht,
R¹³ und R¹⁴ unabhängig voneinander für Wasserstoff, Methyl, Ethyl, n-Propyl, Isopropyl, n-Butyl, Isobutyl, sec.-Butyl, tert.-Butyl, -CH₂CF₃, Methoxy, Ethoxy, Allyl, für jeweils gegebenenfalls einfach oder zweifach durch Fluor, Chlor, Methyl, Ethyl, n-Propyl, Isopropyl oder Trifluormethyl substituiertes Cyclopropyl, Cyclopentyl, Cyclohexyl, Cyclopropylmethyl, Cyclopentylmethyl oder Cyclohexylmethyl, für jeweils gegebenenfalls einfach oder zweifach durch Reste aus der Liste W³ substituiertes Phenyl, Benzyl oder Phenethyl, für -OR¹² oder -NR¹¹R¹² stehen,
R¹⁵ für -OR¹², -NR¹¹R¹² oder -N(R¹¹)-COOR¹² steht,
R¹⁶, R¹⁷ und R¹⁸ unabhängig voneinander für Methyl, Ethyl, n-Propyl oder Isopropyl stehen,
W¹ für Wasserstoff, Fluor, Chlor, Brom, Cyano, Formyl, Nitro, Methyl, Ethyl, n-Propyl, Isopropyl, n-Butyl, Isobutyl, sec.-Butyl, tert.-Butyl, Methoxy, Ethoxy, n-Propoxy, Isopropoxy, n-Butoxy, Isobutoxy, sec.-Butoxy, tert.-Butoxy, -CF₃, -CHF₂, -CClF₂, -CF₂CHFCl, -CF₂CH₂F, -CF₂CHF₂, -CF₂CCl₃, -CH₂CF₃, -CF₂CHFCF₃, -CH₂CF₂CHF₂, -CH₂CF₂CF₃, Trifluormethoxy, Difluormethoxy, Chlordifluormethoxy, Acetyl, Propionyl, Butyryl, Isobutyryl, Methoxycarbonyl, Ethoxycarbonyl, n-Propoxycarbonyl, Isopropoxycarbonyl, n-Butoxycarbonyl, Isobutoxycarbonyl, sec.-Butoxycarbonyl, tert.-Butoxycarbonyl oder -S(O)ₒR³ steht,
W² für Fluor, Chlor, Brom, Cyano, Methyl, Ethyl, n-Propyl, Isopropyl, Trifluormethyl, Trifluormethoxy, Difluormethoxy, Chlordifluormethoxy, Acetyl, Trifluormethylthio, -CH=N-OCH₃, -CH=N-OC₂H₅, - CH=N-OC₃H₇, -C(CH₃)=N-OCH₃, -C(CH₃)=N-OC₂H₅, - C(CH₃)=N-OC₃H₇, -C(C₂H₅)=N-OCH₃, -C(C₂H₅)=N-OC₂H₅ oder - C(C₂H₅)=N-OC₃H₇ steht,
W³ für Fluor, Chlor, Cyano, Nitro, Methyl, Ethyl, Methoxy, Ethoxy, Methylthio, Trifluormethyl, Trifluormethoxy, Trifluormethylthio, Dimethylamino, Diethylamino, -COOR¹⁹ oder -CONR²⁰R²¹ steht,
R¹⁹ für Wasserstoff, Methyl, Ethyl, n-Propyl, Isopropyl, tert.-Butyl, -CH₂CF₃, für jeweils gegebenenfalls einfach oder zweifach durch Fluor, Chlor, Methyl, Ethyl, n-Propyl, Isopropyl oder -CF₃ substituiertes Cyclopropyl, Cyclopentyl oder Cyclohexyl oder für gegebenenfalls einfach oder zweifach durch Reste aus der Liste W⁴ substituiertes Phenyl steht,
R²⁰ und R²¹ stehen unabhängig voneinander für Wasserstoff, Methyl, Ethyl, n-Propyl, Isopropyl, n-Butyl, Isobutyl, sec.-Butyl, tert.-Butyl, -CH₂CF₃, Methoxy, Ethoxy, Allyl, für jeweils gegebenenfalls einfach oder zweifach durch Fluor oder Chlor substituiertes Cyclopropyl, Cyclopentyl, Cyclohexyl, Cyclopropylmethyl, Cyclopentylmethyl oder Cyclohexylmethyl, für jeweils gegebenenfalls einfach oder zweifach durch Reste aus der Liste W⁴ substituiertes Phenyl, Benzyl oder Phenethyl, für -OR¹⁶ oder -NR¹⁷R¹⁸.
W⁴ für Fluor, Chlor, Brom, Cyano, Nitro, Methyl, Ethyl, tert.-Butyl, Methoxy, Ethoxy, Methylthio, Trifluormethyl, Trifluormethoxy oder Trifluormethylthio steht.

4. Verfahren zur Herstellung von Verbindungen der Formel (I) gemäß Anspruch 1, **dadurch gekennzeichnet, daß** man
A) Verbindungen der Formel (I) 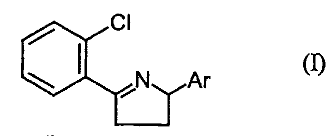 in welcher
Ar die in Anspruch 1 angegebenen Bedeutungen hat,
erhält, indem man
a) Aminoketonderivate der Formel (VIII) 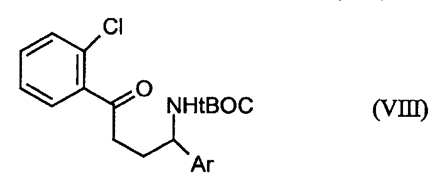 in welcher
Ar die oben angegebenen Bedeutungen hat,
mit einer Säure umsetzt und anschließend gegebenenfalls in Gegenwart eines Säurebindemittels cyclokondensiert, oder
b) die Nitrogruppe von Nitroketonen der Formel (XVIII), 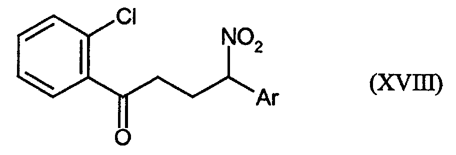 in welcher
Ar die in Anspruch 1 genannten Bedeutungen hat, reduziert, wobei 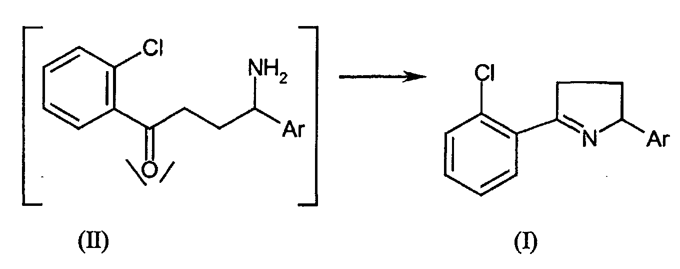 intermediär ein Aminoketon der Formel (II) auftritt, welches jedoch, insbesondere in saurem Medium, in situ zu (I) cyclokondensiert wird, oder
c) Imine der Formel (XXVII) 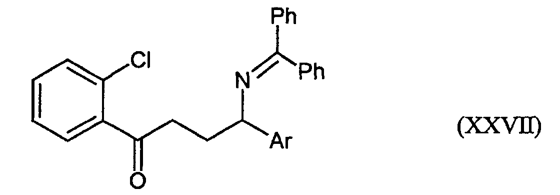
in welcher Ar die in Anspruch 1 genannten Bedeutungen hat, mit wässrigen Säuren hydrolysiert 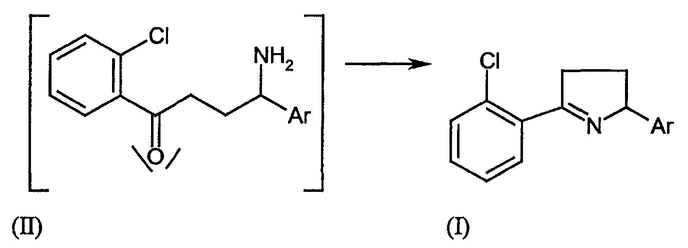 wobei
intermediär ein Aminoketon der Formel (II) auftritt, welches jedoch in situ zu (I) cyclokondensiert wird,
B) Verbindungen der Formel (III) 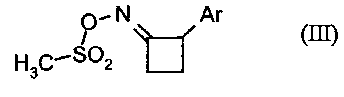 in welcher
Ar die in Anspruch 1 angegebenen Bedeutungen hat,
mit Aryl-Grignard-Verbindungen der Formel (IV) 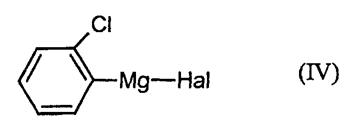 in welcher
Hal für Brom oder Iod steht,
in Gegenwart eines Verdünnungsmittels umsetzt,
C) Verbindungen der Formel (I-b) 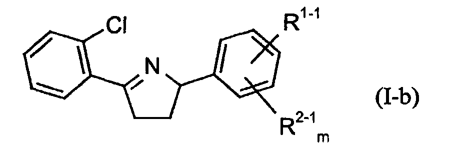 in welcher
m die in Anspruch 1 angegebenen Bedeutungen hat,
R¹⁻¹ für A oder eine der folgenden Gruppierungen
(m) ―B―Z-D 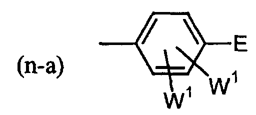
steht, wobei
A, B, D, E, W¹ und Z die in Anspruch 1 angegebenen Bedeutungen haben und
R²⁻¹ für Wasserstoff, Fluor, Cyano, Nitro, Alkyl, Alkoxy, Halogenalkyl, Halogenalkoxy, Alkoxyalkoxy oder -SR³ steht, wobei
R³ die in Anspruch 1 angegebenen Bedeutungen hat,
erhält, indem man Verbindungen der Formel (V) 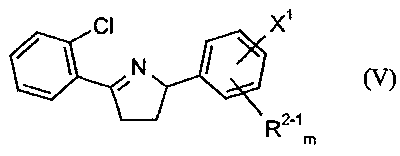
in welcher
m die in Anspruch 1 angegebenen Bedeutungen haben,
R²⁻¹ die oben angegebenen Bedeutungen hat und
X¹ für Brom, Iod oder -OSO₂CF₃ steht
mit Boronsäuren der Formel (VI)
R¹⁻¹B(OH)₂ (VI),
in welcher
R¹⁻¹ die oben angegebenen Bedeutungen hat,
in Gegenwart eines Katalysators und in Gegenwart eines Säurebindemittels und in Gegenwart eines Lösungsmittels kuppelt,
D) Verbindungen der Formel (I-c) 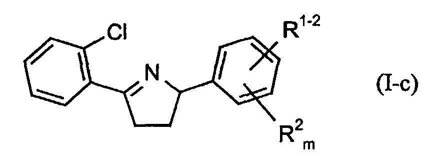 in welcher
R² und m die in Anspruch 1 angegebenen Bedeutungen haben,
R¹⁻² für eine der folgenden Gruppierungen
(m-b) -B - Z - D¹
(n-b) -Y¹ - E¹
steht, in denen
B und Z die in Anspruch 1 angegebenen Bedeutungen haben,
Y¹ für Sauerstoff oder Schwefel steht und
D¹ und E¹ für die Gruppierung
-(CH₂)ₚ-(CR⁹R¹⁰)_{q}-(CH₂)ᵣ - G
stehen, in der
R⁹, R¹⁰, G, p, q und r die oben angegebenen Bedeutungen haben,
erhält, indem man cyclische Imine der Formel (I-d) 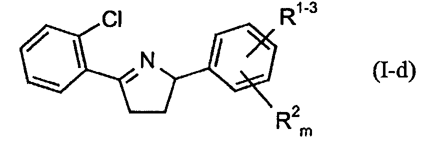 in welcher
R² und m die in Anspruch 1 angegebenen Bedeutungen haben und
R¹⁻³ für eine der folgenden Gruppierungen
(m-c) -B - Z - H
(n-c) -Y¹ - H
steht, in denen
B und Z die in Anspruch 1 angegebenen Bedeutungen haben,
Y¹ die oben angegebenen Bedeutungen hat,
mit Verbindungen der Formel (VII)
Ab - (CH₂)ₚ - (CR⁹R¹⁰)_{q} - (CH₂)ᵣ - G (VII),
in welcher
R⁹, R¹⁰, G, p, q und r die in Anspruch 1 angegebenen Bedeutungen haben und
Ab für eine Abgangsgruppe steht,
kondensiert,
E) Verbindungen der Formel (I-e) 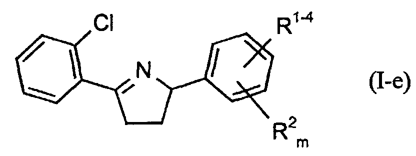 in welcher
R² und m die in Anspruch 1 angegebenen Bedeutungen haben und
R¹⁻⁴ für eine den Rest G enthaltenden Gruppierung aus der Beschreibung der erfindungsgemäßen Verbindungen der Formel (I) steht, wobei
G für eine der oben genannten Gruppierungen (e) bis (k) steht,
durch allgemein übliche und bekannte Derivatisierungen der entsprechenden Keto-Derivate, Carbonsäure-Derivate oder Nitrile, d.h. Verbindungen der Formel (I), in denen G für Cyano oder eine der Gruppierungen (a) bis (d) steht, erhält,
F) Verbindungen der Formel (I-f) 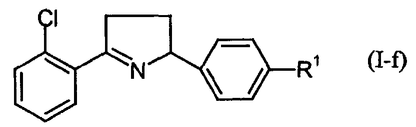 in welcher R¹ die in Anspruch 1 angegebenen Bedeutungen hat, erhält man auch, indem man im ersten Schritt
α) O-Chloracetophenon der Formel (F-I) 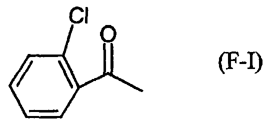 mit Dimethylmethylenammoniumchlorid der Formel (F-II) 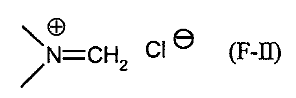 zur Verbindung der Formel (F-III) 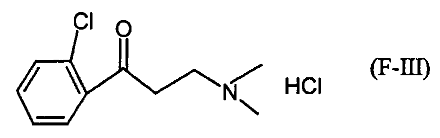 umsetzt und diese in einem zweiten Schritt
β) mit Benzylcyaniden der Formel (F-IV) 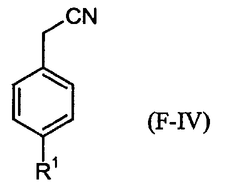 in denen R¹ die in Anspruch 1 angegebenen Bedeutung hat, zu Verbindungen der Formel (F-V) 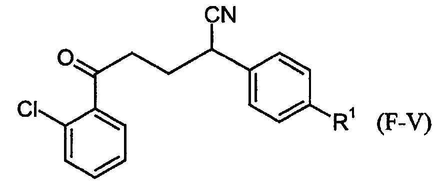 umsetzt, welche im nächsten Schritt
δ) mit Natronlauge/H₂O₂ zu Verbindungen der Formel (F-VI) 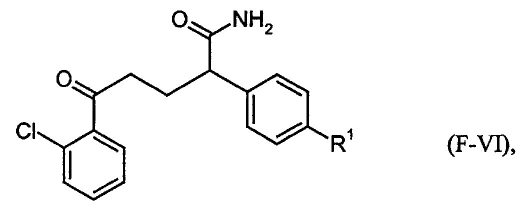 in welcher R¹ die in Anspruch 1 angegebenen Bedeutungen besitzt, derivatisiert, und diese in einem abschließenden Schritt
γ) durch Umsetzung mit PIFA (1,1-Bis(Trifluoroacetoxy)iodbenzol) der Formel (F-VII) 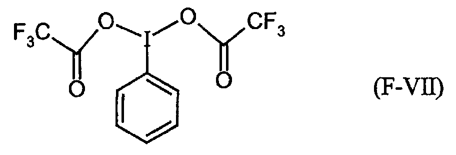 zu cyclischen Iminen der Formel (I-f) cyclisiert,
G) Verbindungen der Formel (I-f) 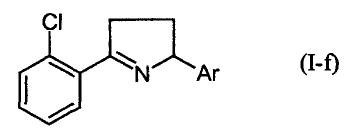 in welcher Ar die in Anspruch 1 angegebenen Bedeutungen hat, erhält man auch, indem man im ersten Schritt
α) Arylbutyrolactame der Formel (XI) 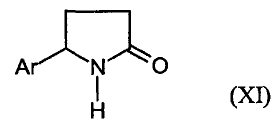 mit o-Chlorbenzoesäurechlorid 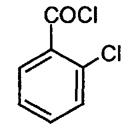 zu Verbindungen der Formel G(I) 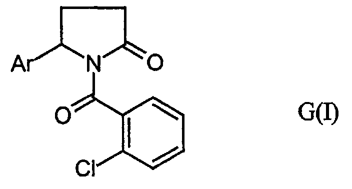 umsetzt, und diese in einem zweiten Schritt
β) mit o-Chlorbenzoesäuremethylester 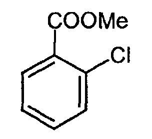 zu Verbindungen der Formel G(II) 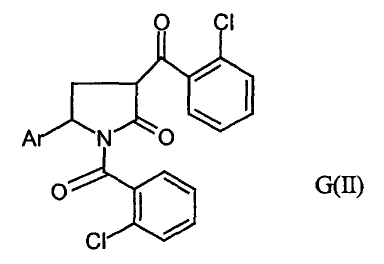 umsetzt, welche in einem abschließenden Schritt
δ) mit HBr/Eisessig zu Verbindungen der Formel (I-f) 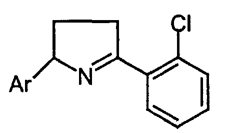 umgesetzt werden.

5. Verbindungen der Formel (VIII) 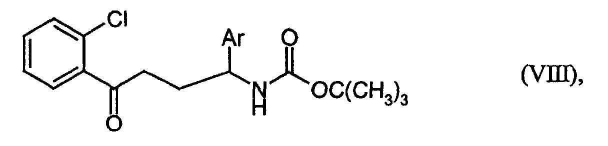 in welcher
Ar die in Anspruch 1 angegebenen Bedeutungen hat.

6. Verbindungen der Formel (XVIII) 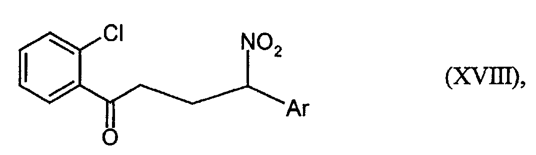 in welcher
Ar die in Anspruch 1 angegebenen Bedeutungen hat.

7. Schädlingsbekämpfungsmittel, **gekennzeichnet durch** einen Gehalt an mindestens einer Verbindung der Formel (I) gemäß Anspruch 1.

8. Verwendung von Verbindungen der Formel (I) gemäß Anspruch 1 zur Bekämpfung von Schädlingen mit Ausnahme der therapeutische Behandlung von Menschen und Tieren.

9. Verfahren zur Bekämpfung von Schädlingen mit Ausnahme der therapeutische Behandlung von Menschen und Tieren, **dadurch gekennzeichnet, daß** man Verbindungen der Formel (I) gemäß Anspruch 1 auf Schädlinge und/oder ihren Lebensraum einwirken läßt.

10. Verfahren zur Herstellung von Schädlingsbekämpfungsmitteln, **dadurch gekennzeichnet, daß** man Verbindungen der Formel (I) gemäß Anspruch 1 mit Streckmitteln und/oder oberflächenaktiven Mitteln vermischt.

11. Verwendung von Verbindungen der Formel (I) gemäß Anspruch 1 zur Herstellung von Schädlingsbekämpfungsmitteln.

12. Verwendung von Verbindungen der Formel (I) gemäß Anspruch 1 zur Herstellung eines Mittels zur Bekämpfung von Arthropoden in der Tierhaltung.

## Claims

1. Compounds of the formula (I) 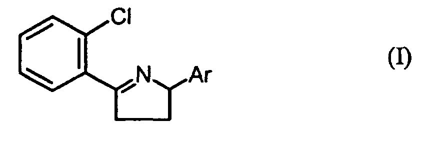 in which
Ar represents the radical 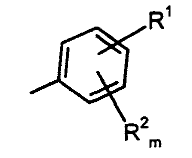
where
m represents 0, 1, 2, 3,
R¹ represents a substituent in the meta or para position from the group consisting of hydrogen, halogen, cyano, tri-(C₁-C₆-alkyl)-silyl, -CO-NR⁴R⁵, tetrahydropyranyl or one of the following groupings
(l) -X - A
(m) -B - Z - D
(n) -Y ― E,
R² represents hydrogen, halogen, cyano, nitro, C₁-C₁₆-alkyl, C₁-C₁₆-alkoxy, C₁-C₆-halogenoalkyl, C₁-C₆-halogenoalkoxy, C₁-C₈-alkoxy-C₁-C₈-alkoxy or -S(O)ₒR³,
o represents 0, 1 or 2,
R³ represents optionally fluorine- or chlorine-substituted C₁-C₆-alkyl,
R⁴ and R⁵ independently of one another each represent hydrogen, C₁-C₆-alkyl, C₁-C₆-halogenoalkyl or represent phenyl or phenyl-C₁-C₄-alkyl, each of which is optionally mono- to trisubstituted by radicals from the list W¹,
X represents a direct bond, oxygen, sulphur, carbonyl, carbonyloxy, oxycarbonyl, C₁-C₄-alkylene, C₂-C₄-alkenylene, C₂-C₄-alkinylene, C₁-C₄-alkyleneoxy, C₁-C₄-oxyalkylene, C₁-C₄-thioalkylene, C₁-C₄-alkylenedioxy or di-C₁-C₄-alkylsilylene,
A represents phenyl, naphthyl or tetrahydronaphthyl, each of which is optionally mono- to tetrasubstituted by radicals from the list W¹, or represents 5- to 10-membered heterocyclyl containing 1 or 2 aromatic rings and having 1 to 4 heteroatoms, which contains 0 to 4 nitrogen atoms, 0 to 2 oxygen atoms and 0 to 2 sulphur atoms (in particular furyl, benzofuryl, thienyl, benzothienyl, oxazolyl, benzoxazolyl, thiazolyl, benzothiazolyl, pyrrolyl, pyridyl, pyrimidyl, 1,3,5-triazinyl, quinolinyl, isoquinolinyl, indolyl, purinyl, benzodioxolyl, indanyl, benzodioxanyl or chromanyl), and is in each case optionally mono- to tetrasubstituted by radicals from the list W²,
B represents p-phenylene which is optionally mono- or disubstituted by radicals from the list W¹,
Z represents oxygen or sulphur,
D represents hydrogen, C₁-C₁₆-alkyl, C₂-C₁₆-alkenyl, C₂-C₆-alkinyl, C₁-C₁₆-halogenoalkyl, C₂-C₁₆-halogenoalkenyl, in each case optionally halogen-, C₁-C₄-alkyl-, C₂-C₄-alkenyl-, C₂-C₄-halogenoalkenyl-, phenyl-, styryl-, halogenophenyl- or halogenostyryl-substituted C₃-C₈-cycloalkyl or C₃-C₈-cycloalkyl-C₁-C₆-alkyl, represents in each case optionally halogen- or C₁-C₄-alkyl-substituted C₅-C₈-cycloalkenyl or C₅-C₈-cycloalkenyl-C₁-C₄-alkyl, represents in each case optionally nitro-, halogen-, C₁-C₆-alkyl-, C₁-C₆-alkoxy-, C₁-C₆-halogenoalkyl- or C₁-C₆-halogenoalkoxy-substituted phenyl-C₁-C₆-alkyl, naphthyl-C₁-C₆-alkyl, tetrahydronaphthyl-C₁-C₆-alkyl or hetaryl-C₁-C₆-alkyl having 5 or 6 ring members and 1 or 2 heteroatoms from the group consisting of nitrogen, oxygen and sulphur (in particular furyl methyl, thienylmethyl, pyrrolylmethyl, oxazolylmethyl, isoxazolylmethyl, thiazolylmethyl or pyridylmethyl), represents -CO-R⁶, -CO-NR⁷R⁸ or represents the grouping
-(CH₂)ₚ - (CR⁹R¹⁰)_{q} - (CH₂)ᵣ - G,
Z and D together may also represent in each case optionally nitro-, halogen-, C₁-C₆-alkyl-, C₁-C₆-alkoxy-, C₁-C₆-halogenoalkyl- or C₁-C₆-halogenoalkoxy-substituted phenoxy-C₁-C₄-alkyl,
Y represents a direct bond, oxygen, sulphur, carbonyl, carbonyloxy, oxycarbonyl, C₁-C₄-alkylene, C₂-C₄-alkenylene, C₂-C₄-alkinylene, C₁-C₄-alkyleneoxy, C₁-C₄-oxyalkylene, C₁-C₄-thioalkylene, C₁-C₄-alkylenedioxy or represents p-phenylene which is optionally mono- or disubstituted by radicals from the list W¹,
E represents hydrogen, C₁-C₁₆-alkyl, C₂-C₁₆-alkenyl, C₂-C₆-alkinyl, C ₁-C₁₆-halogenoalkyl, C₂-C₁₆-halogenoalkenyl, optionally halogen-, C₁-C₄-alkyl-, C₂-C₄-alkenyl-, C₂-C₄-halogenoalkenyl, phenyl-, styryl-, halogenophenyl- or halogenostyryl-substituted C₃-C₈-cycloalkyl, represents optionally halogen- or C₁-C₄-alkyl-substituted C₅-C₈-cycloalkenyl, represents phenyl which is optionally mono- to tetrasubstituted by radicals from the list W¹ or represents 5- or 6-membered hetaryl having 1 or 2 heteroatoms from the group consisting of nitrogen, oxygen and sulphur (in particular furyl, thienyl, pyrrolyl, oxazolyl, isoxazolyl, thiazolyl or pyridyl) which is in each case optionally mono- to tetrasubstituted by radicals from the list W² or represents the grouping
-(CH₂)ₚ - (CR⁹R¹⁰)_{q} - (CH₂)ᵣ - G,
R⁶ represents C₁-C₁₂-alkyl, C₁-C₁₂-alkoxy, C₂-C₁₂-alkenyl, C₂-C₁₂-alkenyloxy, in each case optionally halogen-, C₁-C₄-alkyl-, C₂-C₄-alkenyl-, C₁-C₄-halogenoalkyl- or C₂-C₄-halogenoalkenyl-substituted C₃-C₈-cycloalkyl, C₃-C₈-cycloalkyloxy or C₃-C₈-cycloalkyl-C₁-C₆-alkyloxy or represents phenyl or naphthyl which is in each case optionally mono- to tetrasubstituted by nitro, halogen, C₁-C₁₂-alkyl, C₁-C₁₂-alkoxy, C₁-C₁₂-halogenoalkyl or C₁-C₁₂-halogenoalkoxy,
R⁷ represents hydrogen or C₁-C₁₂-alkyl,
R⁸ represents C₁-C₁₂-alkyl, C₁-C₁₂-halogenoalkyl, in each case optionally halogen-, C₁-C₄-alkyl-, C₂-C₄-alkenyl-, C₁-C₄-halogenoalkyl- or C₂-C₄-halogenoalkenyl-substituted C₃-C₈-cycloalkyl or C₃-C₈-cycloalkyl-C₁-C₆-alkyl or represents phenyl or phenyl-C₁-C₆-alkyl, each of which is optionally mono- to tetrasubstituted by halogen, C₁-C₁₂-alkyl, C₁-C₁₂-alkoxy, C₁-C₁₂-halogenoalkyl or C₁-C₁₂-halogenoalkoxy,
p, q and r independently of one another each represent 0, 1, 2 or 3, their sum being smaller than 6,
R⁹ and R¹⁰ independently of one another each represent hydrogen or C₁-C₄-alkyl,
G represents cyano, represents a 5- or 6-membered heterocycle having 1 to 3 identical or different heteroatoms from the group consisting of nitrogen, oxygen and sulphur (in particular 5,6-dihydrodioxazin-2-yl, 3-pyridyl, 3-furyl, 3-thienyl, 2-thiazolyl, 5-thiazolyl, 2-dioxolanyl, 1,3-dioxan-2-yl, 2-dithiolanyl, 1,3-dithian-2-yl or 1,3-thioxan-2-yl) and being optionally mono- to trisubstituted by halogen, C₁-C₄-alkyl or C₁-C₄-halogenoalkyl and optionally, at the point of linkage, by the radical R¹¹, or represents one of the following groupings:
(a) ―CO―R¹¹
(b) ―CO―OR¹²
(c) ―CO―NR¹³R¹⁴
(d) ―CS―NR¹³R¹⁴ 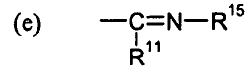 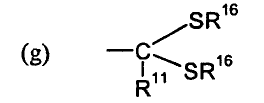 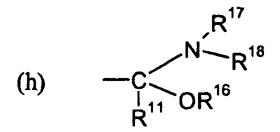 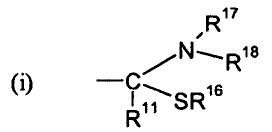 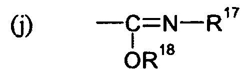 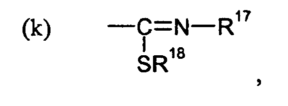
R¹¹ represents hydrogen, C₁-C₆-alkyl, C₂-C₆-alkenyl, C₁-C₄-halogenoalkyl, C₂-C₆-halogenoalkenyl, optionally halogen-, C₁-C₄-alkyl- or C₁-C₄-halogenoalkyl-substituted C₃-C₆-cycloalkyl or represents phenyl which is optionally mono- to pentasubstituted by C₁-C₄-alkylcarbonylamino, C₁-C₄-alkylcarbonyl-C₁-C₄-alkylamino and/or radicals from the list W³,
R¹² represents hydrogen, C₁-C₄-alkyl, C₂-C₆-alkenyl, C₁-C₄-halogenoalkyl, C₂-C₆-halogenoalkenyl, in each case optionally halogen-, C₁-C₄-alkyl- or C₁-C₄-halogenoalkyl-substituted C₃-C₆-cycloalkyl or C₃-C₆-cycloalkyl-C₁-C₄-alkyl or represents C₆-C₁₀-aryl-C₁-C₄-alkyl (in particular phenyl-C₁-C₄-alkyl or naphthyl-C₁-C₄-alkyl) which is optionally mono- to tetrasubstituted by radicals from the list W³,
R¹³ and R¹⁴ independently of one another each represent hydrogen, C₁-C₄-alkyl, C₃-C₆-alkenyl, C₁-C₄-halogenoalkyl, C₃-C₆-halogenoalkenyl, C₁-C₄-alkoxy, in each case optionally halogen-, C₁-C₄-alkyl- or C₁-C₄-halogenoalkyl-substituted C₃-C₆-cycloalkyl or C₃-C₆-cycloalkyl-C₁-C₄-alkyl, represent phenyl or phenyl-C₁-C₄-alkyl, each of which is optionally mono- to pentasubstituted by radicals from the list W³, represent -OR¹² or -NR¹¹R¹² or together represent an alkylene chain having 4 to 6 members in which optionally one methylene group is replaced by oxygen,
R¹⁵ represents -OR¹², -NR¹¹R¹² or -N(R¹¹)-COOR¹²,
R¹⁶, R¹⁷ and R¹⁸ independently of one another each represent C₁-C₆-alkyl,
W¹ represents hydrogen, halogen, cyano, formyl, nitro, C₁-C₆-alkyl, tri-C₁-C₄-alkylsilyl, C₁-C₁₆-alkoxy, C₁-C₆-halogenoalkyl, C₁-C₆-halogenoalkoxy, C₂-C₆-halogenoalkenyloxy, C₁-C₆-alkylcarbonyl, C₁-C₁₆-alkoxycarbonyl, pentafluorothio or -S(O)ₒR³,
W² represents halogen, cyano, formyl, nitro, C₁-C₆-alkyl, tri-C₁-C₄-alkylsilyl, C₁-C₁₆-alkoxy, C₁-C₆-halogenoalkyl, C₁-C₆-halogenoalkoxy, C₁-C₆-alkylcarbonyl, C₁-C₁₆-alkoxycarbonyl, pentafluorothio, -S(O)ₒR³ or -C(R¹¹)=N-R¹⁵,
W³ represents halogen, cyano, nitro, C₁-C₄-alkyl, C₁-C₄-alkoxy, C₁-C₄-halogenoalkyl, C₁-C₄-halogenoalkoxy, di-C₁-C₄-alkylamino, -S(O)ₒR³, -COOR¹⁹ or -CONR²⁰R²¹,
R¹⁹ represents hydrogen, C₁-C₄-alkyl, C₁-C₄-halogenoalkyl, optionally halogen-, C₁-C₄-alkyl- or C₁-C₄-halogenoalkyl-substituted C₃-C₇-cycloalkyl or represents phenyl which is optionally mono- to pentasubstituted by radicals from the list W⁴,
R²⁰ and R²¹ independently of one another each represent hydrogen, C₁-C₄-alkyl, C₃-C₆-alkenyl, C₁-C₄-halogenoalkyl, C₃-C₆-halogenoalkenyl, C₁-C₄-alkoxy, in each case optionally halogen-, C₁-C₄-alkyl- or C₁-C₄-halogenoalkyl-substituted C₃-C₆-cycloalkyl or C₃-C₆-cycloalkyl-C₁-C₄-alkyl or represent phenyl or phenyl-C₁-C₄-alkyl, each of which is optionally mono- to pentasubstituted by radicals from the list W⁴, represent -OR¹⁶ or - NR¹⁷R¹⁸ or together represent an alkylene chain having 4 to 6 members in which optionally one methylene group is replaced by oxygen, and
W⁴ represents halogen, cyano, nitro, C₁-C₆-alkyl, C₁-C₆-alkoxy, C₁-C₆-halogenoalkyl, C₁-C₆-halogenoalkoxy, di-C₁-C₄-alkylamino, C₁-C₆-alkoxycarbonyl, di-C₁-C₆-alkylaminocarbonyl or -S(O)ₒR³.

2. Compounds of the formula (I) according to Claim 1, in which
Ar represents the radical 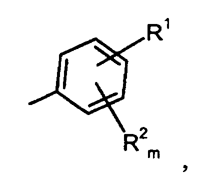
m represents 0, 1 or 2,
R¹ represents a substituent in the meta or para position from the group consisting of hydrogen, fluorine, chlorine, bromine, iodine, cyano, tri-(C₁-C₄-alkyl)-silyl, -CO-NR⁴R⁵, tetrahydropyranyl or one of the following groupings
(l)- X - A
(m)- B - Z - D
(n)- Y - E,
R² represents hydrogen, fluorine, chlorine, bromine, iodine, cyano, nitro, C₁-C₁₆-alkyl, C₁-C₁₆-alkoxy, in each case fluorine- or chlorine-substituted C₁-C₆-alkyl or C₁-C₆-alkoxy, represents C₁-C₈-alkoxy-C₁-C₈-alkoxy or -S(O)ₒR³,
o represents 0, 1 or 2,
R³ represents C₁-C₄-alkyl or in each case fluorine- or chlorine-substituted methyl or ethyl,
R⁴ and R⁵ independently of one another each represent hydrogen, C₁-C₆-alkyl, fluorine- or chlorine-substituted C₁-C₆-alkyl or represent phenyl or benzyl, each of which is optionally mono- or disubstituted by radicals from the list W¹,
X represents a direct bond, oxygen, sulphur, carbonyl, carbonyloxy, oxycarbonyl, C₁-C₄-alkylene C₂-C₄-alkenylene C₂-C₄-alkinylene C₁-₄-alkylenoxy, C₁-C₄-oxyalkylene, C₁-C₄-thioalkylene, C₁-C₄-alkylenedioxy or di-C₁-C₄-alkylsilylene,
A represents phenyl, naphthyl or tetrahydronaphthyl, each of which is optionally mono- to trisubstituted by radicals from the list W¹, or represents 5- to 10-membered heterocyclyl containing one or two aromatic rings and having 1 to 4 heteroatoms which contains 0 to 4 nitrogen atoms, 0 to 2 oxygen atoms and 0 to 2 sulphur atoms (in particular furyl, benzofuryl, thienyl, benzothienyl, oxazolyl, benzoxazolyl, thiazolyl, benzothiazolyl, pyrrolyl, pyridyl, pyrimidyl, 1,3,5-triazinyl, quinolinyl, isoquinolinyl, indolyl, purinyl, benzodioxolyl, indanyl, benzodioxanyl or chromanyl) and is in each case optionally mono- to trisubstituted by radicals from the list W²,
B represents p-phenylene which is optionally mono- or disubstituted by radicals from the list W¹,
Z represents oxygen or sulphur,
D represents hydrogen, C₁-C₁₆-alkyl, C₂-C₁₆-alkenyl, C₂-C₆-alkinyl, in each case fluorine- or chlorine-substituted C₁-C₄-alkyl or C₂-C₄-alkenyl, represents C₃-C₆-cycloalkyl or C₃-C₆-cycloalkyl-C₁-C₄-alkyl, each of which is optionally substituted by fluorine, chlorine, bromine, C₁-C₄-alkyl, C₂-C₄-alkenyl, by fluorine- or chlorine-substituted C₂-C₄-alkenyl, by phenyl, styryl, in each case fluorine-, chlorine- or bromine-substituted phenyl or styryl, represents in each case optionally fluorine-, chlorine-, bromine- or C₁-C₄-alkyl-substituted C₅-C₆-cycloalkenyl or C₅-C₆-cycloalkenyl-C₁-C₄-alkyl, represents phenyl-C₁-C₄-alkyl, napthyl-C₁-C₄-alkyl, tetrahydronaphthyl-C₁-C₆-alkyl or hetaryl-C₁-C₄-alkyl having 5 or 6 ring members and one or two heteroatoms from the group consisting of nitrogen, oxygen and sulphur (in particular furylmethyl, thienylmethyl, pyrrolylmethyl, oxazolylmethyl, isoxazolylmethyl, thioazolylmethyl or pyridylmethyl), each of which is optionally substituted by nitro, fluorine, chlorine, bromine, C₁-C₆-alkyl, C₁-C₆-alkoxy, in each case fluorine- or chlorine-substituted C₁-C₄-alkyl or C₁-C₄-alkoxy, represents -CO-R⁶, -CO-NR⁷R⁸ or the grouping
- (CH₂)ₚ - (CR⁹R¹⁰)_{q} - (CH₂)ᵣ - G,
Z and D together may also represent substituted phenoxy-C₁-C₃-alkyl which is optionally substituted by nitro, fluorine, chlorine, bromine, C₁-C₄-alkyl, C₁-C₄-alkoxy, in each case fluorine-, or chlorine-substituted C₁-C₄-alkyl or C₁-C₄-alkoxy,
Y represents a direct bond, oxygen, sulphur, carbonyl, carbonyloxy, oxycarbonyl, C₁-C₄-alkylene, C₂-C₄-alkenylene, C₂-C₄-alkinylene, C₁-C₄-alkyleneoxy, C₁-C₄-oxyalkylene, C₁-C₄-thioalkylene, C₁-C₄-alkylenedioxy or represents p-phenylene which is optionally mono- or disubstituted by radicals from the list W¹,
E represents hydrogen, C₁-C₁₆-alkyl, C₂-C₁₆-alkenyl, C₂-C₆-alkinyl, in each case fluorine- or chlorine-substituted C₁-C₄-alkyl or C₂-C₄-alkenyl, represents C₃-C₆-cycloalkyl which is optionally substituted by fluorine, chlorine, bromine, C₁-C₄-alkyl, C₂-C₄-alkenyl, by fluorine- or chlorine-substituted C₂-C₄-alkenyl, by phenyl, styryl or in each case fluorine-, chlorine- or bromine- substituted phenyl or styryl, represents optionally fluorine-, chlorine-, bromine- or C₁-C₄-alkyl-substituted C₅-C₆-cycloalkenyl, represents phenyl which is optionally mono- to trisubstituted by radicals from the list W¹ or represents 5- or 6-membered hetaryl having 1 or 2 heteroatoms from the group consisting of nitrogen, oxygen and sulphur (in particular furyl, thienyl, pyrrolyl, oxazolyl, isoxazolyl, thiazolyl or pyridyl), each of which is optionally mono- or disubstituted by radicals from the list W², or represents the grouping
- (CH₂)ₚ - (CR⁹R¹⁰)_{q} - (CH₂)ᵣ - G,
R⁶ represents C₁-C₆-alkyl, C₁-C₆-alkoxy, C₂-C₆-alkenyl, C₂-C₆-alkenyloxy, represents C₃-C₆-cycloalkyl, C₃-C₆-cycloalkyloxy or C₃-C₆-cycloalkyl-C₁-C₂-alkyloxy, each of which is optionally substituted by fluorine, chlorine, C₁-C₃-alkyl or in each case fluorine- or chlorine-substituted C₁-C₂-alkyl or C₂-C₃-alkenyl, or represents phenyl which is optionally mono- or disubstituted by fluorine, chlorine, bromine, iodine, C₁-C₄-alkyl, C₁-C₄-alkoxy or by in each case fluorine- or chlorine-substituted C₁-C₃-alkyl or C₁-C₄-alkoxy,
R⁷ represents hydrogen or C₁-C₄-alkyl,
R⁸ represents C₁-C₄-alkyl or represents phenyl or benzyl, each of which is optionally mono- or disubstituted by fluorine, chlorine, bromine, C₁-C₄-alkyl or by in each case fluorine-, or chlorine- substituted C₁-C₄-alkyl or C₁-C₄-alkoxy,
p, q and r independently of one another each represent 0, 1, 2 or 3, their sum being smaller than 6,
R⁹ and R¹⁰ independently of one another each represent hydrogen or C₁-C₄-alkyl,
G represents cyano, represents a 5- or 6-membered heterocycle having 1 to 3 identical or different heteroatoms from the group consisting of nitrogen, oxygen and sulphur (in particular 5,6-dihydrodioxazin-2-yl, 3-pyridyl, 3-furyl, 3-thienyl, 2-thiazolyl, 5-thiazolyl, 2-dioxolanyl, 1,3-dioxan-2-yl, 2-dithiolanyl, 1,3-dithian-2-yl or 1,3-thioxan-2-yl) and being optionally mono- to trisubstituted by fluorine, chlorine, bromine, C₁-C₄-alkyl or by fluorine- or chlorine-substituted C₁-C₄-alkyl and optionally, at the point of linkage, by the radical R¹¹, or represents one of the following groupings:
(a) ―CO―R¹¹
(b) ―CO―OR¹²
(c) ―CO―NR¹³R¹⁴
(d) ―CS―NR¹³R¹⁴ 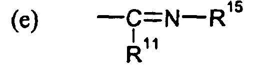 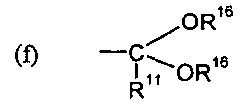 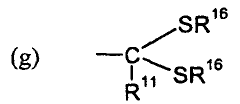 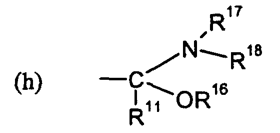 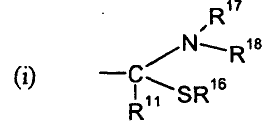 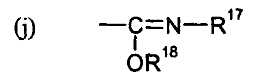 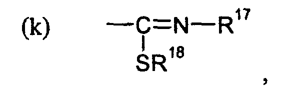
R¹¹ represents hydrogen, C₁-C₆-alkyl, C₂-C₆-alkenyl, in each case fluorine- or chlorine-substituted C₁-C₄-alkyl or C₂-C₆-alkenyl, represents C₃-C₆-cycloalkyl which is optionally substituted by fluorine, chlorine, C₁-C₄-alkyl or by fluorine- or chlorine-substituted C₁-C₄-alkyl, or represents phenyl which is optionally mono- to trisubstituted by C₁-C₄-alkylcarbonylamino, C₁-C₄-allcylcarbonyl-C₁-C₄-alkylamino and/or radicals from the list W³,
R¹² represents hydrogen, C₁-C₄-alkyl, C₃-C₆-alkenyl, in each case fluorine- or chlorine-substituted C₁-C₄-alkyl or C₃-C₆-alkenyl, represents C₃-C₆-cycloalkyl or C₃-C₆-cycloalkyl-C₁-C₄-alkyl, each of which is optionally substituted by fluorine, chlorine, C₁-C₄-alkyl or by fluorine- or chlorine-substituted C₁-C₄-alkyl, or represents phenyl-C₁-C₄-alkyl or naphthyl-C₁-C₄-alkyl, each of which is optionally mono- to trisubstituted by radicals from the list W³,
R¹³ and R¹⁴ independently of one another each represent hydrogen, C₁-C₄-alkyl, C₃-C₆-alkenyl, in each case fluorine- or chlorine-substituted C₁-C₄-alkyl or C₃-C₆-alkenyl, represent C₁-C₄-alkoxy, represent C₃-C₆-cycloalkyl or C₃-C₆-cycloalkyl-C₁-C₄-alkyl, each of which is optionally substituted by fluorine, chlorine, C₁-C₄-alkyl or by fluorine- or chlorine-substituted C₁-C₄-alkyl, represent phenyl or phenyl-C₁-C₄-alkyl, each of which is optionally mono- to trisubstituted by radicals from the list W³, represent -OR¹² or -NR¹¹R¹² or together represent -(CH₂)₅-, -(CH₂)₆- or -(CH₂)₂-O-(CH₂)₂-,
R¹⁵ represents -OR¹², -NR¹¹R¹² or -N(R¹¹)-COOR¹²,
R¹⁶, R¹⁷ and R¹⁸ independently of one another each represent C₁-C₄-alkyl,
W¹ represents hydrogen, fluorine, chlorine, bromine, iodine, cyano, formyl, nitro, C₁-C₄-alkyl, C₁-C₄-alkoxy, in each case fluorine- or chlorine-substituted C₁-C₄-alkyl or C₁-C₄-alkoxy, represents C₁-C₄-alkylcarbonyl, C₁-C₄-alkoxycarbonyl or -S(O)ₒR³,
W² represents fluorine, chlorine, bromine, cyano, formyl, nitro, C₁-C₄-alkyl, C₁-C₄-alkoxy, in each case fluorine- or chlorine-substituted C₁-C₄-alkyl or C₁-C₄-alkoxy, represents C₁-C₄-alkylcarbonyl, C₁-C₄-alkoxycarbonyl, -S(O)ₒR³ or -C(R¹¹)=N-R¹⁵,
W³ represents fluorine, chlorine, bromine, cyano, nitro, C₁-C₄-alkyl, C₁-C₄-alkoxy, in each case fluorine- or chlorine-substituted C₁-C₄-alkyl or C₁-C₄-alkoxy, represents di-C₁-C₄-alkylamino, -S(O)ₒR³, -COOR¹⁹ or -CONR²⁰R²¹,
R¹⁹ represents hydrogen, C₁-C₄-alkyl, fluorine- or chlorine-substituted C₁-C₄-alkyl, represents C₃-C₆-cycloalkyl which is optionally substituted by fluorine, chlorine, C₁-C₄-alkyl or by fluorine- or chlorine-substituted C₁-C₄-alkyl, or represents phenyl, which is optionally mono- to trisubstituted by radicals from the list W⁴,
R²⁰ and R²¹ independently of one another each represent hydrogen, C₁-C₄-alkyl, C₃-C₆-alkenyl, in each case fluorine- or chlorine-substituted C₁-C₄-alkyl or C₃-C₆-alkenyl, represent C₁-C₄-alkoxy, represent C₃-C₆-cycloalkyl or C₃-C₆-cycloalkyl-C₁-C₄-alkyl, each of which is optionally substituted by fluorine, chlorine, C₁-C₄-alkyl or by fluorine- or chlorine-substituted-C₁-C₄-alkyl, or represent phenyl or phenyl-C₁-C₄-alkyl, each of which is optionally mono- to trisubstituted by radicals from the list W⁴, represent -OR¹⁶ or - NR¹⁷R¹⁸ or together represent -(CH₂)₅-, -(CH₂)₆- or -(CH2)2-O-(CH₂)₂-, and
W⁴ represents fluorine, chlorine, bromine, cyano, nitro, C₁-C₄-alkyl, C₁-C₄-alkoxy, in each case fluorine- or chlorine-substituted C₁-C₄-alkyl or C₁-C₄-alkoxy, di-C₁-C₄-alkylamino, C₁-C₄-alkoxycarbonyl, di-C₁-C₆-alkylanunocarbonyl or -S(O)ₒR³.

3. Compounds of the formula (I) according to Claim 1, in which
Ar represents the radical 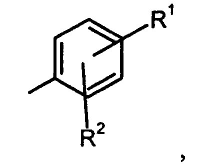
R¹ represents a substituent in the meta or para position from the group consisting of hydrogen, fluorine, chlorine, bromine, iodine, cyano, - CO-NR⁴R⁵, tetrahydropyranyl or one of the groupings below
(l) - X - A 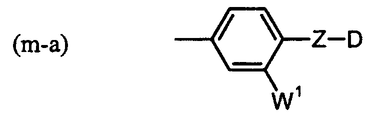
(n) -Y-E,
R² represents hydrogen, fluorine, chlorine, bromine, methyl, ethyl, methoxy, ethoxy, methylthio, ethylthio, trifluoromethyl, difluoromethoxy, trifluoromethoxy or trifluormethylthio,
o represents 0 or 2,
R³ represents methyl, ethyl, n-propyl, isopropyl, difluoromethyl or trifluoromethyl,
R⁴ and R⁵ independently of one another each represent hydrogen, methyl, ethyl, n-propyl, isopropyl, n-butyl, isobutyl, sec-butyl, tert-butyl or represent phenyl or benzyl, each of which is optionally monosubstituted by a radical from the list W¹,
X represents a direct bond, oxygen, sulphur, carbonyl, -CH₂-, -(CH₂)₂-, -CH=CH- (E or Z), -C≡C-, -CH₂O-, -(CH₂)₂O-, -CH(CH₃)O-, -OCH₂-, -O(CH₂)₂-, -SCH₂-, -S(CH₂)₂-, -SCH(CH₃)-, C₁-C₄-alkylenedioxy, in particular -OCH₂O-, -O(CH₂)₂O- or -OCH(CH₃)O-,
A represents phenyl which is optionally mono- or disubstituted by radicals from the list W¹ or represents furyl, benzofuryl, thienyl, benzothienyl, oxazolyl, benzoxazolyl, thiazolyl, benzothiazolyl, pyrrolyl, pyridyl, pyrimidyl, 1,3,5-triazinyl, quinolinyl, isoquinolinyl, indolyl, purinyl, benzodioxolyl, indanyl, benzodioxanyl or chromanyl, each of which is optionally mono- or disubstituted by radicals from the list W²,
Z represents oxygen or sulphur,
D represents hydrogen, methyl, ethyl, n-propyl, isopropyl, n-butyl, isobutyl, sec-butyl, tert-butyl, the isomeric pentyls, the isomeric hexyls, n-heptyl, n-octyl, n-isooctyl, n-nonyl, n-decyl, n-undecyl, n-dodecyl, n-tridecyl, n-tetradecyl, n-pentadecyl, n-hexadecyl, 2-propenyl, butenyl, pentenyl, hexenyl, propargyl, butinyl, pentinyl, -CF₃, -CHF₂, -CClF₂, -CF₂CHFCl, -CF₂CH₂F, -CF₂CHF₂, -CF₂CCl₃, -CH₂CF₃, -CF₂CHFCF₃, -CH₂CF₂CHF₂, -CH₂CF₂CF₃, represents cyclopropyl, cyclobutyl, cyclopentyl, cyclohexyl, cyclopropylmethyl, cyclobutylmethyl, cyclopentylmethyl or cyclohexylmethyl, each of which is optionally mono- to trisubstituted by fluorine, chlorine, bromine, methyl, ethyl, n-propyl, isopropyl, n-butyl, isobutyl, sec-butyl, tert-butyl, ethenyl, 1-propenyl, 2,2-dimethylethenyl, -CH=CCl₂, phenyl, styryl, in each case fluorine-, chlorine- or bromine-substituted phenyl or 4-chlorostyryl, represents cyclopentenyl, cyclohexenyl, cyclohexenylmethyl or cyclopentenylmethyl, each of which is optionally substituted by fluorine, chlorine, methyl, ethyl, n-propyl, isopropyl, n-butyl, isobutyl, sec-butyl or tert-butyl, represents benzyl, phenethyl, naphthylmethyl, tetrahydronaphthylmethyl, furylmethyl, thienylmethyl, pyrrolylmethyl, oxazolylmethyl, isoxazolylmethyl, thiazolylmethyl or pyridylmethyl, each of which is optionally mono- or disubstituted by nitro, fluorine, chlorine, bromine, methyl, ethyl, n-propyl, isopropyl, n-butyl, isobutyl, sec-butyl, tert-butyl, methoxy, ethoxy, n-propoxy, isopropoxy, n-butoxy, isobutoxy, sec-butoxy, tert-butoxy, trifluoromethyl, trifluoromethoxy, difluoromethoxy or chlorodifluoromethoxy, represents -CO-R⁶, -CO-NR⁷R⁸ or the grouping
- (CH₂)ₚ - (CR⁹R¹⁰)_{q} - (CH₂)ᵣ - G,
Z and D together may also very particularly preferably represent phenoxymethyl which is optionally mono- or disubstituted by nitro, fluorine, chlorine, bromine, methyl, ethyl, n-propyl, i-propyl, methoxy, ethoxy, n-propoxy, i-propoxy, trifluoromethyl, trifluoromethoxy, difluoromethoxy or chlorodifluoromethoxy,
Y represents a direct bond, oxygen, sulphur, carbonyl, -CH₂-, -(CH₂)₂-, -CH=CH- (E or Z), -C≡C-, -CH₂O-, -(CH₂)₂O-, -CH(CH₃)O-, -OCH₂-, -O(CH₂)₂-, -SCH₂-, -S(CH₂)₂-, -SCH(CH₃)-, C₁-C₄-alkylenedioxy, in particular -OCH₂O- or -O(CH₂)₂O- or represents p-phenylene which is optionally monosubstituted by a radical from the list W¹,
E represents hydrogen, methyl, ethyl, n-propyl, isopropyl, n-butyl, isobutyl, sec-butyl, tert-butyl, the isomeric pentyls, the isomeric hexyls, n-heptyl, n-octyl, n-isooctyl, n-nonyl, n-decyl, n-undecyl, n-dodecyl, n-tridecyl, n-tetradecyl, n-pentadecyl, n-hexadecyl, 2-propenyl, butenyl, pentenyl, hexenyl, propargyl, butinyl, pentinyl, -CF₃, -CHF₂, -CClF₂, -CF₂CHFCl, -CF₂CH₂F, -CF₂CHF₂, -CF₂CCl₃, -CH₂CF₃, -CF₂CHFCF₃, -CH₂CF₂CHF₂, -CH₂CF₂CF₃, represents cyclopropyl, cyclobutyl, cyclopentyl or cyclohexyl, each of which is optionally mono- to trisubstituted by fluorine, chlorine, bromine, methyl, ethyl, n-propyl, isopropyl, n-butyl, isobutyl, sec-butyl, tert-butyl, ethenyl, 1-propenyl, 2,2-dimethylethenyl, -CH=CCl₂, phenyl, styryl, in each case fluorine-, chlorine- or bromine-substituted phenyl or by 4-chlorostyryl, represents cyclopentenyl or cyclohexenyl, each of which is optionally substituted by fluorine, chlorine, methyl, ethyl, n-propyl, isopropyl, n-butyl, isobutyl, sec-butyl or tert-butyl, represents phenyl which is optionally mono- or disubstituted by radicals from the list W¹, represents furyl, thienyl, pyrrolyl, oxazolyl, isoxazolyl, thiazolyl or pyridyl, each of which is optionally mono- or disubstituted by radicals from the list W², or represents the grouping
- (CH₂)ₚ - (CR⁹R¹⁰)_{q} - (CH₂)ᵣ - G,
R⁶ represents methyl, ethyl, n-propyl, isopropyl, n-butyl, isobutyl, sec-butyl, tert-butyl, methoxy, ethoxy, n-propoxy, isopropoxy, n-butoxy, isobutoxy, sec-butoxy, tert-butoxy, cyclopropyl, cyclohexyl, cyclohexyloxy, cyclohexylmethyloxy, phenyl, 2-chlorophenyl, 3-chlorophenyl, 2,6-difluorophenyl, 2,4-dichlorophenyl, 3,4-dichlorophenyl, 2-trifluoromethoxyphenyl or 4-trifluoromethoxyphenyl,
R⁷ represents hydrogen,
R⁸ represents methyl, ethyl or phenyl which is optionally monosubstituted by chlorine,
p, q and r independently of one another each represent 0, 1, 2 or 3, their sum being smaller than 4,
R⁹ and R¹⁰ independently of one another each represent hydrogen, methyl, ethyl, n-propyl, isopropyl, n-butyl, isobutyl, sec-butyl, tert-butyl,
G represents cyano, represents 5,6-dihydrodioxazin-2-yl, 3-pyridyl, 3-furyl, 3-thienyl, 2-thiazolyl, 5-thiazolyl, 2-dioxolanyl, 1,3-dioxan-2-yl, 2-dithiolanyl, 1,3-dithian-2-yl or 1,3-thioxan-2-yl, each of which is optionally mono- to trisubstituted by fluorine, chlorine, bromine, methyl, ethyl, n-propyl, isopropyl or trifluoromethyl and optionally, at the point of linkage, by the radical R¹¹, or represents one of the groupings below:
(a) ―CO―R¹¹
(b) ―CO―OR¹²
(c) ―CO―NR¹³R¹⁴
(d) ―CS―NR¹³R¹⁴ 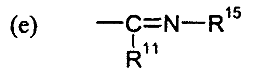 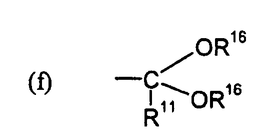 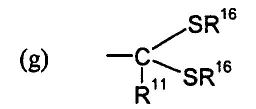 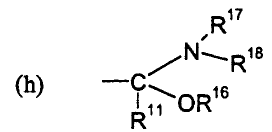 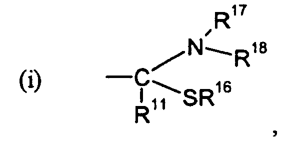
R¹¹ represents hydrogen, methyl, ethyl, n-propyl, isopropyl, n-butyl, isobutyl, sec-butyl, tert-butyl, the isomeric pentyls, the isomeric hexyls, -CF₃, -CHF₂, -CClF₂, -CF₂CHFCl, -CF₂CH₂F, -CF₂CHF₂, -CF₂CCl₃, -CH₂CF₃, C₃-C₆-alkenyl, C₃-C₆-alkenyl which is mono- to trisubstituted by fluorine or chlorine, represents cyclopropyl, cyclopentyl or cyclohexyl, each of which is optionally mono- or disubstituted by fluorine, chlorine, methyl, ethyl, n-propyl, isopropyl, -CF₃, -CHF₂, -CClF₂, -CF₂CHFCl, -CF₂CH₂F, -CF₂CHF₂, -CF₂CCl₃ or -CH₂CF₃, or represents phenyl which is optionally mono- or disubstituted by methylcarbonylamino, ethylcarbonylamino, methylcarbonyl-methylamino and/or radicals from the list W³,
R¹² represents hydrogen, methyl, ethyl, n-propyl, isopropyl, n-butyl, isobutyl, sec-butyl, tert-butyl, -CH₂CF₃, allyl, represents cyclopropyl, cyclopentyl, cyclohexyl, cyclopropylmethyl, cyclopentylmethyl, cyclohexylmethyl, cyclopropylethyl, cyclopentylethyl or cyclohexylethyl, each of which is optionally mono- or disubstituted by fluorine, chlorine, methyl, ethyl, n-propyl, isopropyl, -CF₃, -CHF₂, -CClF₂, -CF₂CHFCl, -CF₂CH₂F, -CF₂CHF₂, -CF₂CCl₃ or -CH₂CF₃, or represents benzyl or phenethyl, each of which is optionally mono- or disubstituted by radicals from the list W³,
R¹³ and R¹⁴ independently of one another each represent hydrogen, methyl, ethyl, n-propyl, isopropyl, n-butyl, isobutyl, sec-butyl, tert-butyl, -CH₂CF₃, methoxy, ethoxy, allyl, represent cyclopropyl, cyclopentyl, cyclohexyl, cyclopropylmethyl, cyclopentylmethyl or cyclohexylmethyl, each of which is optionally mono- or disubstituted by fluorine, chlorine, methyl, ethyl, n-propyl, isopropyl or trifluoromethyl, represent phenyl, benzyl or phenethyl, each of which is optionally mono- or disubstituted by radicals from the list W³, represent -OR¹² or -NR¹¹R¹²,
R¹⁵ represents -OR¹², -NR¹¹R¹² or -N(R¹¹)-COOR¹²,
R¹⁶, R¹⁷ and R¹⁸ independently of one another each represent methyl, ethyl, n-propyl or isopropyl,
W¹ represents hydrogen, fluorine, chlorine, bromine, cyano, formyl, nitro, methyl, ethyl, n-propyl, isopropyl, n-butyl, isobutyl, sec-butyl, tert-butyl, methoxy, ethoxy, n-propoxy, isopropoxy, n-butoxy, isobutoxy, sec-butoxy, tert-butoxy, -CF₃, -CHF₂, -CClF₂, -CF₂CHFCl, -CF₂CH₂F, -CF₂CHF₂, -CF₂CCl₃, -CH₂CF₃, -CF₂CHFCF₃, -CH₂CF₂CHF₂, -CH₂CF₂CF₃, trifluoromethoxy, difluoromethoxy, chlorodifluoromethoxy, acetyl, propionyl, butyryl, isobutyryl, methoxycarbonyl, ethoxycarbonyl, n-propoxycarbonyl, isopropoxycarbonyl, n-butoxycarbonyl, isobutoxycarbonyl, sec-butoxycarbonyl, tert-butoxycarbonyl or -S(O)ₒR³,
W² represents fluorine, chlorine, bromine, cyano, methyl, ethyl, n-propyl, isopropyl, trifluoromethyl, trifluoromethoxy, difluoromethoxy, chlorodifluoromethoxy, acetyl, trifluoromethylthio, -CH=N-OCH₃, -CH=N-OC₂H₅, -CH=N-OC₃H₇, -C(CH₃)=N-OCH₃, -C(CH₃)=N-OC₂H₅, -C(CH₃)=N-OC₃H₇, -C(C₂H₅)=N-OCH₃, -C(C₂H₅)=N-OC₂H₅ or -C(C₂H₅)=N-OC₃H₇,
W³ represents fluorine, chlorine, cyano, nitro, methyl, ethyl, methoxy, ethoxy, methylthio, trifluoromethyl, trifluoromethoxy, trifluoromethylthio, dimethylamino, diethylamino, -COOR¹⁹ or -CONR²⁰R²¹,
R¹⁹ represents hydrogen, methyl, ethyl, n-propyl, isopropyl, tert-butyl, -CH₂CF₃, represents cyclopropyl, cyclopentyl or cyclohexyl, each of which is optionally mono- or disubstituted by fluorine, chlorine, methyl, ethyl, n-propyl, isopropyl or -CF₃, or represents phenyl which is optionally mono- or disubstituted by radicals from the list W⁴,
R²⁰ and R²¹ independently of one another each represent hydrogen, methyl, ethyl, n-propyl, isopropyl, n-butyl, isobutyl, sec-butyl, tert-butyl, -CH₂CF₃, methoxy, ethoxy, allyl, represent cyclopropyl, cyclopentyl, cyclohexyl, cyclopropylmethyl, cyclopentylmethyl or cyclohexylmethyl, each of which is optionally mono- or disubstituted by fluorine or chlorine, represent phenyl, benzyl or phenethyl, each of which is optionally mono- or disubstituted by the radicals from the list W⁴, represent -OR¹⁶ or -NR¹⁷R¹⁸,
W⁴ represents fluorine, chlorine, bromine, cyano, nitro, methyl, ethyl, tert-butyl, methoxy, ethoxy, methylthio, trifluoromethyl, trifluoromethoxy or trifluoromethylthio.

4. Process for preparing compounds of the formula (I) according to Claim 1, **characterized in that**
A) compounds of the formula (I) 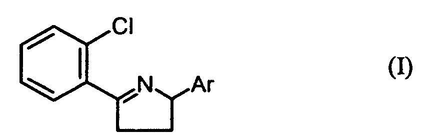 in which
Ar is as defined in Claim 1
are obtained by
a) reacting aminoketone derivatives of the formula (VIII) 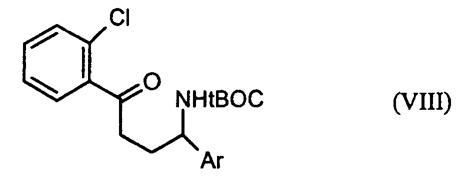 in which
Ar is as defined above
with an acid, followed by cyclocondensation, if appropriate in the presence of an acid binder, or
b) reducing the nitro group of nitroketones of the formula (XVIII), 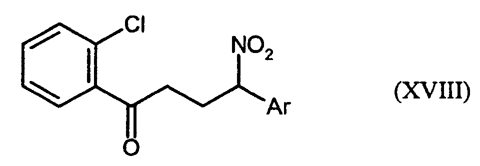 in which
Ar is as defined in Claim 1, where 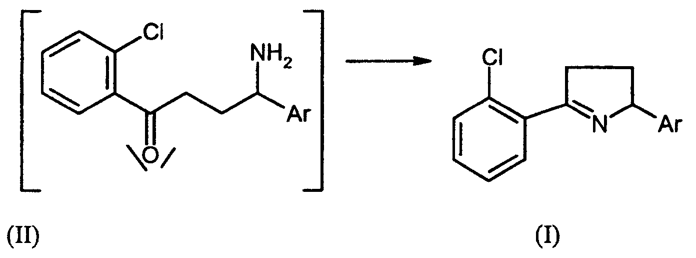 an aminoketone intermediate of the formula (II) is formed which, however, is cyclocondensed in situ to (I), in particular in an acidic medium, or
c) hydrolysing imines of the formula (XXVII) 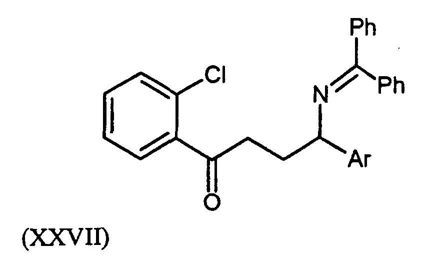
in which Ar is as defined in Claim 1
with aqueous acids 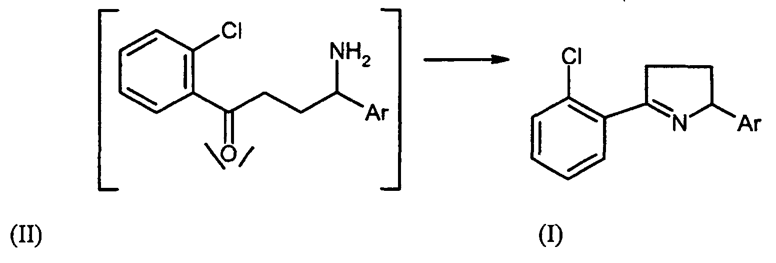 where
an aminoketone intermediate of the formula (II) is formed which, however, is cyclocondensed in situ to (I),
B) reacting compounds of the formula (III) 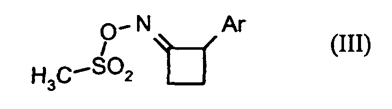 in which
Ar is as defined in Claim 1
with aryl Grignard compounds of the formula (IV) 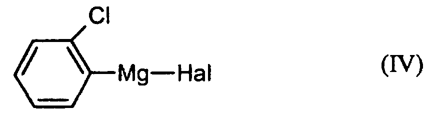 in which
Hal represents bromine or iodine
in the presence of a diluent,
C) compounds of the formula (I-b) 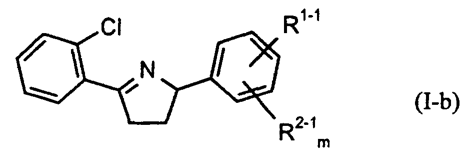 in which
m is as defined in Claim 1
R¹⁻¹ represents A or one of the groupings below
(m) ―B―Z-D 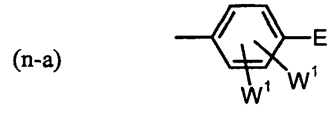
where
A, B, D, E, W¹ and Z are each as defined in Claim 1 and
R²⁻¹ represents hydrogen, fluorine, cyano, nitro, alkyl, alkoxy, halogenoalkyl, halogenoalkoxy, alkoxyalkoxy or -SR³ where
R³ is as defined in Claim 1
are obtained by coupling compounds of the formula (V) 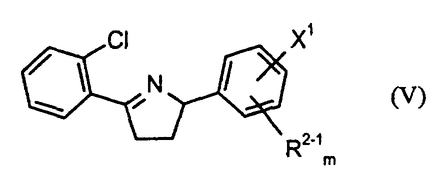 in which
m are as defined in Claim 1,
R²⁻¹ is as defined above and
X¹ represents bromine, iodine or -OSO₂CF₃
with boronic acids of the formula (VI)
R¹⁻¹ B(OH)₂ (VI)
in which
R¹⁻¹ is as defined above
in the presence of a catalyst and in the presence of an acid binder and in the presence of a solvent,
D) compounds of the formula (I-c) 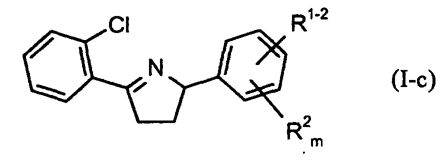 in which
R² and m are each as defined in Claim 1,
R¹⁻² represents one of the groupings below
(m-b) -B - Z - D¹
(n-b) -Y¹ - E¹
in which
B and Z are each as defined in Claim 1,
Y¹ represents oxygen or sulphur and
D¹ and E¹ represent the grouping
-(CH₂)ₚ-(CR⁹R¹⁰)_{q}-(CH₂)ᵣ - G
in which
R⁹, R¹⁰, G, p, q and r are each as defined above
are obtained by condensing cyclic imines of the formula (I-d) 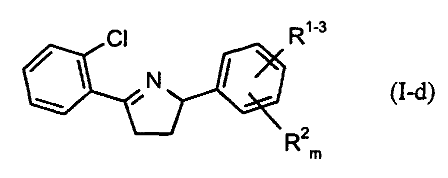 in which
R² and m are each as defined in Claim 1 and
R¹⁻³ represents one of the groupings below
(m-c) -B - Z - H
(n-c) -Y¹ - H
in which
B, and Z are each as defined in Claim 1
Y¹ is as defined above
with compounds of the formula (VII)
Ab - (CH₂)ₚ - (CR⁹R¹⁰)_{q} - (CH₂)ᵣ - G (VII)
in which
R⁹, R¹⁰, G, p, q and r are each as defined in Claim 1 and
Ab represents a leaving group,
E) compounds of the formula (I-e) 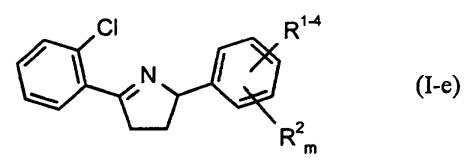 in which
R² and m are each as defined in Claim 1 and
R¹⁻⁴ represents a grouping from the description of the compounds of the formula (I) according to the invention which contains the radical G, where
G represents one of the abovementioned groupings (e) to (k),
are obtained by generally customary and known derivatizations of the corresponding keto derivatives, carboxylic acid derivatives or nitriles, i.e. of compounds of the formula (I) in which G represents cyano or one of the groupings (a) to (d),
F) compounds of the formula (I-f) 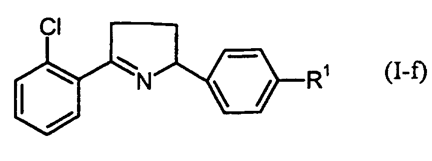 in which R¹ is as defined in Claim 1 are also obtained by reacting, in a first step,
α) o-chloroacetophenone of the formula (F-I) 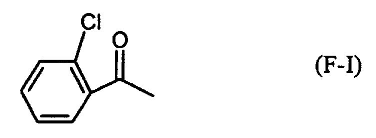 with dimethylmethyleneammonium chloride of the formula (F-II) 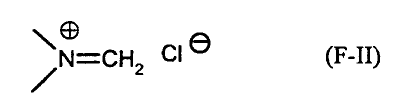 to the compound of the formula (F-III) 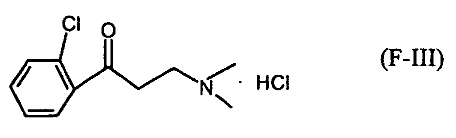 and reacting this, in a second step,
β) with benzyl cyanides of the formula (F-IV) 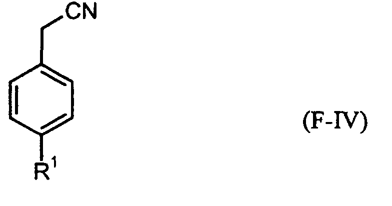 in which R¹ is as defined in Claim 1 to give compounds of the formula (F-V) 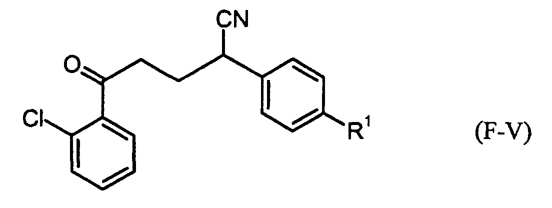 which, in the next step,
δ) are derivatized with aqueous sodium hydroxide solution/H₂O₂ to give compounds of the formula (F-VI) 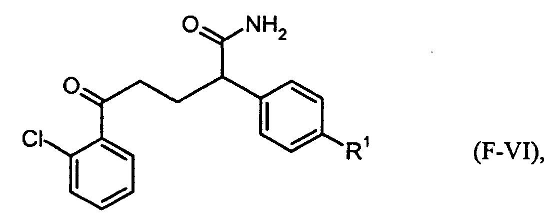 in which R¹ is as defined in Claim 1, and cyclizing these, in a final step,
γ) by reaction with PIFA (1,1-bis(trifluoroacetoxy)iodobenzene) of the formula (F-VII) 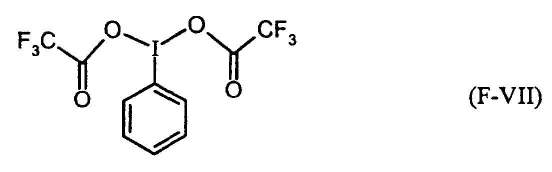 to cyclic imines of the formula (I-f),
G) compounds of the formula (I-f) 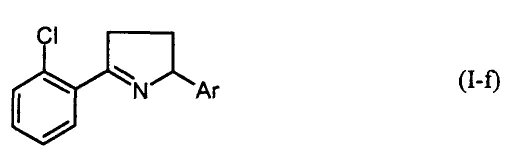 in which Ar is as defined in Claim 1 are also obtained by reacting, in a first step,
α) arylbutyrolactams of the formula (XI) 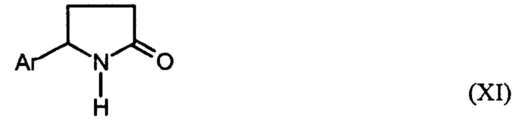 with o-chlorobenzoyl chloride 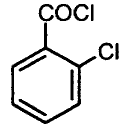 to give compounds of the formula G(I) 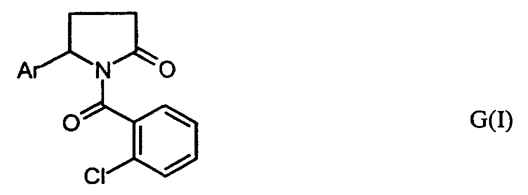 and reacting these, in a second step,
β) with methyl o-chlorobenzoate 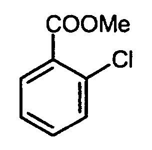 to give compounds of the formula G(II) 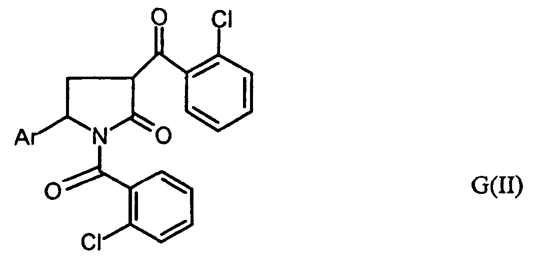 which, in a final step, are reacted
δ) with HBr/glacial acetic acid to give compounds of the formula (I-f)

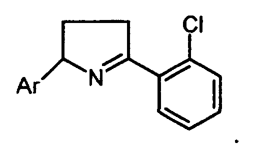

5. Compounds of the formula (VIII) 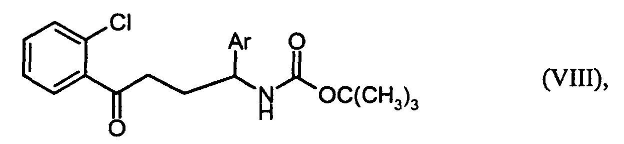 in which
Ar is as defined in Claim 1.

6. Compounds of the formula (XVIII) 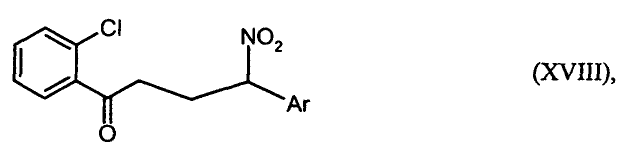 in which
Ar is as defined in Claim 1.

7. Pesticides, **characterized in that** they contain at least one compound of the formula (I) according to Claim 1.

8. Use of compounds of the formula (I) according to Claim 1 for controlling pests, with the exception of the therapeutic treatment of humans and animals.

9. Method of controlling pests, with the exception of the therapeutic treatment of humans and animals, **characterized in that** compounds of the formula (I) according to Claim 1 are allowed to act on pests and/or their habitat.

10. Process for preparing pesticides, **characterized in that** compounds of the formula (I) according to Claim 1 are mixed with extenders and/or surfactants.

11. Use of compounds of the formula (I) according to Claim 1 for preparing pesticides.

12. Use of compounds of the formula (I) according to Claim 1 for preparing a composition for controlling arthropods in animal husbandry.

## Revendications

1. Composés de formule (I) 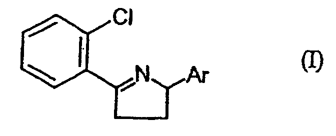 dans laquelle
Ar représente le reste 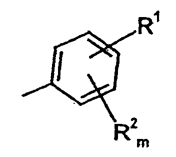
dans lequel
m a la valeur 0, 1, 2 ou 3,
R¹ est un substituant en position méta ou para de la série hydrogène, halogène, cyano, tri-(alkyle en C₁ à C₆)-silyle, -CO-NR⁴R⁵, tétrahydropyrannyle ou l'un des groupements suivants
(l) -X-A
(m) -B-Z-D
(n) -Y-E,
R² représente l'hydrogène, un halogène, un groupe cyano, nitro, un reste alkyle en C₁ à C₁₆, alkoxy en C₁ à C₁₆, halogénalkyle en C₁ à C₆, halogénalkoxy en C₁ à C₆, (alkoxy en C₁ à C₈)-(alkoxy en C₁ à C₈) ou -S(O)ₒR³,
o a la valeur 0, 1 ou 2,
R³ est un reste alkyle en C₁ à C₆ éventuellement substitué par du fluor ou du chlore,
R⁴ et R⁵ représentent, indépendamment l'un de l'autre, l'hydrogène, un reste alkyle en C₁ à C₆, halogénalkyle en C₁ à C₆ ou un reste phényle ou phényl-(alkyle en C₁ à C₄) dont chacun est éventuellement substitué une à trois fois par des restes de la liste W¹,
X désigne une liaison directe, l'oxygène, le soufre, un groupe carbonyle, carbonyloxy, oxycarbonyle, un reste alkylène en C₁ à C₄, alcénylène en C₂ à C₄, alcynylène en C₂ à C₄, alkylènoxy en C₁ à C₄, oxyalkylène en C₁ à C₄, thioalkylène en C₁ à C₄, alkylènedioxy en C₁ à C₄ ou di-(alkyle en C₁ à C₄)-silylène,
A désigne un reste phényle, naphtyle ou tétrahydronaphtyle dont chacun est éventuellement substitué une à quatre fois par des restes de la liste W¹ ou un reste hétérocyclyle pentagonal à décagonal à 1 ou 2 noyaux aromatiques et 1 à 4 hétéroatomes, éventuellement substitué dans chaque cas 1 à 4 fois par des restes de la liste W², qui contient 0 à 4 atomes d'azote, 0 à 2 atomes d'oxygène et 0 à 2 atomes de soufre (en particulier furyle, benzofuryle, thiényle, benzothiényle, oxazolyle, benzoxazolyle, thiazolyle, benzthiazolyle, pyrrolyle, pyridyle, pyrimidyle, 1,3,5-triazinyle, quinolinyle, isoquinolinyle, indolyle, purinyle, benzodioxolyle, indanyle, benzodioxanyle ou chromanyle),
B représente un reste p-phénylène éventuellement substitué une ou deux fois par des restes de la liste W¹,
Z représente l'oxygène ou le soufre,
D représente l'hydrogène, un reste alkyle en C₁ à C₁₆, alcényle en C₂ à C₁₆, alcynyle en C₂ à C₁₆, halogénalkyle en C₁ à C₁₆, halogénalcényle en C₂ à C₁₆, un reste cycloalkyle en C₃ à C₈ ou (cycloalkyle en C₃ à C₈)-(alkyle en C₁ à C₆) dont chacun est éventuellement substitué par un halogène, un radical alkyle en C₁ à C₄, alcényle en C₂ à C₄, halogénalcényle en C₂ à C₄, phényle, styryle, halogénophényle ou halogénostyryle, un reste cycloalcényle en C₅ à C₈ ou (cycloalcényle en C₅ à C₈)-(alkyle en C₁ à C₄) dont chacun est éventuellement substitué par un halogène ou un radical alkyle en C₁ à C₄, un reste phényl-(alkyle en C₁ à C₆), naphtyle-(alkyle en C₁ à C₆), tétrahydronaphtyle-(alkyle en C₁ à C₆) ou hétaryl-(alkyle en C₁ à C₆) pentagonal ou hexagonal ayant 1 ou 2 hétéroatomes de la série azote, oxygène et soufre (en particulier furylméthyle, thiénylméthyle, pyrrolylméthyle, oxazolylméthyle, isoxazolylméthyle, thiazolylméthyle ou pyridylméthyle) dont chacun est éventuellement substitué par un groupe nitro, un halogène, un radical alkyle en C₁ à C₆, alkoxy en C₁ à C₆, halogénalkyle en C₁ à C₆ ou halogénalkoxy en C₁ à C₆, un groupement -CO-R⁶, -CO-NR⁷-R⁸ ou le groupement
- (CH₂)ₚ - (CR⁹R¹⁰)_{q} - (CH₂)ᵣ - G
Z et D peuvent aussi former ensemble un reste phénoxy-(alkyle en C₁ à C₄) éventuellement substitué dans chaque cas par un radical nitro, halogéno, alkyle en C₁ à C₆, alkoxy en C₁ à C₆, halogénalkyle en C₁ à C₆ ou halogénalkoxy en C₁ à C₆,
Y représente une liaison directe, l'oxygène, le soufre, un groupe carbonyle, carbonyloxy, oxycarbonyle, un reste alkylène en C₁ à C₄, alcénylène en C₂ à C₄, alcynylène en C₂ à C₄, alkylènoxy en C₁ à C₄, oxyalkylène en C₁ à C₄, thioalkylène en C₁ à C₄, alkylènedioxy en C₁ à C₄ ou un reste p-phénylène éventuellement substitué une ou deux fois par des restes de la liste W¹,
E représente l'oxygène, un reste alkyle en C₁ à C₁₆, alcényle en C₂ à C₁₆, alcynyle en C₂ à C₆, halogénalkyle en C₁ à C₁₆, halogénalcényle en C₂ à C₁₆, un reste cycloalkyle en C₃ à C₈ éventuellement substitué par un halogène, un radical alkyle en C₁ à C₄, alcényle en C₂ à C₄, halogénalcényle en C₂ à C₄, phényle, styryle, halogénophényle ou halogénostyryle, un reste cycloalcényle en C₅ à C₈ éventuellement substitué par un halogène ou un radical alkyle en C₁ à C₄, un reste phényle éventuellement substitué une à quatre fois par des restes de la liste W¹ ou un reste hétaryle pentagonal ou hexagonal ayant 1 ou 2 hétéroatomes de la série azote, oxygène et soufre (en particulier furyle, thiényle, pyrrolyle, oxazolyle, isoxazolyle, thiazolyle ou pyridyle) éventuellement substitué une à quatre fois par des restes de la liste W², ou le groupement
-(CH₂)ₚ - (CR⁹R¹⁰)_{q} - (CH₂)ᵣ - G
R⁶ est un reste alkyle en C₁ à C₁₂, alkoxy en C₁ à C₁₂, alcényle en C₂ à C₁₂, alcényloxy en C₂ à C₁₂, un reste cycloalkyle en C₃ à C₈, cycloalkyloxy en C₃ à C₈ ou (cycloalkyle en C₃ à C₈)-(alkyloxy en C₁ à C₆) dont chacun est éventuellement substitué par un halogène, un radical alkyle en C₁ à C₄, alcényle en C₂ à C₄, halogénalkyle en C₁ à C₄ ou halogénalcényle en C₂ à C₄, ou un reste phényle ou naphtyle dont chacun est éventuellement substitué une à quatre fois par un radical nitro, halogéno, alkyle en C₁ à C₁₂, alkoxy en C₁ à C₁₂, halogénalkyle en C₁ à C₁₂ ou halogénalkoxy en C₁ à C₁₂,
R⁷ représente l'hydrogène ou un reste alkyle en C₁ à C₁₂,
R⁸ représente un reste alkyle en C₁ à C₁₂, halogénalkyle en C₁ à C₁₂, un reste cycloalkyle en C₃ à C₈ ou (cycloalkyle en C₃ à C₈) - (alkyle en C₁ à C₆) dont chacun est éventuellement substitué par un radical halogéno, alkyle en C₁ à C₄, alcényle en C₂ à C₄, halogénalkyle en C₁ à C₄ ou halogénalcényle en C₂ à C₄, ou bien un reste phényle ou phényl-(alkyle en C₁ à C₆) dont chacun est éventuellement substitué une à quatre fois par un radical halogéno, alkyle en C₁ à C₁₂, alkoxy en C₁ à C₁₂, halogénalkyle en C₁ à C₁₂ ou halogénalkoxy en C₁ à C₁₂,
p, q et r ont indépendamment les uns des autres la valeur O, 1, 2 ou 3, leur somme étant inférieure à 6,
R⁹ et R¹⁰ représentent indépendamment l'un de l'autre l'hydrogène ou un reste alkyle en C₁ à C₄,
G est un groupe cyano, un hétérocycle pentagonal ou hexagonal ayant 1 à 3 hétéroatomes identiques ou différents de la série azote, oxygène et soufre, éventuellement substitué une à trois par un radical halogéno, alkyle en C₁ à C₄ ou halogénalkyle en C₁ à C₄, et, le cas échéant, dans la position de liaison par le reste R¹¹ (en particulier 5,6-dihydrodioxazine-2-yle, 3-pyridyle, 3-furyle, 3-thiényle, 2-thiazolyle, 5-thiazolyle, 2-dioxolanyle, 1,3-dioxanne-2-yle, 2-dithiolanyle, 1,3-dithiane-2-yle ou 1,3-thioxane-2-yle) ou l'un des groupements suivants :
(a) ―CO―R¹¹
(b) ―CO―OR¹²
(c) ―CO―NR¹³R¹⁴
(d) ―CS―NR¹³R¹⁴ 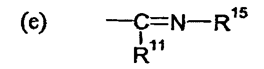 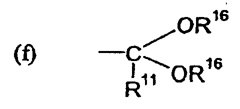 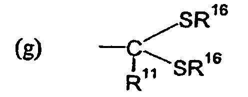 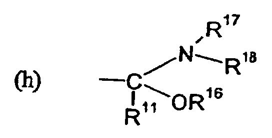 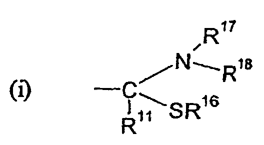 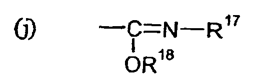 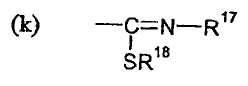
R¹¹ représente l'hydrogène, un reste alkyle en C₁ à C₆, alcényle en C₂ à C₆, halogénalkyle en C₁ à C₄, halogénalcényle en C₂ à C₄, un reste cycloalkyle en C₃ à C₆ éventuellement substitué par un radical halogéno, alkyle en C₁ à C₄ ou halogénalkyle en C₁ à C₄ ou un reste phényle éventuellement substitué une à cinq fois par un radical (alkyle en C₁ à C₄)-carbonylamino, (alkyle en C₁ à C₄)-carbonyle-(alkylamino en C₁ à C₄) et/ou des restes de la liste W³,
R¹² représente l'hydrogène, un reste alkyle en C₁ à C₄, alcényle en C₂ à C₆, halogénalkyle en C₁ à C₄, halogénalcényle en C₂ à C₆, un reste cycloalkyle en C₃ à C₆ ou (cycloalkyle en C₃ à C₆)-(alkyle en C₁ à C₆) dont chacun est éventuellement substitué par un radical halogéno, alkyle en C₁ à C₄ ou halogénalkyle en C₁ à C₄, ou un reste (aryle en C₆ à C₁₀)-(alkyle en C₁ à C₄) (notamment phényl-(alkyle en C₁ à C₄) ou naphtyl-(alkyle en C₁ à C₄)) éventuellement substitué une à quatre fois par des restes de la liste W³,
R¹³ et R¹⁴ représentent indépendamment l'un de l'autre l'hydrogène, un reste alkyle en C₁ à C₄, alcényle en C₃ à C₆, halogénalkyle en C₁ à C₄, halogénalcényle en C₃ à C₆, alkoxy en C₁ à C₄, un reste cycloalkyle en C₃ à C₆ ou (cycloalkyle en C₃ à C₆)-(alkyle en C₁ à C₄) dont chacun est éventuellement substitué par un radical halogéno, alkyle en C₁ à C₄ ou halogénalkyle en C₁ à C₄, un reste phényle ou phényl-(alkyle en C₁ à C₄) dont chacun est éventuellement substitué une à cinq fois par des restes de la liste W³, un groupement -OR¹² ou -NR¹¹R¹² ou forment ensemble une chaîne alkylénique de 4 à 6 chaînons, dans laquelle un groupe méthylène est éventuellement remplacé par de l'oxygène,
R¹⁵ est un groupement -OR¹², -NR¹¹R¹² ou -N(R¹¹)-COOR¹²,
R¹⁶, R¹⁷ et R¹⁸ représentent indépendamment les uns des autres un reste alkyle en C₁ à C₆,
W₁ représente l'hydrogène, un halogène, un groupe cyano, formyle, nitro, un reste alkyle en C₁ à C₆, tri-(alkyle en C₁ à C₄)-silyle, alkoxy en C₁ à C₁₆, halogénalkyle en C₁ à C₆, halogénalkoxy en C₁ à C₆, halogénalcényloxy en C₂ à C₆, (alkyle en C₁ à C₆)-carbonyle, (alkoxy en C₁ à C₁₆)-carbonyle, pentafluorothio ou -S(O)ₒR¹³,
W² représente un halogène, un groupe cyano, formyle, nitro, un reste alkyle en C₁ à C₆, tri-(alkyle en C₁ à C₄)-silyle, alkoxy en C₁ à C₁₆, halogénalkyle en C₁ à C₆, halogénalkoxy en C₁ à C₆, (alkyle en C₁ à C₆)-carbonyle, (alkoxy en C₁ à C₁₆)-carbonyle, pentafluorothio, -S(O)ₒR³ ou -C(R¹¹)=N-R¹⁵,
W³ représente un halogène, un groupe cyano, nitro, un reste alkyle en C₁ à C₄, alkoxy en C₁ à C₄, halogénalkyle en C₁ à C₄, halogénalkoxy en C₁ à C₄, di-(alkyle en C₁ à C₄)-amino, -S(O)ₒR³, -COOR¹⁹ ou -CONR²⁰R²¹,
R¹⁹ représente l'hydrogène, un reste alkyle en C₁ à C₄, halogénalkyle en C₁ à C₄, un reste cycloalkyle en C₃ à C₇ éventuellement substitué par un radical halogéno, alkyle en C₁ à C₄ ou halogénalkyle en C₁ à C₄ ou un reste phényle éventuellement substitué une à cinq fois par des restes de la liste W⁴,
R²⁰ et R²¹ représentent indépendamment l'un de l'autre l'hydrogène, un reste alkyle en C₁ à C₄, alcényle en C₃ à C₆, halogénalkyle en C₁ à C₄, halogénalcényle en C₃ à C₆, alkoxy en C₁ à C₄, un reste cycloalkyle en C₃ à C₆ ou (cycloalkyle en C₃ à C₆) - (alkyle en C₁ à C₄) dont chacun est éventuellement substitué par un radical halogéno, alkyle en C₁ à C₄ ou halogénalkyle en C₁ à C₄ ou un reste phényle ou phényl-(alkyle en C₁ à C₄) dont chacun est éventuellement substitué une à cinq fois par des restes de la liste W⁴, un reste -OR¹⁶ ou -NR¹⁷R¹⁸ ou forment ensemble une chaîne alkylénique de 4 à 6 chaînons dans laquelle, le cas échéant, un groupe méthylène est remplacé par de l'oxygène, et représente un halogène, un groupe cyano, nitro, un reste alkyle en C₁ à C₆, alkoxy en C₁ à C₆, halogénalkyle en C₁ à C₆, halogénalkoxy en C₁ à C₆, di-(alkyle en C₁ à C₄)-amino, (alkoxy en C₁ à C₆)-carbonyle, di-(alkyle en C₁ à C₆)-aminocarbonyle ou -S(O)ₒR³.

2. Composés de formule (I) suivant la revendication 1, formule dans laquelle
Ar représente le reste 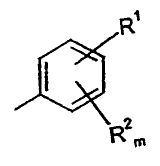
m a la valeur 0, 1 ou 2,
R¹ est un substituant en position méta ou para de la série hydrogène, fluor, chlore, brome, iode, cyano, tri-(alkyle en C₁ à C₆)-silyle, -CO-NR⁴R⁵, tétrahydropyrannyle ou l'un des groupements suivants
(l) -X-A
(m) -B-Z-D
(n) -Y-E,
R² représente l'hydrogène, le fluor, le chlore, le brome, l'iode, un groupe cyano, nitro, un reste alkyle en C₁ à C₁₆ ou alkoxy en C₁ à C₆ substitué dans chaque cas par du fluor ou du chlore, un reste (alkoxy en C₁ à C₈)-(alkoxy en C₁ à C₈) ou -S(O)ₒR³,
o a la valeur 0, 1 ou 2,
R³ est un reste alkyle en C₁ à C₄ ou un reste méthyle ou éthyle dont chacun est substitué par du fluor ou du chlore,
R⁴ et R⁵ représentent, indépendamment l'un de l'autre, l'hydrogène, un reste alkyle en C₁ à C₆, un reste alkyle en C₁ à C₆ substitué par du fluor ou du chlore ou un reste phényle ou benzyle dont chacun est éventuellement substitué une ou deux fois par des restes de la liste W¹,
X représente une liaison directe, l'oxygène, le soufre, un groupe carbonyle, carbonyloxy, oxycarbonyle, un reste alkylène en C₁ à C₄, alcénylène en C₂ à C₄, alcynylène en C₂ à C₄, alkylènoxy en C₁ à C₄, oxyalkylène en C₁ à C₄, thioalkylène en C₁ à C₄, alkylènedioxy en C₁ à C₄ ou di-(alkyle en C₁ à C₄)-silylène,
A est un reste phényle, naphtyle ou tétrahydronaphtyle dont chacun est éventuellement substitué une à trois fois par des restes de la liste W¹ ou un reste hétérocyclyle pentagonal à décagonal à 1 ou 2 noyaux aromatiques, éventuellement substitué 1 à 3 fois par des restes de la liste W², et ayant 1 à 4 hétéroatomes, qui contient 0 à 4 atomes d'azote, 0 à 2 atomes d'oxygène et 0 à 2 atomes de soufre (en particulier furyle, benzofuryle, thiényle, benzothiényle, oxazolyle, benzoxazolyle, thiazolyle, benzthiazolyle, pyrrolyle, pyridyle, pyrimidyle, 1,3,5-triazinyle, quinolinyle, isoquinolinyle, indolyle, purinyle, benzodioxolyle, indanyle, benzodioxanyle ou chromanyle),
B est un reste p-phénylène éventuellement substitué une ou deux fois par des restes de la liste W¹,
Z représente l'oxygène ou le soufre,
D représente l'hydrogène, un reste alkyle en C₁ à C₁₆, alcényle en C₂ à C₁₆, alcynyle en C₂ à C₆, un reste alkyle en C₁ à C₄ ou alcényle en C₂ à C₄ dont chacun est éventuellement substitué par du fluor ou du chlore, un reste cycloalkyle en C₃ à C₆ ou (cycloalkyle en C₃ à C₆)-(alkyle en C₁ à C₄) dont chacun est éventuellement substitué par du fluor, du chlore, du brome, un radical alkyle en C₁ à C₄, alcényle en C₁ à C₄, alcényle en C₂ à C₄ substitué par du fluor ou du chlore, phényle, styryle, phényle ou styryle dont chacun est substitué par du fluor, du chlore ou du brome, un reste cycloalcényle en C₅ ou C₆ ou (cycloalcényle en C₅ ou C₆)-(alkyle en C₁ à C₄) dont chacun est éventuellement substitué par du fluor, du chlore, du brome ou un radical alkyle en C₁ à C₄, un reste phényl-(alkyle en C₁ à C₄), naphtyl-(alkyle en C₁ à C₄), tétrahydronaphtyle-(alkyle en C₁ à C₆) ou hétaryl-(alkyle en C₁ à C₆) pentagonal ou hexagonal ayant 1 ou 2 hétéroatomes de la série azote, oxygène et soufre (en particulier furylméthyle, thiénylméthyle, pyrrolylméthyle, oxazolylméthyle, isoxazolylméthyle, thioazolylméthyle ou pyridylméthyle) dont chacun est éventuellement substitué par un radical nitro, fluoro, chloro, bromo, alkyle en C₁ à C₄, alkoxy en C₁ à C₆, alkyle en C₁ à C₄ ou alkoxy en C₁ à C₄, dont chacun est substitué par du fluor ou du chlore, un groupement -CO-R⁶, -CO-NR⁷-R⁸ ou le groupement
-(CH₂)ₚ - (CR⁹R¹⁰)_{q} - (CH₂)ᵣ - G
Z et D peuvent aussi former ensemble un reste phénoxy-(alkyle en C₁ à C₃) éventuellement substitué par un radical nitro, fluoro, chloro, bromo, alkyle en C₁ à C₄, alkoxy en C₁ à C₄, un radical alkyle en C₁ à C₄ ou alkoxy en C₁ à C₄ dont chacun est substitué par du fluor ou du chlore,
Y représente une liaison directe, l'oxygène, le soufre, un groupe carbonyle, carbonyloxy, oxycarbonyle, un reste alkylène en C₁ à C₄, alcénylène en C₂ à C₄, alcynylène en C₂ à C₄, alkylènoxy en C₁ à C₄, oxyalkylène en C₁ à C₄, thioalkylène en C₁ à C₄, alkylènedioxy en C₁ à C₄ ou un reste p-phénylène éventuellement substitué une ou deux fois par des restes de la liste W¹,
E représente l'hydrogène, un reste alkyle en C₁ à C₁₆, alcényle en C₂ à C₁₆, alcynyle en C₂ à C₆, un reste alkyle en C₁ à C₄ ou alcényle en C₂ à C₄ dont chacun est substitué par du fluor ou du chlore, un reste cycloalkyle en C₃ à C₆, éventuellement substitué par du fluor, du chlore, du brome, un radical alkyle en C₁ à C₄, alcényle en C₂ à C₄, alcényle en C₂ à C₄ substitué par du fluor ou du chlore, un radical phényle, styryle, ou un radical phényle ou styryle dont chacun est substitué par du fluor, du chlore ou du brome, un reste cycloalcényle en C₅ ou C₆ éventuellement substitué par du fluor, du chlore, du brome ou un radical alkyle en C₁ à C₄, un reste phényle éventuellement substitué une à trois fois par des restes de la liste W¹ ou un reste hétaryle pentagonal ou hexagonal ayant 1 ou 2 hétéroatomes de la série azote, oxygène et soufre (en particulier furyle, thiényle, pyrrolyle, oxazolyle, isoxazolyle, thiazolyle ou pyridyle) dont chacun est éventuellement substitué une ou deux fois par des restes de la liste W², ou le groupement
- (CH₂)ₚ - (CR⁹R¹⁰)_{q} - (CH₂)ᵣ - G
R⁶ représente un reste alkyle en C₁ à C₆, alkoxy en C₁ à C₆, alcényle en C₂ à C₆, alcényloxy en C₂ à C₆, un reste cycloalkyle en C₃ à C₆, cycloalkyloxy en C₃ à C₆ ou (cycloalkyle en C₃ à C₆)-(alkyloxy en C₁ ou C₂) dont chacun est éventuellement substitué par du fluor, du chlore, un radical alkyle en C₁ à C₃, ou un radical alkyle en C₁ ou C₂ ou alcényle en C₂ ou C₃, dont chacun est éventuellement substitué par du fluor ou du chlore, ou bien un reste phényle éventuellement substitué une ou deux fois par du fluor, du chlore, du brome, de l'iode, un radical alkyle en C₁ à C₄, alkoxy en C₁ à C₄ ou un radical alkyle en C₁ à C₃ ou alkoxy en C₁ à C₄ dont chacun est substitué par du fluor ou du chlore,
R⁷ représente l'hydrogène ou un reste alkyle en C₁ à C₄,
R⁸ est un reste alkyle en C₁ à C₄ ou un reste phényle ou benzyle dont chacun est éventuellement substitué une ou deux fois par du fluor, du chlore, du brome, un radical alkyle en en C₁ à C₄ ou un radical alkyle en C₁ à C₄ ou alkoxy en C₁ à C₄, dont chacun est substitué par du fluor ou du chlore,
p, q et r ont indépendamment les uns des autres la valeur O, 1, 2 ou 3, leur somme étant inférieure à 6,
R⁹ et R¹⁰ représentent indépendamment l'un de l'autre l'hydrogène ou un reste alkyle en C₁ à C₄,
G est un groupe cyano, un hétérocycle pentagonal ou hexagonal ayant 1 à 3 hétéroatomes identiques ou différents de la série azote, oxygène et soufre, (en particulier 5,6-dihydrodioxazine-2-yle, 3-pyridyle, 3-furyle, 3-thiényle, 2-thiazolyle, 5-thiazolyle, 2-dioxolanyle, 1,3-dioxanne-2-yle, 2-dithiolanyle, 1,3-dithiane-2-yle ou 1,3-thioxane-2-yle) éventuellement substitué une à trois par du fluor, du chlore, du brome, un radical alkyle en C₁ à C₄ ou un radical alkyle en C₁ à C₄ substitué par du fluor ou du chlore, et éventuellement substitué dans la position de liaison par le reste R¹¹, ou l'un des groupements suivants :
(a) ―CO―R¹¹
(b) ―CO―OR¹²
(c) ―CO―NR¹³R¹⁴
(d) ―CS―NR¹³R¹⁴ 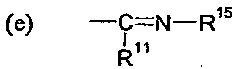 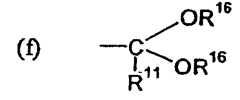 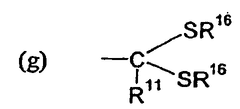 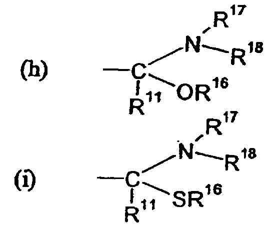 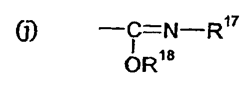 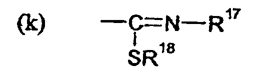
R¹¹ représente l'hydrogène, un reste alkyle en C₁ à C₆, alcényle en C₂ à C₆, un reste alkyle en C₁ à C₄ ou alcényle en C₂ à C₄ dont chacun est éventuellement substitué par du fluor ou du chlore, un reste cycloalkyle en C₃ à C₆ éventuellement substitué par du fluor, du chlore, un radical alkyle en C₁ à C₄ ou un radical alkyle en C₁ à C₄ substitué par du fluor ou du chlore, ou un reste phényle éventuellement substitué une à trois fois par un radical (alkyle en C₁ à C₄)-carbonylamino, (alkyle en C₁ à C₄)-carbonyle-(alkylamino en C₁ à C₄) et/ou des restes de la liste W³,
R¹² représente l'hydrogène, un reste alkyle en C₁ à C₄, alcényle en C₂ à C₆, un reste alkyle en C₃ à C₆ ou alcényle en C₁ à C₆ dont chacun est substitué par du fluor ou du chlore, un reste cycloalkyle en C₃ à C₆ ou (cycloalkyle en C₃ à C₆)-(alkyle en C₁ à C₄) dont chacun est éventuellement substitué par du fluor, du chlore, un radical alkyle en C₁ à C₄ ou un radical alkyle en C₁ à C₄ substitué par du fluor ou du chlore, ou un reste phényl-(alkyle en C₁ à C₄) ou naphtyle-(alkyle en C₁ à C₄) dont chacun est éventuellement substitué une à trois fois par des restes de la liste W³,
R¹³ et R¹⁴ représentent indépendamment l'un de l'autre l'hydrogène, un reste alkyle en C₁ à C₄, alcényle en C₃ à C₆, un reste alkyle en C₁ à C₄ ou alcényle en C₃ à C₆, dont chacun est éventuellement substitué par du fluor ou du chlore, un reste alkoxy en C₁ à C₄, un reste cycloalkyle en C₃ à C₆ ou (cycloalkyle en C₃ à C₆)-(alkyle en C₁ à C₄) dont chacun est éventuellement substitué par du fluor, du chlore, un radical alkyle en C₁ à C₄ ou un radical alkyle en C₁ à C₄ substitué par du fluor ou du chlore, un reste phényle ou phényl-(alkyle en C₁ à C₄) dont chacun est éventuellement substitué une à trois fois par des restes de la liste W³, un groupement -OR¹² ou -NR¹¹R¹² ou forment ensemble un groupement -(CH₂)₅-, -(CH₂)₆- ou -(CH₂)₂-O-(CH₂)₂-,
R¹⁵ est un groupement -OR¹², -NR¹¹R¹² ou -N(R¹¹)-COOR¹²,
R¹⁶, R¹⁷ et R¹⁸ représentent indépendamment les uns des autres un reste alkyle en C₁ à C₄,
W₁ représente l'hydrogène, le fluor, le chlore, le brome, l'iode, un groupe cyano, formyle, nitro, un reste alkyle en C₁ à C₄, alkoxy en C₁ à C₄, un reste alkyle en C₁ à C₄ ou alkoxy en C₁ à C₄ dont chacun est substitué par du fluor ou du chlore, un reste (alkyle en C₁ à C₄)-carbonyle, (alkoxy en C₁ à C₄)-carbonyle ou -S(O)ₒR³,
W² représente le fluor, le chlore, le brome, un groupe cyano, formyle, nitro, un reste alkyle en C₁ à C₄, alkoxy en C₁ à C₄, un reste alkyle en C₁ à C₄ ou alkoxy en C₁ à C₄ dont chacun est substitué par du fluor ou du chlore, un reste (alkyle en C₁ à C₄)-carbonyle, (alkoxy en C₁ à C₄)-carbonyle, -S(O)ₒR³ ou -C(R¹¹)=N-R¹⁵,
W³ représente le fluor, le chlore, le brome, un groupe cyano, nitro, un reste alkyle en C₁ à C₄, alkoxy en C₁ à C₄, un reste alkyle en alkyle en C₁ à C₄) ou alkoxy en C₁ à C₄ dont chacun est substitué par du fluor ou du chlore, un reste di-(alkyle en C₁ à C₄)-amino, -S(O)ₒR³, -COOR¹⁹ ou -CONR²⁰R²¹,
R¹⁹ représente l'hydrogène, un reste alkyle en C₁ à C₄, un reste alkyle en C₁ à C₄ substitué par du fluor ou du chlore, un reste cycloalkyle en C₃ à C₆ éventuellement substitué par du fluor, du chlore, un radical alkyle en C₁ à C₄ ou un radical alkyle en C₁ à C₄ substitué par du fluor ou du chlore, ou un reste phényle éventuellement substitué une à trois fois par des restes de la liste W⁴,
R²⁰ et R²¹ représentent indépendamment l'un de l'autre l'hydrogène, un reste alkyle en C₁ à C₄, alcényle en C₃ à C₆, un reste alkyle en C₁ à C₄ ou alcényle en C₃ à C₆, dont chacun est substitué par du fluor ou du chlore, un reste alkoxy en C₁ à C₄, un reste cycloalkyle en C₃ à C₆ ou (cycloalkyle en C₃ à C₆)-(alkyle en C₁ à C₄) dont chacun est éventuellement substitué par du fluor, du chlore, un radical alkyle en C₁ à C₄ ou un radical alkyle en C₁ à C₄ substitué par du fluor ou du chlore, ou un reste phényle ou phényl-(alkyle en C₁ à C₄) dont chacun est éventuellement substitué une à trois fois par des restes de la liste W⁴, un groupement -OR¹⁶ ou -NR¹⁷R¹⁸ ou forment ensemble un groupement -(CH₂)₅-, -(CH₂)₆- ou - (CH₂)₂-O-(CH₂)₂-, et
W⁴ représente le fluor, le chlore, le brome, un groupe cyano, nitro, un reste alkyle en C₁ à C₄, alkoxy en C₁ à C₄, un reste alkyle en C₁ à C₄ ou alkoxy en C₁ à C₄, dont chacun est éventuellement substitué par du fluor ou du chlore, un reste di-(alkyle en C₁ à C₄)-amino, (alkoxy en C₁ à C₆)-carbonyle, di-(alkyle en C₁ à C₆)-aminocarbonyle ou -S(O)ₒR³.

3. Composés de formule (I) suivant la revendication 1, formule dans laquelle
Ar représente le reste 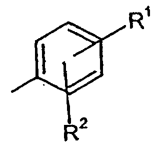
R¹ représente un substituant en position méta ou para de la série hydrogène, fluor, chlore, brome, iode, cyano, - CO-NR⁴R⁵, tétrahydropyrannyle ou l'un des groupements suivants:
(l) - X - A 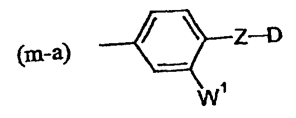
(n) - Y - E
R² représente l'hydrogène, le fluor, le chlore, le brome, un reste méthyle, éthyle, méthoxy, éthoxy, méthylthio, éthylthio, trifluorométhyle, difluorométhoxy, trifluorométhoxy ou trifluorométhylthio,
o a la valeur 0 ou 2,
R³ est un reste méthyle, éthyle, n-propyle, isopropyle, difluorométhyle ou trifluorométhyle,
R₄ et R⁵ représentent indépendamment l'un de l'autre l'hydrogène, un reste méthyle, éthyle, n-propyle, isopropyle, n-butyle, isobutyle, sec.-butyle, tertiobutyle ou un reste phényle ou benzyle dont chacun est éventuellement substitué une fois par un reste de la liste W¹,
X représente une liaison directe, l'oxygène, le soufre, un groupe carbonyle, -CH₂-, -(CH₂)₂-, -CH=CH-, (E ou Z) , -C≡C-, -CH₂O-, -(CH₂)₂O-, -CH(CH₃)O-, -OCH₂-, -O(CH₂)₂-, -SCH₂-, -S(CH₂)₂-, -SCH(CH₃)-, alkylènedioxy en C₁ à C₄, en particulier -OCH₂O-, -O(CH₂)₂O- ou -OCH(CH₃)O-.
A est un reste phényle éventuellement substitué une ou deux fois par des restes de la liste W¹ ou un reste furyle, benzofuryle, thiényle, benzothiényle, oxazolyle, benzoxazolyle, thiazolyle, benzthiazolyle, pyrrolyle, pyridyle, pyrimidyle, 1,3,5-triazinyle, quinolinyle, isoquinolinyle, indolyle, purinyle, benzodioxolyle, indanyle, benzodioxanyle ou chromanyle dont chacun est éventuellement substitué une ou deux fois par des restes de la liste W²,
Z représente l'oxygène ou le soufre,
D représente l'hydrogène, un reste méthyle, éthyle, n-propyle, isopropyle, n-butyle, isobutyle, sec.-butyle, tertiobutyle, les restes pentyle isomères, les restes hexyle isomères, un reste n-heptyle, n-octyle, n-iso-octyle, n-nonyle, n-décyle, n-undécyle, n-dodécyle, n-tridécyle, n-tétradécyle, n-pentadécyle, n-hexadécyle, 2-propényle, butényle, pentényle, hexényle, propargyle, butynyle, pentynyle, -CF₃, -CHF₂, -CClF₂, -CF₂CHFCl, -CF₂CH₂F, -CF₂CHF₂, -CF₂CCl₃, -CH₂CF₃, -CF₂CHFCF₃, -CH₂CF₂CHF₂, -CH₂CF₂CF₃, un reste cyclopropyle, cyclobutyle, cyclopentyle, cyclohexyle, cyclopropylméthyle, cyclobutylméthyle, cyclopentylméthyle ou cyclohexylméthyle dont chacun est éventuellement substitué une à trois fois par du fluor, du chlore, du brome, un radical méthyle, éthyle, n-propyle, isopropyle, n-butyle, isobutyle, sec.-butyle, tertiobutyle, éthényle, 1-propényle, 2,2-diméthyléthényle, -CH=CCl₂, phényle, styryle, un radical phényle ou 4-chlorostyryle dont chacun est éventuellement substitué par du fluor, du chlore ou du brome, un reste cyclopentényle, cyclohexényle, cyclohexénylméthyle ou cyclopenténylméthyle, dont chacun est éventuellement substitué par du fluor, du chlore, un radical méthyle, éthyle, n-propyle, isopropyle, n-butyle, isobutyle, sec.-butyle ou tertiobutyle, un reste benzyle, phénéthyle, naphtylméthyle, tétrahydronaphtylméthyle, furylméthyle, thiénylméthyle, pyrrolylméthyle, oxazolylméthyle, isoxazolylméthyle, thiazolylméthyle ou pyridylméthyle dont chacun est éventuellement substitué une ou deux fois par un radical nitro, fluoro, chloro, bromo, méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, trifluorométhyle, trifluorométhoxy, difluorométhoxy ou chlorodifluorométhoxy, un groupement -CO-R⁶, -CO-NR⁷R⁸ ou le groupement
- (CH₂)ₚ - (CR⁹R¹⁰)_{q} - (CH₂)ᵣ - G
Z et D peuvent aussi former ensemble de façon tout particulièrement appréciée un reste phénoxyméthyle éventuellement substitué une ou deux fois par un radical nitro, fluoro, chloro, bromo, méthyle, éthyle, n-propyle, isopropyle, méthoxy, éthoxy, n-propoxy, isopropoxy, trifluorométhyle, trifluorométhoxy, difluorométhoxy ou chlorodifluorométhoxy,
Y représente une liaison simple, l'oxygène, le soufre, un groupe carbonyle, -CH₂-, -(CH₂)₂-, -CH=CH- (E ou Z), -C≡C-, -CH₂O-, -(CH₂)₂O-, -CH(CH₃)O-, -OCH₂-, -O(CH₂)₂-, -SCH₂-, -S(CH₂)₂-, -SCH(CH₃)-, alkylènedioxy en C₁ à C₄, notamment -OCH₂O- ou -O(CH₂)₂O- ou un reste p-phénylène éventuellement substitué par un reste de la liste W¹,
E représente l'hydrogène, un reste méthyle, éthyle, n-propyle, isopropyle, n-butyle, isobutyle, sec.-butyle, tertiobutyle, les restes pentyle isomères, les restes hexyle isomères, un reste n-heptyle, n-octyle, n-iso-octyle, n-nonyle, n-décyle, n-undécyle, n-dodécyle, n-tridécyle, n-tétradécyle, n-pentadécyle, n-hexadécyle, 2-propényle, butényle, pentényle, hexényle, propargyle, butynyle, pentynyle, -CF₃, -CHF₂, -CClF₂-, -CF₂CHFCl, -CF₂CH₂F, -CF₂CHF₃, -CF₂CCl₃, -CH₂CF₃, -CF₂CHFCF₃, -CH₂CF₂CHF₂, -CH₂CF₂CF₃, un reste cyclopropyle, cyclobutyle, cyclopentyle ou cyclohexyle dont chacun est éventuellement substitué une à trois fois par du fluor, du chlore, du brome, un radical méthyle, éthyle, n-propyle, isopropyle, n-butyle, isobutyle, sec.-butyle, tertiobutyle, éthényle, 1-propényle, 2,2-diméthyléthényle, -CH=CCl₂, phényle, styryle, un radical phényle éventuellement substitué par du fluor, du chlore ou du brome, ou un radical 4-chlorostyryle, un reste cyclopentényle ou cyclohexényle dont chacun est éventuellement substitué par du fluor, du chlore, un radical méthyle, éthyle, n-propyle, isopropyle, n-butyle, isobutyle, sec.-butyle ou tertiobutyle, un reste phényle éventuellement substitué une ou deux fois par des restes de la liste W¹, un reste furyle, thiényle, pyrrolyle, oxazolyle, isoxazolyle, thiazolyle ou pyridyle dont chacun est éventuellement substitué une ou deux fois par des restes de la liste W², ou le groupement
- (CH₂)ₚ - (CR⁹R¹⁰)_{q} - (CH₂)ᵣ - G
R⁶ est un reste méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, cyclopropyle, cyclohexyle, cyclohexyloxy, cyclohexylméthyloxy, phényle, 2-chlorophényle, 3-chlorophényle, 2,6-difluorophényle, 2,4-dichlorophényle, 3,4-dichlorophényle, 2-trifluorométhoxyphényle ou 4-trifluorométhoxyphényle,
R⁷ représente l'hydrogène,
R⁸ est un reste méthyle, éthyle ou un reste phényle éventuellement substitué une fois par du chlore,
p, q, et r ont indépendamment les uns des autres la valeur 0, 1, 2 ou 3, leur somme étant inférieure à 4,
R⁹ et R¹⁰ représentent indépendamment l'un de l'autre, l'hydrogène un reste méthyle, éthyle, n-propyle, isopropyle, n-butyle, isobutyle, sec.-butyle ou tertiobutyle,
G est un groupe cyano, un reste 5,6-dihydrodioxazine-2-yle, 3-pyridyle, 3-furyle, 3-thiényle, 2-thiazolyle, 5-thiazolyle, 2-dioxolanyle, 1,3-dioxanne-2-yle, 2-dithiolanyle, 1,3-dithiane-2-yle ou 1,3-thioxane-2-yle dont chacun est éventuellement substitué dans la position de liaison par le reste R¹¹, ou l'un des groupements suivants :
(a) ―CO―R¹¹
(b) ―CO―OR¹²
(c) ―CO―NR¹³R¹⁴
(d) ―CS―NR¹³R¹⁴ 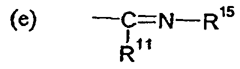 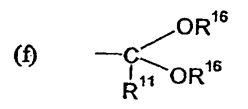 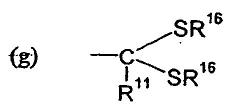 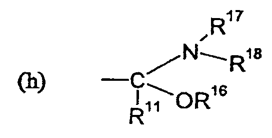 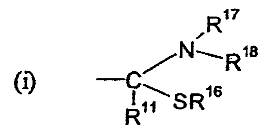
R¹¹ représente l'hydrogène, un reste méthyle, éthyle, n-propyle, isopropyle, n-butyle, isobutyle, sec.-butyle, tertiobutyle, les restes pentyle isomères, les restes hexyle isomères, un reste -CF₃, -CHF₂, -CClF₂-, -CF₂CHFCl, -CF₂CH₂F, -CF₂CHF₂, -CF₂CCl₃, -CH₂CF₃, alcényle en C₃ à C₆, un reste alcényle en C₃ à C₆ substitué une à trois fois par du fluor ou du chlore, un reste cyclopropyle, cyclopentyle ou cyclohexyle éventuellement substitué une à deux fois par du fluor, du chlore, un radical méthyle, éthyle, n-propyle, isopropyle, -CF₃, -CHF₂, -CClF₂-, -CF₂CHFCl, -CF₂CH₂F, -CF₂CHF₂, -CF₂CCl₃ ou -CH₂CF₃ ou un reste phényle éventuellement substitué une ou deux fois par un radical méthylcarbonylamino, éthylcarbonylamino, méthylcarbonylméthylamino et/ou par des restes de la liste W³,
R¹² représente l'hydrogène, un reste méthyle, éthyle, n-propyle, isopropyle, n-butyle, isobutyle, sec.-butyle, tertiobutyle, -CH₂CF₃, allyle, un reste cyclopropyle, cyclopentyle, cyclohexyle, cyclopropylméthyle, cyclopentylméthyle, cyclohexylméthyle, cyclopropyléthyle, cyclopentyléthyle ou cyclohexyléthyle dont chacun est éventuellement substitué une ou deux fois par du fluor, du chlore, un radical méthyle, éthyle, n-propyle, isopropyle, -CF₃, -CHF₂, -CClF₂-, -CF₂CHFCl, -CF₂CH₂F, -CF₂CHF₂, -CF₂CCl₃ ou -CH₂CF₃, ou bien un radical benzyle ou phénéthyle dont chacun est éventuellement substitué une ou deux fois par des restes de la liste W³,
R¹³ et R¹⁴ représentent indépendamment l'un de l'autre l'hydrogène, un reste méthyle, éthyle, n-propyle, isopropyle, n-butyle, isobutyle, sec.-butyle, tertiobutyle, -CH₂CF₃, méthoxy, éthoxy, allyle, un reste cyclopropyle, cyclopentyle, cyclohexyle, cyclopropylméthyle, cyclopentylméthyle ou cyclohexyl-méthyle dont chacun est éventuellement substitué une ou deux fois par du fluor, du chlore, un radical méthyle, éthyle, n-propyle, isopropyle ou trifluoro-méthyle, un reste phényle, benzyle ou phénéthyle dont chacun est éventuellement substitué une ou deux fois par des restes de la liste W³, un groupement -OR¹² ou -NR¹¹R¹²,
R¹⁵ est un groupement -OR¹², -NR¹¹R¹² ou -N(R¹¹)-COOR¹²,
R¹⁶, R¹⁷ et R¹⁸ représentent indépendamment les uns des autres un radical méthyle, éthyle, n-propyle ou isopropyle,
W¹ représente l'hydrogène, le fluor, le chlore, le brome, un groupe cyano, formyle, nitro, un radical méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, -CF₃, -CHF₂, -CClF₂-, -CF₂CHFCl, -CF₂CH₂F, -CF₂CHF₂, -CF₂CCl₃, -CH₂CF₃, -CF₂CHFCF₃, -CH₂CF₂CHF₂, -CH₂CF₂CF₃, trifluorométhoxy, difluorométhoxy, chlorodifluorométhoxy, acétyle, propionyle, butyryle, isobutyryle, méthoxycarbonyle, éthoxycarbonyle, n-propoxycarbonyle, isopropoxycarbonyle, n-butoxycarbonyle, isobutoxycarbonyle, sec.-butoxycarbonyle, tertiobutoxycarbonyle ou -S(O)ₒR³,
W² représente le fluor, le chlore, le brome, le groupe cyano, un reste méthyle, éthyle, n-propyle, isopropyle, trifluorométhyle, trifluorométhoxy, difluorométhoxy, chlorodifluorométhoxy, acétyle, trifluorométhylthio, -CH=N-OCH₃, -CH=N-OC₂H₅, -CH=N-OC₃H₇, -C(CH₃)=N-OCH₃, -C (CH₃) =N-OC₂H₅, -C (CH₃) =N-OC₃H₇, -C(C₂H₅)=N-OCH₃, -C (C₂H₅) =N-OC₂H₅ ou -C (C₂H₅) =N-OC₃H₇,
W³ représente le fluor, le chlore, un groupe cyano, nitro, un reste méthyle, éthyle, méthoxy, éthoxy, méthylthio, trifluorométhyle, trifluorométhoxy, trifluorométhylthio, diméthylamino, diéthylamino, - COOR¹⁹ ou -CONR²⁰R²¹,
R¹⁹ représente l'hydrogène, un reste méthyle, éthyle, n-propyle, isopropyle, tertiobutyle, -CH₂CF₃, un reste cyclopropyle, cyclopentyle ou cyclohexyle dont chacun est éventuellement substitué une ou deux fois par du fluor, du chlore, un radical méthyle, éthyle, n-propyle, isopropyle ou -CF₃, ou un reste phényle éventuellement substitué une ou deux fois par des restes de la liste W₄,
R²⁰ et R²¹ représentent indépendamment l'un de l'autre l'hydrogène, un reste méthyle, éthyle, n-propyle, isopropyle, n-butyle, isobutyle, sec.-butyle, tertiobutyle, -CH₂CF₃, méthoxy, éthoxy, allyle, un reste cyclopropyle, cyclopentyle, cyclohexyle, cyclopropylméthyle, cyclopentylméthyle ou cyclohexylméthyle dont chacun est éventuellement substitué une ou deux fois par du fluor ou du chlore, un reste phényle, benzyle ou phénéthyle dont chacun est éventuellement substitué une ou deux fois par des restes de la liste W⁴, un groupement -OR¹⁶ ou -NR¹⁷R¹⁸,
W⁴ représente le fluor, le chlore, le brome, un groupe cyano, nitro, un reste méthyle, éthyle, tertiobutyle, méthoxy, éthoxy, méthylthio, trifluorométhyle, trifluorométhoxy ou trifluorométhylthio.

4. Procédé de production de composés de formule (I) suivant la revendication 1, **caractérisé en ce que** :
A) on obtient des composés de formule (I) 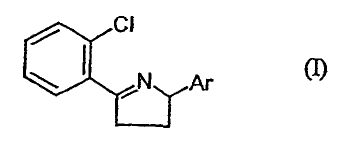 dans laquelle
Ar a les définitions indiquées dans la revendication 1,
a) par réaction de dérivés aminocétoniques de formule (VIII) 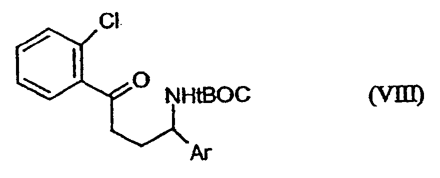 dans laquelle
Ar a les définitions indiquées ci-dessus,
avec un acide puis condensation cyclique éventuellement en présence d'un accepteur d'acide, ou bien
b) par réduction du groupe nitro de nitrocétones de formule (XVIII) 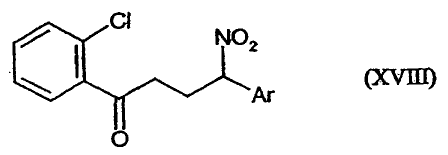 dans laquelle
A a les définitions indiquées dans la revendication 1, auquel cas 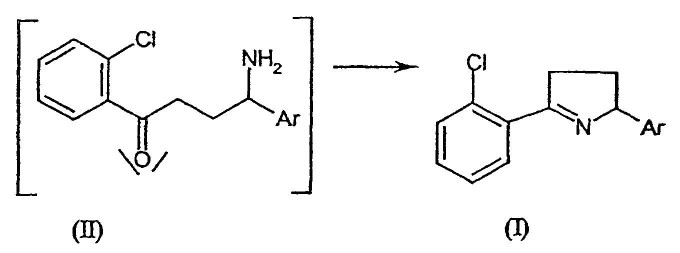 il se forme à un stade intermédiaire une aminocétone de formule (II) qui est toutefois transformée *in situ* par condensation cyclique en composé (I), en particulier en milieu acide, ou bien
c) par hydrolyse d'imines de formule (XXVII) 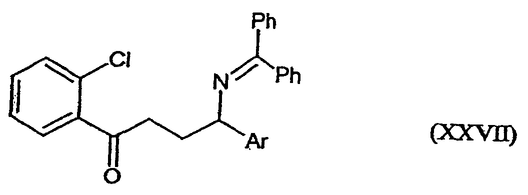
dans laquelle Ar a les définitions indiquées dans la revendication 1,
avec des acides aqueux 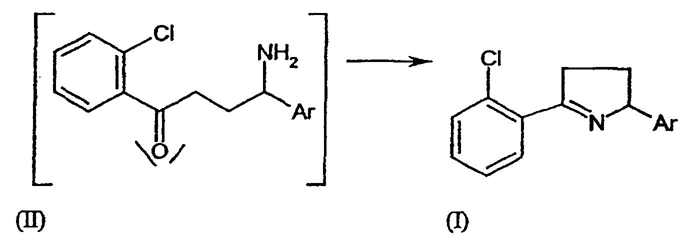 auquel cas
il se forme à un stade intermédiaire une aminocétone de formule (II) qui est toutefois transformée *in situ* en composé (I) par condensation cyclcique,
B) par réaction de composés de formule (III) 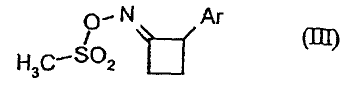 dans laquelle
Ar a les définitions indiquées dans la revendication 1,
avec des composés aryliques de Grignard de formule (IV) 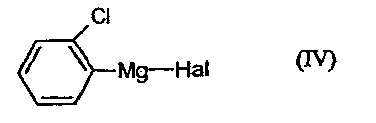 dans laquelle
Hal représente le brome ou l'iode,
en présence d'un diluant,
C) on obtient des composés de formule (I-b) 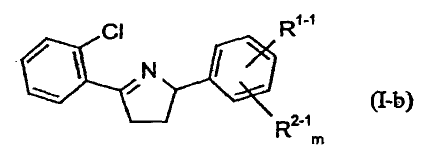 dans laquelle
m a les définitions indiquées dans la revendication 1,
R¹⁻¹ représente A ou l'un des groupements suivants
(m) ―B―Z-D 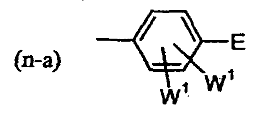
dans lesquels
A, B, D, E, W¹ et Z ont les définitions indiquées dans la revendication 1 et
R²⁻¹ par représente l'hydrogène, le fluor, un groupe cyano, nitro, un reste alkyle, alkoxy, halogénalkyle, halogénalkoxy, alkoxyalkoxy ou -SR³,
où
R³ a les définitions indiquées dans la revendication 1,
par couplage de composés de formule (V) 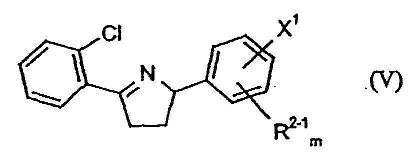 dans laquelle m a les définitions indiquées dans la revendication 1,
R²⁻¹ a les définitions indiquées ci-dessus et
X¹ représente le brome, l'iode, ou un groupe -OSO₂CF₃,
avec des acides boroniques de formule (VI)
R¹⁻¹ B(OH)₂ (VI)
dans laquelle
R¹⁻¹ a les définitions indiquées ci-dessus,
en présence d'un catalyseur et en présence d'un accepteur d'acide et en présence d'un solvant,
D) on obtient des composés de formule (I-c) 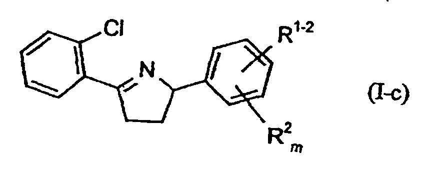 dans laquelle
R² et m ont les définitions indiquées dans la revendication 1,
R¹⁻² représente l'un des groupements suivants
(m-b) -B-ZS-D¹
(n-b) -Y¹-E¹
dans lesquels
B et Z ont les définitions indiquées dans la revendication 1,
Y¹ représente l'oxygène ou le soufre et
D¹ et E¹ représentent le groupement
-(CH₂)ₚ-(CR⁹R¹⁰)_{q}-(CH₂)ᵣ - G
dans lequel
R⁹, R¹⁰, G, p, q et r ont les définitions indiquées ci-dessus,
par condensation d'imines cycliques de formule (I-d) 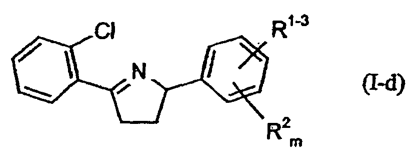 dans laquelle
R² et m ont les définitions indiquées dans la revendication 1 et
R¹⁻³ représente l'un des groupements
(m-c) -B-Z-H
(n-c) -Y¹-H
dans lesquels
B et Z ont les définitions indiquées dans la revendication 1,
Y¹ a les définitions indiquées ci-dessus,
avec des composés de formule (VII)
Ab - (CH₂)ₚ - (CR⁹R¹⁰)_{q} - (CH₂)ᵣ - G (VII),
dans laquelle
R⁹, R¹⁰, G, p, q et r ont les définitions indiquées dans la revendication 1 et
Ab représentent un groupe partant,
E) On obtient des composés de formule (I-e) 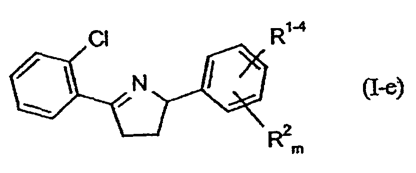 dans laquelle
R² et m ont les définitions indiquées dans la revendication 1 et
R¹⁻⁴ représentent un groupement contenant le reste G choisi dans la description des composés de formule (I) conformes à l'invention, où
G représente l'un des groupements (e) à (k) mentionnés ci-dessus,
par des dérivatisations généralement classiques et connues des dérivés cétoniques, des dérivés carboxyliques ou des nitriles correspondants, c'est-à-dire des composés de formule (I) dans lesquels G est un groupe cyano ou l'un des groupements (a) à (d),
F) on obtient aussi des composés de formule (I-f) 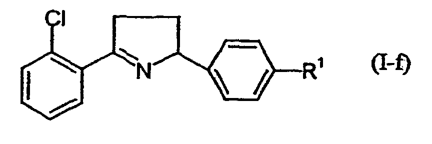 dans laquelle R¹ a les définitions indiquées dans la revendication 1, en faisant réagir dans une première étape
α) l'O-chloracétophénone de formule (F-I) 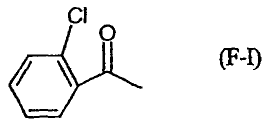 avec le chlorure de diméthylméthylène-ammonium de formule (F-II) 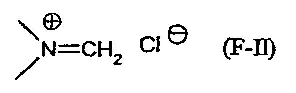 pour obtenir le composé de formule (F-III) 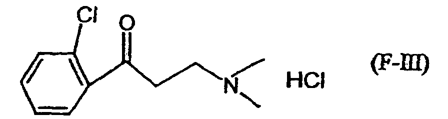 et en faisant réagir ce composé dans une deuxième étape
β) avec des cyanures de benzyle de formule (F-IV) 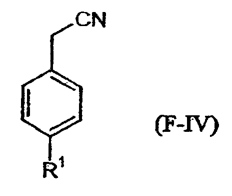 dans lesquels R¹ a la définition indiquée dans la revendication 1, pour obtenir des composés de formule (F-V) 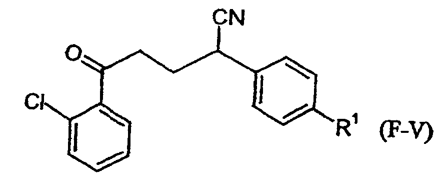 qu'on dérivative dans l'étape suivante
δ) avec le système lessive de soude/H₂O₂ en composés de formule (F-VI) 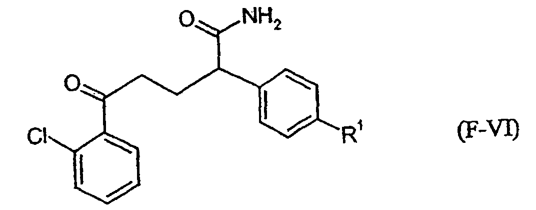 dans laquelle R¹ possède les définitions indiquées dans la revendication 1, et on cyclise ces composés dans une étape finale
γ) par réaction avec le PIFA (1,1-bis-(trifluoracétoxy)-iodobenzène) de formule (F-VII) 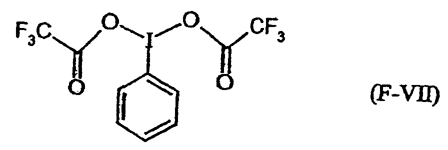 en imines cycliques de formule (I-f),
G) on obtient aussi des composés de formule (I-f) 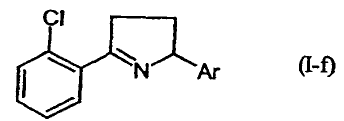 dans laquelle Ar a les définitions indiquées dans la revendication 1, en faisant réagir dans une première étape
α) des arylbutyrolactames de formule (XI) 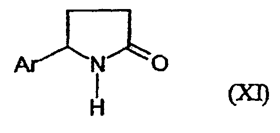 avec le chlorure d'acide o-chlorobenzoïque 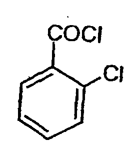 pour obtenir des composés de formule G(I) 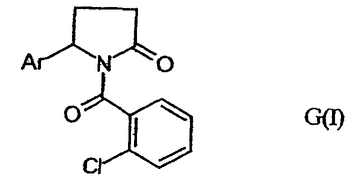 et on fait réagir ces composés dans une deuxième étape
β) avec l'ester méthylique d'acide o-chlorobenzoïque 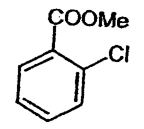 pour obtenir des composés de formule G(II) 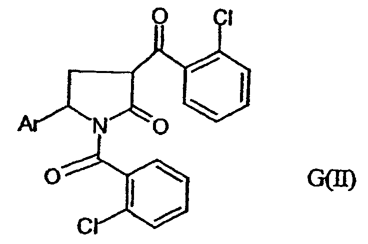 qu'on fait réagir dans une dernière étape
δ) avec le système HBr/acide acétique cristallisable pour obtenir des composés de formule (I-f)

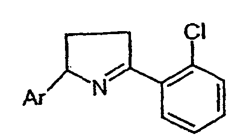

5. Composés de formule (VIII) 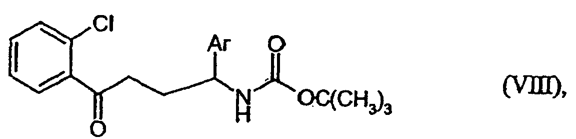 dans laquelle
Ar a les définitions indiquées dans la revendication 1.

6. Composés de formule (XVIII) 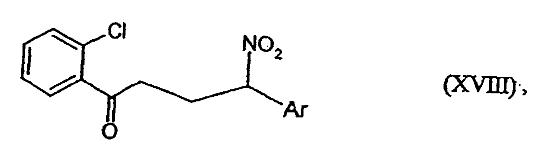 dans laquelle
Ar a les définitions indiquées dans la revendication 1.

7. Compositions pesticides, **caractérisées par** une teneur en au moins un composé de formule (I) suivant la revendication 1.

8. Utilisation de composés de formule (I) suivant la revendication 1 pour combattre des parasites, à l'exception du traitement thérapeutique d'être humains et d'animaux.

9. Procédé pour combattre des parasites à l'exception du traitement thérapeutique d'être humains et d'animaux, **caractérisé en ce qu'**on fait agir des composés de formule (I) suivant la revendication 1 sur les parasites et/ou sur leur milieu.

10. Procédé de préparation de compositions pesticides, **caractérisé en ce qu'**on mélange des composés de formule (I) suivant la revendication 1 avec des diluants et/ou des agents tensioactifs.

11. Utilisation de composés de formule (I) suivant la revendication 1 pour la préparation de compositions pesticides.

12. Utilisation de composés de formule (I) suivant la revendication 1 pour la préparation d'une composition destinée à combattre des arthropodes dans l'exploitation du bétail.
